(19) Europäisches Patentamt
European Patent Office
Office européen des brevets

(11) EP 4 349 987 A1

(12) **EUROPEAN PATENT APPLICATION**
published in accordance with Art. 153(4) EPC

(43) Date of publication:
**10.04.2024 Bulletin 2024/15**

(21) Application number: **22811423.7**

(22) Date of filing: **27.05.2022**

(51) International Patent Classification (IPC):
***C12N 15/113*** *(2010.01)* ***A61K 31/7088*** *(2006.01)*
***A61K 31/7115*** *(2006.01)* ***A61K 31/712*** *(2006.01)*
***A61K 31/7125*** *(2006.01)* ***A61K 47/54*** *(2017.01)*
***A61K 47/55*** *(2017.01)* ***A61K 47/60*** *(2017.01)*
***A61K 47/61*** *(2017.01)* ***A61K 47/62*** *(2017.01)*
***A61K 47/68*** *(2017.01)* ***A61K 48/00*** *(2006.01)*
***A61P 43/00*** *(2006.01)* ***C12N 15/12*** *(2006.01)*
***C12N 15/55*** *(2006.01)*

(52) Cooperative Patent Classification (CPC):
**A61K 31/7088; A61K 31/7115; A61K 31/712; A61K 31/7125; A61K 47/54; A61K 47/55; A61K 47/60; A61K 47/61; A61K 47/62; A61K 47/68; A61K 48/00; A61P 43/00; C07K 14/435; C12N 9/14; C12N 15/113**

(86) International application number:
**PCT/JP2022/021818**

(87) International publication number:
**WO 2022/250155 (01.12.2022 Gazette 2022/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2021 JP 2021090359**

(71) Applicant: **Sumitomo Pharma Co., Ltd.**
**Osaka 541-0045 (JP)**

(72) Inventors:
- **MAN, Zhiqiu**
  **Osaka-shi, Osaka 554-0022 (JP)**
- **ASANO, Shigehiro**
  **Osaka-shi, Osaka 554-0022 (JP)**
- **HINO, Kyosuke**
  **Osaka-shi, Osaka 554-0022 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

(54) **ANTISENSE NUCLEIC ACID**

(57) An oligonucleotide that expresses a functional human WRN protein against a skip mutation in the 26th or 28th exon of a human WRN gene, or a pharmaceutically acceptable salt thereof, wherein one nucleotide is bound to another via a phosphate group and/or modified phosphate group in the oligonucleotide, the oligonucleotide contains a modified nucleic acid having at least one modified sugar, the oligonucleotide is 10 to 30mer in nucleotide length, and the nucleotide sequence of the oligonucleotide is: a nucleotide sequence having a sequence identity of 90% or more and 100% or less with a nucleotide sequence complementary to at least one target region having the same nucleotide length as the oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6; a nucleotide sequence complementary to a nucleotide sequence formed by deleting, substituting, inserting, or adding one or several nucleotides in the target region; or a nucleotide sequence hybridizable with an oligonucleotide having the target region under stringent conditions.



Processed by Luminess, 75001 PARIS (FR)

(Cont. next page)

FIG.3

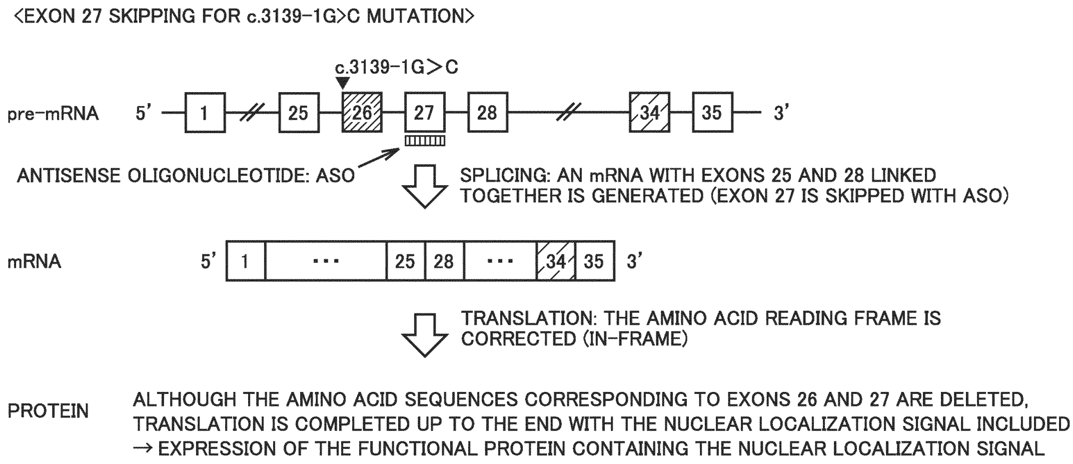
<EXON 27 SKIPPING FOR c.3139-1G>C MUTATION>
c.3139-1G>C
pre-mRNA
5'
1
25
26
27
28
34
35
3'
ANTISENSE OLIGONUCLEOTIDE: ASO
SPLICING: AN mRNA WITH EXONS 25 AND 28 LINKED TOGETHER IS GENERATED (EXON 27 IS SKIPPED WITH ASO)
mRNA
5'
1
...
25
28
...
34
35
3'
TRANSLATION: THE AMINO ACID READING FRAME IS CORRECTED (IN-FRAME)
PROTEIN
ALTHOUGH THE AMINO ACID SEQUENCES CORRESPONDING TO EXONS 26 AND 27 ARE DELETED, TRANSLATION IS COMPLETED UP TO THE END WITH THE NUCLEAR LOCALIZATION SIGNAL INCLUDED
→ EXPRESSION OF THE FUNCTIONAL PROTEIN CONTAINING THE NUCLEAR LOCALIZATION SIGNAL

**Description**

TECHNICAL FIELD

The present invention relates to an antisense oligonucleotide (antisense nucleic acid) that enables skipping of the 27th exon of a WRN gene, which is the causal gene for Werner syndrome, and a pharmaceutical composition containing the oligonucleotide.

BACKGROUND ART

Werner syndrome is a rare disease that causes premature ageing, and is known to present in the juvenile stage with common features found in elderly such as generation of white hair, hair loss, diabetes mellitus, heart disease, cancers, skin retraction, scleroderma-like skin changes, juvenile cataract, progeria, and hypogonadism. More specifically, geriatric symptoms including skin atrophy and sclerosis, hair change such as white hair and baldness, and cataracts begin to appear in the twenties and thirties. In addition, diabetes mellitus, arteriosclerosis, malignant tumors, and others are likely to occur in combination, and most of the patients die in their fifties. In many cases, exacerbation of the refractory skin ulcer that usually develops on the elbows, knees, heels, and the like, leads to amputation of the extremities, with a consequent deterioration in quality of life (NPL 1).

Werner syndrome is an autosomal recessive rare disease for which the WRN gene, located on the short arm of chromosome 8 (8p12) and encoding the RecQ DNA/RNA helicase (WRN helicase), has been identified as the causative gene (NPL 2, NPL 3).

The WRN protein consists of 1432 amino acids, and has ATP-dependent DNA/RNA helicase activity and 3' → 5' exonuclease activity. The WRN protein has various physiological functions including DNA replication, base excision repair, DNA double-strand break repair, transcription, and telomere maintenance, and serves for gene repair and maintenance of the stability of chromosomes. Therefore, significant genomic destabilization is found in Werner syndrome. Fibroblasts derived from a Werner syndrome patient have a shorter replicative lifespan and exhibit significantly lower growth rates than normal cells derived from a healthy individual (NPL 4). The reduced proliferative potential of fibroblasts is inferred to be a cause for delayed of wound healing of skin ulcers in Werner syndrome (NPL 5).

A nuclear localization signal (NLS) is located near the C terminus of the WRN protein and is involved in normal physiological functions by inducing nuclear localization (Fig. 1). However, when an incomplete WRN protein with a deletion of the nuclear localization signal is generated due to the occurrence of a premature stop codon (termination codon) as a result of a mutation such as splicing, nonsense, and frameshift mutations, the WRN helicase, which is supposed to function in the nuclei, is prevented from being localized in the nuclei, and is thus unable to perform its functions, resulting in the onset of Werner syndrome (Fig. 1 and Fig. 2) (NPL 3).

In approximately 7/10 of Japanese patients with Werner syndrome, the reading frame is shifted (out-of-frame) by the skipping mutation c.3139-1G>C in the 26th exon of the human WRN gene, resulting in the occurrence of a stop codon (termination codon) in the 27th exon (hereinafter, occasionally referred to as "exon 27").
Therefore, a WRN protein with deletion of the nuclear localization signal, which is otherwise present at the C-terminal part corresponding to the downstream of the 27th exon of the human WRN gene, is generated, leading to the loss of the original functions (Fig. 2) (NPL 3).

CITATION LIST

PATENT LITERATURE

PTL 1: WO 2011/052436
PTL 2: WO 2014/046212
PTL 3: WO 2015/125783
PTL 4: WO 1991/009033
PTL 5: WO 2009/064471


NON PATENT LITERATURE

NPL 1: Geriatr Gerontol Int (2013) 13: 475-481

NPL 2: Nature (1992) 355: 735-738
NPL 3: Am. J. Hum. Genet. (1997) 60: 330-341
NPL 4: Human Genetics (1981) 58: 310-316
NPL 5: Rinsho derma (2000) 40: 1512-1513
NPL 6: Annu. Rev. Pharmacol. Toxicol. (2010) 50: 259-293
NPL 7: J. Med. Chem. (2016) 59: 9645-9667
NPL 8: J. Am. Chem. Soc (2016) 138: 15663-15672

## SUMMARY OF INVENTION

### TECHNICAL PROBLEM

Although Werner syndrome is an orphan disease that threatens the lives and quality of life of patients, there is currently no effective treatment for Werner syndrome and the discovery of new therapeutic drugs is urgently needed.

In recent years, nucleic acid drugs have been used as new therapeutic agents. Nucleic acid drugs, each of which is composed of an oligonucleic acid, which is a nucleic acid or modified nucleic acid of ten to several tens of nucleotides linked together, are pharmaceuticals produced by chemical synthesis, some of which are known to act directly on messenger RNA involved in protein translation or noncoding RNA or the like, and some of which are known to act on target protein like antibody drugs. Splicing-switching antisense oligonucleotides, one of the nucleic acid drugs, can inhibit the binding of a splicing factor to an mRNA precursor (hereinafter, occasionally referred to as "pre-mRNA") to switch splicing of a nearby exon (splice-out), thereby normalizing the reading frame of frameshifted abnormal RNA. Accordingly, one exon of a gene is spliced out to allow expression of the corresponding protein retaining the N-terminal and C-terminal parts that are important for functional expression to be expressed. Thus, skipping a targeted exon with a splice-switching antisense oligonucleotide allows a functional protein to be re-expressed that has been translated to the C-terminus, thereby potentially ameliorating the pathological condition of a disease. This is called exon-skipping therapy, and the proteins expressed by the treatment are shorter than normal, but can still restore the functions that were defective due to a mutation (NPL 6).

As described above, the skipping mutation c.3139-1G>C in the 26th exon of the human WRN gene has been reported as the most common mutation in Werner syndrome. The skipping mutation c.3139-1G>C causes the appearance of a termination codon within the 27th exon, and a WRN protein with a deletion of an amino acid sequence corresponding to the downstream of the termination codon and containing a nuclear localization signal present in the C-terminal part, is generated, resulting in the loss of the original physiological functions. To improve such condition, the present inventors focused on splicing-switching antisense oligonucleotides, and investigated to discover an antisense oligonucleotide that induces skipping of exon 27 of a human WRN gene.

The present invention was made in view of such circumstances, and an object of the technical problem to be solved by the present invention is to provide an oligonucleotide enabling skipping of the 27th exon of the human WRN gene, and an composition containing the oligonucleotide and enabling skipping of the 27th exon of a human WRN gene with high efficiency.

### SOLUTION TO PROBLEM

The present inventors have found that by targeting a sequence consisting of nucleotides of exon 27 and those around it in an mRNA precursor for the human WRN gene with an antisense oligonucleotide, skipping of exon 27 can be induced with high efficiency. On the basis of this finding, the present invention was completed. More particularly, the present invention is as follows.

[1] An oligonucleotide according to the present invention is an oligonucleotide that expresses a functional human WRN protein against a skipping mutation in the 26th or 28th exon of the human WRN gene, or a pharmaceutically acceptable salt thereof, wherein

one nucleotide is bound to another via a phosphate group and/or modified phosphate group in the oligonucleotide,
the oligonucleotide contains a modified nucleic acid having at least one modified sugar,
the oligonucleotide is 10 to 30mer in nucleotide length, and
the nucleotide sequence of the oligonucleotide is:

a nucleotide sequence having a sequence identity of 90% or more and 100% or less with a nucleotide sequence complementary to at least one target region having the same nucleotide length as the oligonucleotide in a nucleotide sequence set forth in SEQ ID NO: 1, SEQ ID NO: 2, SEQ ID NO: 3, SEQ ID NO:

4, SEQ ID NO: 5, or SEQ ID NO: 6;
a nucleotide sequence complementary to a nucleotide sequence formed by deleting, substituting, inserting, or adding one or several nucleotides in the target region; or
a nucleotide sequence hybridizable with an oligonucleotide having the target region under stringent conditions.

An oligonucleotide having the configuration or a pharmaceutically acceptable salt thereof which enables skipping of the 27th exon of the human WRN gene.
[2] It is preferable that the nucleotide sequence of the oligonucleotide be a nucleotide sequence having a sequence identity of 95% or more and 100% or less with a nucleotide sequence complementary to at least one target region having the same nucleotide length as the oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6.
[3] It is preferable that the nucleotide sequence of the oligonucleotide be a nucleotide sequence complementary to at least one target region having the same nucleotide length as the oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6.
[4] It is preferable that the oligonucleotide be 15 to 25mer in nucleotide length.
[5] It is preferable that a sugar constituting the oligonucleotide be D-ribofuranose, and modification of a sugar be sugar modification on a hydroxy group at position 2' of D-ribo furanose.
[6] It is preferable that modification of a sugar constituting the oligonucleotide be made to form a group represented by the following formula (A1):

[Formula 1]

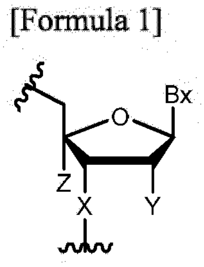

(A1)

wherein Bx is a nucleobase, being independently in each occurrence a group formed from adenine, guanine, cytosine, 5-methylcytosine, thymine, or uracil; X is a phosphate bond, being independently in each occurrence a phosphodiester bond, a phosphorothioate bond, a phosphorodithioate bond, a phosphoamidate bond, a boranophosphate bond, or an alkylphosphonate bond; Y is independently in each occurrence a hydrogen atom, a hydroxy group, a fluorine atom, an optionally substituted alkoxy group having one to six carbon atoms, or an optionally substituted amino group; and Z is independently in each occurrence a hydrogen atom, or an alkyl group having one to five carbon atoms and optionally having a carbon-oxygen double bond or a cyclic structure, or a certain carbon atom in the alkyl group and Y are taken together to form a ring.
[7] It is preferable that modification of a sugar constituting the oligonucleotide be made to form a group represented by the following formula (A2) or formula (A3):

[Formula 2]

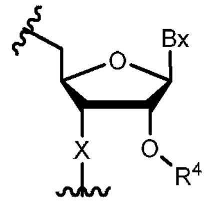

(A2)

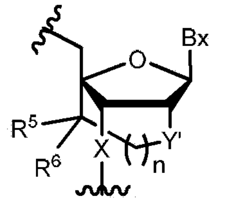

(A3)

wherein Bx is a nucleobase, being independently in each occurrence a group formed from adenine, guanine, cytosine, 5-methylcytosine, thymine, or uracil; X is a phosphate bond, being independently in each occurrence a phosphodiester bond, a phosphorothioate bond, a phosphorodithioate bond, a phosphoamidate bond, a boranophosphate bond, or an alkylphosphonate bond; $R^4$ is independently in each occurrence a hydrogen atom or an optionally substituted alkyl group having one to six carbon atoms; Y' is an oxygen atom or an optionally substituted nitrogen atom; $R^5$ and $R^6$ are each independently in each occurrence a hydrogen atom or an optionally substituted alkyl group having one to six carbon atoms, or $R^5$ and $R^6$ are taken together to form a carbonyl group or a ring; and n is 0 or 1.

[8] It is preferable that modification of a sugar constituting the oligonucleotide be made to form a group represented by the formula (A2), and the $R^4$ be independently in each occurrence a methyl group, a methoxyethyl group, or an N-methylpropanamide group.

[9] It is preferable that modification of a sugar constituting the oligonucleotide be made to form a group represented by the formula (A3), the Y' be an oxygen atom, or a nitrogen atom optionally substituted with a hydrogen atom, a methyl group, a methoxyethyl group, or an N-methylpropanamide group, the $R^5$ and $R^6$ be each independently in each occurrence a hydrogen atom or an alkyl group having one or two carbon atoms, or the $R^5$ and $R^6$ be taken together to form a carbonyl group or a ring having three to six carbon atoms, and n be 0 or 1.

[10] It is preferable that at least one intemucleotide bond in the oligonucleotide be a phosphorothioate bond.

[11] It is preferable that at least one internucleotide bond in the oligonucleotide be a phosphodiester bond.

[12] It is preferable that an intemucleotide bond in the oligonucleotide be a phosphodiester bond or a phosphorothioate bond.

[13] It is preferable that the nucleotide sequence of the oligonucleotide be such that the oligonucleotide is composed of a morpholino nucleic acid represented by a formula (A4) below, and is a morpholino oligonucleic acid having a nucleotide sequence complementary to at least one target region having the same nucleotide length as the oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6:

[Formula 3]

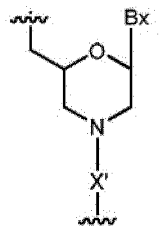

(A4)

wherein Bx is a nucleobase, being independently in each occurrence a group formed from adenine, guanine, cytosine, 5-methylcytosine, thymine, or uracil; and X' is a phosphate bond, being independently in each occurrence a phosphorodiamidate bond, a phosphorodiamidothioate bond, or a phosphorodiamidodithioate bond.

[14] It is preferable that the nucleotide sequence of the oligonucleotide be:

a nucleotide sequence having a sequence identity of 90% or more and 100% or less with a nucleotide sequence

complementary to a target region composed of continuous 15 to 25mer starting from a nucleotide at any of positions 1 to 40, 46, 51, 56, and 62 to 63 counted from a 5' end in a nucleotide sequence set forth in SEQ ID NO: 1 or a target region composed of continuous 15 to 25mer starting from a nucleotide at any of positions 108873, 108878 to 108917, 108923, 108928, 108933, and 108939 to 108940 counted from a 5' end in a nucleotide sequence set forth in SEQ ID NO: 2;

a nucleotide sequence complementary to a nucleotide sequence formed by deleting, substituting, inserting, or adding one or several nucleotides in the target region; or

a nucleotide sequence hybridizable with an oligonucleotide having the target region under stringent conditions.

[15] It is preferable that the nucleotide sequence of the oligonucleotide be a nucleotide sequence having a sequence identity of 90% or more and 100% or less with a nucleotide sequence complementary to a target region composed of continuous 15 to 25mer starting from a nucleotide at any of positions 1 to 32, 34 to 40, 46, 51, and 62 to 63 counted from a 5' end in a nucleotide sequence set forth in SEQ ID NO: 1 or a target region composed of continuous 15 to 25mer starting from a nucleotide at any of positions 108878 to 108909, 108911 to 108917, 108923, 108928, and 108939 to 108940 counted from 5' end in a nucleotide sequence set forth in SEQ ID NO: 2.

[16] It is preferable that the nucleotide sequence of the oligonucleotide be a nucleotide sequence having a sequence identity of 90% or more and 100% or less with a nucleotide sequence complementary to a target region composed of continuous 15 to 25mer starting from a nucleotide at any of positions 1, 3, 5 to 12, 14 to 19, 21, 25, 29, 30, 31, 34, 46, and 51 counted from a 5' end in a nucleotide sequence set forth in SEQ ID NO: 1 or a target region composed of continuous 15 to 25mer starting from a nucleotide at any of positions 108878, 108880, 108882 to 108889, 108891 to 108896, 108898, 108902, 108906 to 108908, 108911, 108923, and 108928 counted from a 5' end in a nucleotide sequence set forth in SEQ ID NO: 2.

[17] It is preferable that the nucleotide sequence of the oligonucleotide be one nucleotide sequence selected from the group consisting of nucleotide sequences set forth in SEQ ID NOs: 9 to 14, 16 to 26, 28 to 39, 41 to 42, 44 to 50, 52, 55 to 56, 69, 73, 80 to 105, 107 to 108, 110, 112, 114, 116, 118 to 131, 133, and 135.

[18] It is preferable that the oligonucleotide be one oligonucleotide selected from the group consisting of 6-20-A, 8-20-A, 8-20-B, 8-20-C, 9-20-A, 10-20-A, 12-20-A, 14-20-A, 16-20-A, 17-20-A, 18-20-A, 19-20-A, 15-25-A, 10-17-A, 10-20-B, 12-20-B, 14-20-B, 15-20-B, 16-20-B, 19-20-B, 31-20-B, 34-20-B, 6-20-B, 7-20-B, 8-20-D, and 9-20-B as sequence names shown in Table 2-1 to Table 2-7.

[19] It is preferable that the oligonucleotide be a single-stranded antisense oligonucleotide.

[20] A double-stranded antisense oligonucleotide according to the present invention is a double-stranded antisense oligonucleotide containing the oligonucleotide according to [19] and a second-strand oligonucleotide hybridizing with the single-stranded antisense oligonucleotide, or a pharmaceutically acceptable salt thereof, wherein

the nucleotide sequence of the second-strand oligonucleotide is a nucleotide sequence having a sequence identity of 90% or more and 100% or less with a nucleotide sequence complementary to the nucleotide sequence of the single-stranded antisense oligonucleotide.

[21] An oligonucleotide complex according to the present invention is an oligonucleotide complex or a pharmaceutically acceptable salt thereof, the oligonucleotide complex containing:

the oligonucleotide or a pharmaceutically acceptable salt thereof according to any one of [1] to [19], or the double-stranded antisense oligonucleotide or a pharmaceutically acceptable salt thereof according to [20]; and

an additive substance bound to the oligonucleotide or the second-strand oligonucleotide directly or via a linker bond, wherein

the additive substance is selected from the group consisting of polyethylene glycol, a peptide, an alkyl chain, a ligand compound, an antibody, a protein, and a sugar chain, and

the linker bond is independently in each occurrence a phosphodiester bond, a phosphorothioate bond, a phosphorodithioate bond, a phosphoamidate bond, a boranophosphate bond, an alkylphosphonate bond, a phosphorodiamidate bond, a phosphorodiamidothioate bond, or a phosphorodiamidodithioate bond.

[22] A pharmaceutical product according to the present invention is a pharmaceutical product containing the oligonucleotide or a pharmaceutically acceptable salt thereof according to any one of [1] to [19], the double-stranded antisense oligonucleotide or a pharmaceutically acceptable salt thereof according to [20], or the oligonucleotide complex or a pharmaceutically acceptable salt thereof according to [21] as an active ingredient.

[23] A skipping agent for the 27th exon of a human WRN gene according to the present invention is a skipping agent for the 27th exon of a human WRN gene, the skipping agent containing the oligonucleotide or a pharmaceutically acceptable salt thereof according to any one of [1] to [19], the double-stranded antisense oligonucleotide or a pharmaceutically acceptable salt thereof according to [20], or the oligonucleotide complex or a pharmaceutically acceptable salt thereof according to [21] as an active ingredient.

[24] A functional WRN recovery agent according to the present invention is a functional WRN recovery agent that possesses a nuclear localization signal by virtue of skipping of the 27th exon of a human WRN gene, the functional WRN recovery agent containing the oligonucleotide or a pharmaceutically acceptable salt thereof according to any one of [1] to [19], the double-stranded antisense oligonucleotide or a pharmaceutically acceptable salt thereof according to [20], or the oligonucleotide complex or a pharmaceutically acceptable salt thereof according to [21] as an active ingredient.

[25] A therapeutic agent for Werner syndrome according to the present invention is a therapeutic agent for Werner syndrome, the therapeutic agent containing the oligonucleotide or a pharmaceutically acceptable salt thereof according to any one of [1] to [19], the double-stranded antisense oligonucleotide or a pharmaceutically acceptable salt thereof according to [20], or the oligonucleotide complex or a pharmaceutically acceptable salt thereof according to [21] as an active ingredient.

[26] A prophylactic agent for Werner syndrome according to the present invention is a prophylactic agent for Werner syndrome, the prophylactic agent containing the oligonucleotide or a pharmaceutically acceptable salt thereof according to any one of [1] to [19], the double-stranded antisense oligonucleotide or a pharmaceutically acceptable salt thereof according to [20], or the oligonucleotide complex or a pharmaceutically acceptable salt thereof according to [21] as an active ingredient.

[27] A method for treating or preventing Werner syndrome according to the present invention is a method for treating or preventing Werner syndrome, the method including administering the oligonucleotide or a pharmaceutically acceptable salt thereof according to any one of [1] to [19], the double-stranded antisense oligonucleotide or a pharmaceutically acceptable salt thereof according to [20], or the oligonucleotide complex or a pharmaceutically acceptable salt thereof according to [21] to an individual.

[28] The present invention provides the oligonucleotide or a pharmaceutically acceptable salt thereof according to any one of [1] to [19], the double-stranded antisense oligonucleotide or a pharmaceutically acceptable salt thereof according to [20], or the oligonucleotide complex or a pharmaceutically acceptable salt thereof according to [21] for use in treating or preventing Werner syndrome.

[29] The present invention provides the oligonucleotide or a pharmaceutically acceptable salt thereof according to any one of [1] to [19], the double-stranded antisense oligonucleotide or a pharmaceutically acceptable salt thereof according to [20], or the oligonucleotide complex or a pharmaceutically acceptable salt thereof according to [21] for use in producing a therapeutic agent or prophylactic agent for Werner syndrome.

## ADVANTAGEOUS EFFECTS OF INVENTION

The present invention can provide an oligonucleotide that enables skipping of the 27th exon of a human WRN gene, and an agent that contains the oligonucleotide and allows the 27th exon of a human WRN gene to be skipped with high efficiency.

## BRIEF DESCRIPTION OF DRAWINGS

Fig. 1 is a schematic diagram illustrating the structure of a human WRN gene product.

Fig. 2 is a schematic diagram for describing the onset mechanism of Werner syndrome involving the skipping mutation c.3139-1G>C in the 26th exon.

Fig. 3 is a schematic diagram for describing the mechanism of skipping of exon 27 with a single-stranded antisense oligonucleotide according to the present embodiment.

Fig. 4 is a schematic diagram illustrating the relative position of an antisense oligonucleotide in exon 27 designed to induce skipping of WRN exon 27 to exon 27.

Fig. 5 is a photograph of electrophoresis showing skipping efficiency for exon 27 of a WRN gene in HEK293T.

Fig. 6 is a photograph of electrophoresis showing skipping activity for exon 27 of a WRN gene in Werner syndrome patient fibroblasts possessing the skipping mutation c.3139-1G>C in the 26th exon.

Fig. 7 is images of fluorescence microscopy showing skipping activity for exon 27 of a WRN gene in Werner syndrome patient fibroblasts possessing the skipping mutation c. 3 13 9-1 G>C in the 26th exon.

## DESCRIPTION OF EMBODIMENTS

«Oligonucleotide that allows 27th exon of human WRN gene to be skipped»

The oligonucleotide of the present embodiment is an oligonucleotide that expresses a functional human WRN

protein against a skipping mutation in the 26th or 28th exon of a human WRN gene, or a pharmaceutically acceptable salt thereof, wherein

one nucleotide is bound to another via a phosphate group and/or modified phosphate group in the oligonucleotide,
the oligonucleotide contains a modified nucleic acid having at least one modified sugar,
the oligonucleotide is 10 to 30mer in nucleotide length, and
the nucleotide sequence of the oligonucleotide is:

a nucleotide sequence having a sequence identity of 90% or more and 100% or less with a nucleotide sequence complementary to at least one target region having the same nucleotide length as the oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6;
a nucleotide sequence complementary to a nucleotide sequence formed by deleting, substituting, inserting, or adding one or several nucleotides in the target region; or
a nucleotide sequence hybridizable with an oligonucleotide having the target region under stringent conditions.
It is preferable that the oligonucleotide be a single-stranded antisense oligonucleotide. In the present embodiment, the term "modified nucleic acid having a modified sugar" encompasses morpholino nucleic acid described later.

The oligonucleotide having the configuration or a pharmaceutically acceptable salt thereof enables skipping of the 27th exon of a human WRN gene. In an aspect of the present embodiment, the oligonucleotide or a pharmaceutically acceptable salt thereof may be regarded as an "oligonucleotide that allows the 27th exon of a human WRN gene to be skipped or a pharmaceutically acceptable salt thereof". The present invention can ameliorate symptoms of Werner syndrome including refractory skin ulcer by inducing skipping of the 27th exon of a human WRN gene for an mRNA precursor with a displaced amino acid reading frame due to abnormal splicing caused by the skipping mutation c.3139-1G>C in the 26th exon to correct the displacement of the reading frame. The present invention can be applied not only to treatment of Werner syndrome patients possessing the skipping mutation c.3139-1G>C in the 26th exon, but also to treatment of those possessing, for example, any of the skipping mutation c.3233+1G>T in the 26th exon, the skipping mutation c.3233+1G>C in the 26th exon, and the skipping mutation c.IVS28+2T>C in the 28th exon. The details will be described below. The oligonucleotide according to the present embodiment is occasionally expressed as "the antisense oligonucleotide", "the single-stranded antisense oligonucleotide", or the like for convenience; however, this is not intended to limit the oligonucleotide according to the present embodiment to such an expression.

<Definition of terms, etc.>

First, definitions of terms used herein, etc., will be described.

(WRN gene)

In the present embodiment, a "WRN gene" can be defined according to Nature (1992) 355: 735-738 (NPL 2) and Am. J. Hum. Genet. (1997) 60: 330-341 (NPL 3).

(Single-stranded antisense oligonucleotide)

In the present embodiment, a "single-stranded antisense oligonucleotide" or "antisense oligonucleotide" (hereinafter, occasionally referred to as "ASO") refers to an oligonucleotide complementary to an mRNA, mRNA precursor, or ncRNA (noncoding RNA) (hereinafter, these three are occasionally referred to as "target RNA", collectively) for a target gene, or a pharmacologically acceptable salt thereof. An antisense oligonucleotide is composed of DNA, RNA, and/or an analog thereof. Antisense oligonucleotides suppress the functions of a targeted mRNA, mRNA precursor, or ncRNA by forming a duplex with the targeted mRNA, mRNA precursor, or ncRNA. Antisense oligonucleotides include those having a nucleotide sequence completely complementary to the nucleotide sequence of an mRNA, mRNA precursor, or ncRNA to be targeted, those having a nucleotide sequence formed by deleting, substituting, inserting, or adding one or several nucleotides in the complementary nucleotide sequence, and those containing bases forming a wobble base pair in the nucleotide sequence.

The antisense oligonucleotide of the present invention may further contain a modified nucleotide known in the art other than a "modified nucleic acid in which a sugar moiety is a modified sugar" (modified nucleotides with sugar modification) described later. Examples of the modified nucleotide known in the art include not only modified nucleotides with sugar modification, but also modified nucleotides with phosphate group modification and modified nucleotides with

nucleobase modification, which will be described later.

The structure of each end of the antisense oligonucleotide in the present embodiment is not limited, and may be, for example, -OH or -OR (wherein R represents an alkyl chain, a phosphate form, or an additive substance described later).

The single-stranded antisense oligonucleotide in the present embodiment may be in a single-stranded form, or take a double-stranded form through hybridizing with a second-strand oligonucleotide described later. A double-stranded oligonucleotide consisting of the single-stranded antisense oligonucleotide and a second-strand oligonucleotide hybridizing with the single-stranded antisense oligonucleotide is occasionally referred to as a "double-stranded antisense oligonucleotide".

(Oligonucleotide)

In the present embodiment, an "oligonucleotide" refers to a polymer of nucleotides in which 2 to 30 identical or different nucleotides are linked via phosphoric diester bonds or bonds of another type. The oligonucleotide may be regarded as being composed of a nucleobase moiety, a phosphate moiety, and a sugar moiety or a morpholino ring moiety, as illustrated by the following structural formula.

[Formula 4]

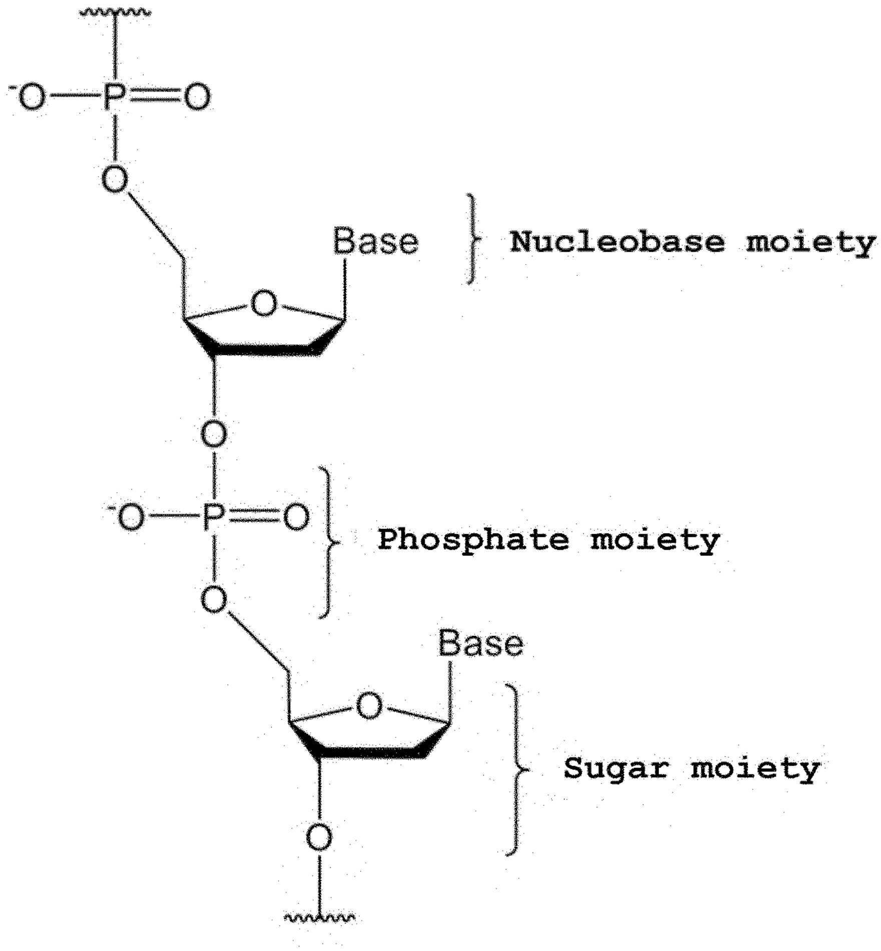


[Formula 5]

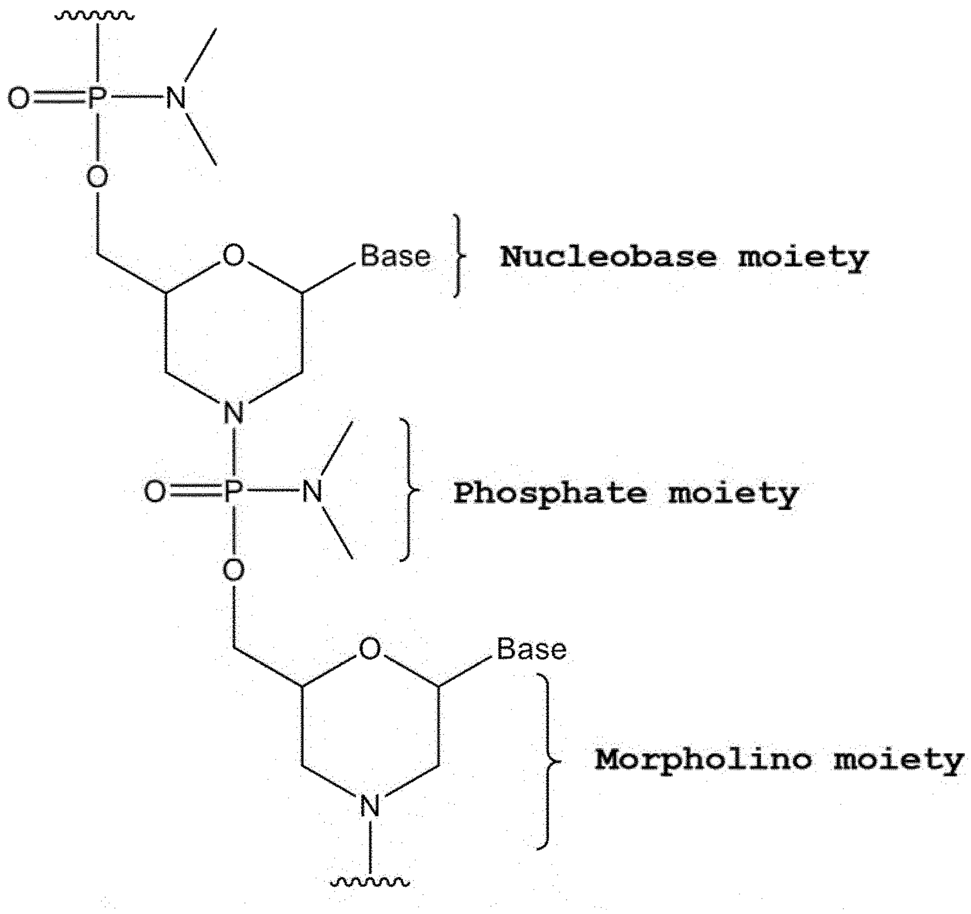


The oligonucleotides are roughly classified into natural oligonucleotides and non-natural oligonucleotides. A "natural oligonucleotide" refers to an oligonucleotide consisting of naturally occurring nucleotides. A "non-natural oligonucleotide" refers to an oligonucleotide containing at least one modified nucleotide, described later, as a constituent unit. Preferred examples of "non-natural oligonucleotides" include modified-sugar derivatives in which a sugar moiety is modified; phosphorothioate derivatives in which one of the non-crosslinking oxygen atoms of a phosphoric diester bond is replaced with a sulfur atom; phosphorodithioate derivatives in which the two non-crosslinking oxygen atoms of a phosphoric diester bond are replaced with sulfur atoms; ester derivatives in which a phosphoric diester bond is triesterified; phosphoamide derivatives in which a phosphoric diester bond is amidated; boranophosphate derivatives in which a phosphoric diester bond is boronated; alkylphosphonate (e.g., methylphosphonate, methoxypropylphosphonate) derivatives in which a non-crosslinking oxygen atom in a phosphoric diester bond is substituted with an alkyl group; amide derivatives in which a phosphoric diester bond is replaced with an amide bond; and modified-base derivatives in which a nucleobase is modified. Further preferred examples of the non-natural oligonucleotides include crosslinked modified-sugar derivatives in which a sugar moiety is modified; phosphorothioate derivatives in which one of the non-crosslinking oxygen atoms of a phosphoric diester bond is replaced with a sulfur atom; ester derivatives in which a phosphoric diester bond is esterified; and derivatives in which a sugar moiety is modified to form a modified sugar described later (e.g., a crosslinked sugar), and one of the non-crosslinking oxygen atoms of a phosphoric diester bond is replaced with a sulfur atom or a non-crosslinked oxygen atom of a phosphoric diester bond is substituted with an alkyl group to form an alkylphosphonate.

(Nucleoside)

In the present embodiment, a "nucleoside" refers to a compound in which a purine base or pyrimidine base and a sugar are bonding together. A naturally occurring nucleoside is occasionally referred to as a "natural nucleoside". A not naturally occurring modified nucleoside is occasionally referred to as a "modified nucleoside". In particular, a modified nucleoside in which the sugar moiety is modified is occasionally referred to as a "modified-sugar nucleoside".

(Nucleotide)

In the present embodiment, a "nucleotide" refers to a compound in which a phosphate group is bonding to the sugar of the nucleoside. A naturally occurring nucleotide is occasionally referred to as a "natural nucleotide". Not naturally occurring, modified nucleotides are occasionally referred to as "modified nucleotides" or a "modified nucleic acid". Examples of the "modified nucleotides" or "modified nucleic acid" include compounds in which a phosphate group is bonding to the sugar moiety of any of the modified nucleosides, and compounds in which a modified phosphate group described later is bonding to the sugar moiety of a natural nucleoside.

(Sugar modification, modified sugar)

In the present embodiment, "sugar modification" means that the sugar moiety of the nucleotide is modified. A modified sugar moiety is occasionally referred to as a "modified sugar". Modified nucleotides subjected to sugar modification are applicable to a modified nucleic acid, and examples thereof include 2'-O-alkylated nucleic acid, 2'-F-nucleic acid, 5'-methylated nucleic acid, 2',4'-BNA (bridged nucleic acid, hereinafter, occasionally referred to as "LNA"), AmNA (amide-crosslinked artificial nucleic acid, amido-bridged nucleic acid), GuNA (guanidine-crosslinked artificial nucleic acid, guanidino-bridged nucleic acid), scpBNA (2'-O,4'-C-spirocyclopropylene-bridged nucleic acid), ENA (2'-O,4'-C-ethylene-bridged nucleic acid), and S-cEt (2',4'-constrained ethyl nucleic acid).

Examples of the 2'-O-alkylated nucleic acid include those including structures denoted by the symbols "A(M)", "A(m)", "C(M)", "5(m)", "G(M)", "G(m)", "U(M)", and "T(m)" shown later. Examples of the LNA include those including structures denoted by the symbols "A(L)", "5(L)", "G(L)", and "T(L)" shown later. Examples of the AmNA include those including structures denoted by the symbols "A(Y)", "5(Y)", "G(Y)", and "T(Y)" shown later. Examples of the GuNA include those including structures denoted by the symbols "A(Gx)", "5(Gx)", "G(Gx)", and "T(Gx)" shown later. Examples of the scpBNA include those including structures denoted by the symbols "A(S)", "5(S)", "G(S)", and "T(S)" shown later.

In an aspect of the present embodiment, it is preferable that modification of a sugar constituting the oligonucleotide be made to form a group represented by a formula (A1) below. In another aspect of the present embodiment, a modified sugar constituting the oligonucleotide may be regarded as being represented by the following formula (A1).

[0057] [Formula 6]

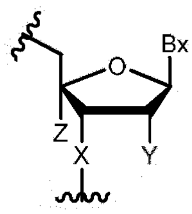

(A1)

In the formula, Bx is a nucleobase, being independently in each occurrence a group formed from adenine, guanine, cytosine, 5-methylcytosine, thymine, or uracil; X is a phosphate bond, being independently in each occurrence a phosphodiester bond, a phosphorothioate bond, a phosphorodithioate bond, a phosphoamidate bond, a boranophosphate bond, or an alkylphosphonate bond; Y is independently in each occurrence a hydrogen atom, a hydroxy group, a fluorine atom, an optionally substituted alkoxy group having one to six carbon atoms, or an optionally substituted amino group; and Z is independently in each occurrence a hydrogen atom, or an alkyl group having one to five carbon atoms and optionally having a carbon-oxygen double bond or a cyclic structure, or a certain carbon atom in the alkyl group and Y are taken together to form a ring. Here, the "alkyl group having one to five carbon atoms" may be regarded as a monovalent group formed by a certain carbon atom of an alkylene group having one to five carbon atoms and a hydrogen atom together.

Examples of the "optionally substituted alkoxy group having one to six carbon atoms" include unsubstituted alkoxy groups having one to six carbon atoms, and alkoxy groups having one to six carbon atoms and substituted with a substituent selected from the group consisting of a hydroxy group, a fluorine atom, a linear or branched alkoxy group having one to six carbon atoms, a cyclic alkoxy group having three to eight carbon atoms, a carbamoyl group, and an alkylamide group having one to eight carbon atoms (a group represented by $-(CO)-NR^7R^8$). Here, $R^7$ and $R^8$ each independently represent a hydrogen atom, or a linear or branched alkyl group having one to eight carbon atoms.

Examples of the "optionally substituted amino group" include an unsubstituted amino group, and amino groups substituted with a substituent selected from the group consisting of a linear or branched alkyl group having one to six carbon atoms (the alkyl group is optionally substituted with a linear or branched alkoxy group having one to six carbon atoms, or a group represented by $-(CO)-NR^9R^{10}$), a cyclic alkyl group having three to eight carbon atoms, an acyl group

having one to six carbon atoms, and an amidine group substituted at one to three positions with a linear or branched alkyl group having one to six carbon atoms. Here, $R^9$ and $R^{10}$ each independently represent a hydrogen atom, or a linear or branched alkyl group having one to six carbon atoms.

Examples of the "alkyl group having one to five carbon atoms and optionally having a carbon-oxygen double bond or a cyclic structure" include unsubstituted alkyl groups having one to five carbon atoms, alkyl groups having one to five carbon atoms with a carbon-oxygen double bond, and cycloalkyl groups having three to five carbon atoms (such as a cyclopropyl group). Here, the number of carbon atoms in the "alkyl groups having one to five carbon atoms with a carbon-oxygen double bond" may include the carbon atom constituting the carbon-oxygen double bond. Accordingly, in the present embodiment, the "alkyl groups having one to five carbon atoms with a carbon-oxygen double bond" can include an aldehyde group. In the case that Z is an aldehyde group and Y is an optionally substituted amino group, the ring formed by Y and Z together may have a structure including an amide bond.

In an aspect of the present embodiment, it is preferable that modification of a sugar constituting the oligonucleotide be made to form a group represented by a formula (A2) or formula (A3) below. In another aspect of the present embodiment, a modified sugar constituting the oligonucleotide may be regarded as being represented by the following formula (A2) or formula (A3).

[0063] [Formula 7]

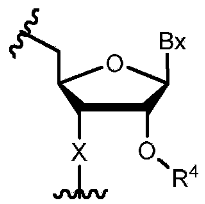

(A2)

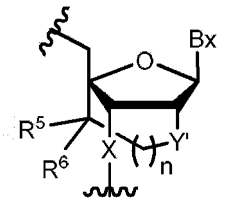

(A3)

In the formulas, Bx is a nucleobase, being independently in each occurrence a group formed from adenine, guanine, cytosine, 5-methylcytosine, thymine, or uracil; X is a phosphate bond, being independently in each occurrence a phosphodiester bond, a phosphorothioate bond, a phosphorodithioate bond, a phosphoamidate bond, a boranophosphate bond, or an alkylphosphonate bond; $R^4$ is independently in each occurrence a hydrogen atom or an optionally substituted alkyl group having one to six carbon atoms; Y' is an oxygen atom or an optionally substituted nitrogen atom; $R^5$ and $R^6$ are each independently in each occurrence a hydrogen atom or an optionally substituted alkyl group having one to six carbon atoms, or $R^5$ and $R^6$ are taken together to form a carbonyl group or a ring; and n is 0 or 1.

In another aspect of the present embodiment, it is preferable that modification of a sugar constituting the oligonucleotide be made to form a group represented by the formula (A2), and the $R^4$ be independently in each occurrence a methyl group, a methoxyethyl group, or an N-methylpropanamide group ($-CH_2CH_2-CONH-CH_3$, a methyliminocarbonylethyl group).

In another aspect of the present embodiment, it is preferable that modification of a sugar constituting the oligonucleotide be made to form a group represented by the formula (A3), the Y' be an oxygen atom, or a nitrogen atom optionally substituted with a hydrogen atom, a methyl group, a methoxyethyl group, or an N-methylpropanamide group, the $R^5$ and $R^6$ be each independently in each occurrence a hydrogen atom, an alkyl group having one or two carbon atoms, or the $R^5$ and $R^6$ be taken together to form a carbonyl group or a ring having three to six carbon atoms, and n be 0 or 1.

(Modification of nucleotide known in the art other than sugar modification)

Modification of a nucleotide known in the art other than the sugar modification is applicable to a modified nucleic acid for producing the single-stranded antisense oligonucleotide of the present invention. Known as modification of a nucleotide are phosphate group modification and nucleobase modification described later. Examples of such modifications of a nucleotide include modification of nucleotides described in J. Med. Chem. (2016) 59: 9645-9667 (NPL 5). Each

of those modifications of a nucleotide can be performed according to a method known in the art, which is described in a literature cited in that literature.

(Phosphate group)

In the present embodiment, a "phosphate group" refers to such a group that the binding mode of the phosphate moiety of the nucleotide is a naturally occurring phosphodiester bond (a bond denoted by the symbol "-" shown later).

(Phosphate group modification, modified phosphate group)

In the present embodiment, "phosphate group modification" means that the phosphate moiety of the nucleotide is modified. In particular, a modified phosphate moiety is occasionally referred to as a "modified phosphate group". Examples of the binding mode including the modified phosphate group include a phosphorothioate bond (a bond denoted by the symbol "^" shown later), a phosphorodithioate bond, a phosphoamidate bond, or a boranophosphate bond, an alkylphosphonate bond, a phosphorodiamidate bond (a bond denoted by the symbol "*" shown later), a phosphorodiamidothioate bond, and a phosphorodiamidodithioate bond.

(Nucleobase modification, modified nucleobase)

In the present embodiment, "nucleobase modification" means that a nucleobase moiety of the nucleotide is modified. In particular, a modified nucleobase moiety is occasionally referred to as a "modified nucleobase". Examples of the modified nucleobase include 5-methylcytosine, 5-hydroxymethylcytosine, and 5-propynylcytosine.

(Analog of DNA or RNA)

The analog of DNA or RNA refers to a molecule having a structure similar to that of DNA or RNA. Examples thereof include peptide nucleic acid (pNA) and morpholino nucleic acid.

For the nucleic acid to be used in the present invention, not only nucleic acids with in which a sugar moiety is modified but also morpholino nucleic acids and peptide nucleic acids may be used.

Thus, in another mode of the single-stranded antisense oligonucleotide of the present invention, the oligonucleotides corresponding to the sequences listed in the column <Nucleotide sequence of single-stranded antisense oligonucleotide> shown later may be each composed of an analog of DNA or RNA. Such an analog of DNA or RNA at least contains a peptide nucleic acid or morpholino nucleic acid. Synthesis of the antisense oligonucleotide of the present invention containing a peptide nucleic acid or morpholino nucleic acid is performed according to a common, conventional method. For example, a morpholino oligonucleic acid can be produced according to WO 1991/009033 (PTL 4), WO 2009/064471 (PTL 5), or J. Am. Chem. Soc (2016) 138: 15663-15672 (NPL 8). Examples of the morpholino nucleic acid include those including structures denoted by the symbols "A(N)", "C(N)", "G(N)", and "T(N)" shown later.

In an aspect of the present embodiment, it is preferable that the oligonucleotide be a morpholino oligonucleic acid composed of a morpholino nucleic acid represented by the following formula (A4).

[0073] [Formula 8]

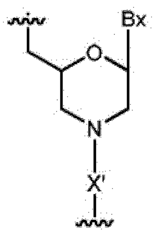

(A4)

In the formula, Bx is a nucleobase, being independently in each occurrence a group formed from adenine, guanine, cytosine, 5-methylcytosine, thymine, or uracil; and X' is a phosphate bond, being independently in each occurrence a phosphorodiamidate bond, a phosphorodiamidothioate bond, or a phosphorodiamidodithioate bond.

(ncRNA)

In the present embodiment, "ncRNA" is a collective term for RNA not involved in translation of proteins. Examples

of the ncRNA include ribosomal RNA, transfer RNA, miRNA, and natural antisense transcripts (NAT).

(Nucleobase moieties of oligonucleotide)

Examples of nucleobase moieties of the oligonucleotide include a thyminyl group, a cytosinyl group, an adeninyl group, a guaninyl group, a 5-methylcytosinyl group, a uracilyl group, a 2-oxo-4-hydroxy-5-methyl-1,2-dihydropyrimidin-1-yl group, a 2-oxo-4-amino-1,2-dihydropyrimidin-1-yl group, a 4-amino-5-methyl-2-oxo-1,2-dihydropyrimidin-1-yl group, and a 2-oxo-4-hydroxy-1,2-dihydropyrimidin-1-yl group. Preferred examples of the nucleobase moieties include a thyminyl group, a cytosinyl group, an adeninyl group, a guaninyl group, a 5-methylcytosinyl group, and a uracilyl group. Among the nucleobases, uracil (U) and thymine (T) are compatible with each other. Both uracil (U) and thymine (T) are capable of forming a base pair with adenine (A) in the complementary strand. The same also applies to nucleobase moieties of the antisense oligonucleotide.

(Target RNA)

In the present embodiment, "target RNA" refers to RNA to which the single-stranded antisense oligonucleotide is to bind. In other words, the target RNA in the present embodiment refers to an mRNA precursor for a WRN gene. Examples of the target RNA include an mRNA precursor having a nucleotide sequence set forth in SEQ ID NO: 2 for a human WRN gene, an mRNA precursor having a nucleotide sequence set forth in any of SEQ ID NOs: 3 to 5 for a human WRN gene with an exon 26 skip mutation, and an mRNA precursor having a nucleotide sequence set forth in SEQ ID NO: 6 for a human WRN gene with an exon 28 skip mutation.

(Binding to target RNA)

In the present embodiment, "binding to target RNA" means that nucleobases of a single-stranded antisense oligonucleotide form a double-stranded nucleic acid together with nucleobases of target RNA through the complementarity to the target RNA. It is enough for the double-stranded nucleic acid to be formed in at least part of the target RNA. The strength of binding to the target RNA can be measured, for example, on the basis of an indicator of thermal stability. Examples of the indicator of thermal stability include the melting temperature (Tm value) of the double-stranded nucleic acid. The Tm value is preferably 40 to 90°C, and more preferably 50 to 70°C.

(Target region)

The target region refers to a region of an mRNA precursor for a WRN gene where binding to the single-stranded antisense oligonucleotide occurs. Such target regions include target regions each consisting of a nucleotide sequence listed, and regions on an mRNA precursor for WRN.

(mRNA precursor)

The mRNA precursor refers to a primary transcript of RNA transcribed from DNA. That is, the mRNA precursor is RNA containing exon regions, intron regions, and untranslated regions (UTR). The mRNA precursor may be regarded as RNA before being subjected to post-transcriptional splicing. After being spliced, the mRNA precursor becomes mRNA.

(Binding to target region)

Binding to the target region means that the single-stranded antisense oligonucleotide of the present invention forms a duplex with the target region. However, the single-stranded antisense oligonucleotide of the present invention does not always need to form a duplex with the whole target region, and it is enough for the single-stranded antisense oligonucleotide of the present invention to form a duplex with a partial region of the target region. Thus, it is preferable that the single-stranded antisense oligonucleotide of the present invention have complete complementarity to the target region, but it is enough for the single-stranded antisense oligonucleotide of the present invention to be complementary to at least a partial region of the target region, to the extent that the single-stranded antisense oligonucleotide of the present invention binds to the target region of WRN.

(Part of target region)

A part of the target region refers to a region of 10 to 15 nucleotides in nucleotide length in the target region.

(Being complementary to at least part of target region)

Being complementary to at least part of the target region means being complementary to nucleotides in at least a partial region of the target region on the target RNA. Here, such situation also includes being complementary to nucleotides in a region on the mRNA precursor corresponding to at least the partial region.

The present inventors focused on splicing-switching antisense oligonucleotides, and examined to discover an antisense oligonucleotide that induces skipping of exon 27 of a human WRN gene.

<Design of single-stranded antisense oligonucleotide>

The single-stranded antisense oligonucleotide is a compound that enables restoration of an abnormalized reading frame in abnormal RNA, for example, due to frameshift, and is for allowing an exon as a target region to be skipped. Accordingly, the structure of the antisense oligonucleotide is designed so as to bind to the target region of target RNA, and, in addition, a sugar moiety-modified nucleic acid is disposed so as not to be recognized by nucleases such as RNase H.

<Nucleotide sequence of single-stranded antisense oligonucleotide>

It is preferable that the nucleotide sequence of the single-stranded antisense oligonucleotide according to the present embodiment be:

(A) a nucleotide sequence having a sequence identity of 90% or more and 100% or less with a nucleotide sequence complementary to a target region composed of continuous 10 to 30mer (preferably 15 to 25mer) starting from a nucleotide at any of positions 1 to 40, 46, 51, 56, and 62 to 63 counted from a 5' end in a nucleotide sequence set forth in SEQ ID NO: 1 or a target region composed of continuous 15 to 25mer starting from a nucleotide at any of positions 108873, 108878 to 108917, 108923, 108928, 108933, and 108939 to 108940 counted from a 5' end in a nucleotide sequence set forth in SEQ ID NO: 2;
(B) a nucleotide sequence complementary to a nucleotide sequence formed by deleting, substituting, inserting, or adding one or several nucleotides in the target region; or
(C) a nucleotide sequence hybridizable with an oligonucleotide having the target region under stringent conditions.

In the present embodiment, nucleotide sequences listed in sequence listings are used for showing only sequence information on nucleobase moieties. Structural information on oligonucleotides including sugar moieties and phosphate moieties in addition to nucleobase moieties is shown in a description format shown later in Table 2-1 to Table 2-7.

In the present embodiment, "sequence identity" refers to the proportion (%) of identical nucleotides in the whole overlapping nucleotide sequence in an optimum alignment when two nucleotide sequences are aligned by using a mathematical algorithm known in the art (preferably, the algorithm is capable of considering introduction of a gap into one or both of the sequences for optimum alignment). Those skilled in the art can check the "sequence identity" of a nucleotide sequence with ease. For example, NCBI BLAST (National Center for Biotechnology Information Basic Local Alignment Search Tool) can be used.

The nucleotide sequence of the single-stranded antisense oligonucleotide according to the present embodiment preferably has a sequence identity of 95% or more and 100% or less, more preferably has a sequence identity of 98% or more and 100% or less, further preferably has a sequence identity of 100%, with a nucleotide sequence complementary to the specific target region described above in a nucleotide sequence set forth in SEQ ID NO: 1.

In the present embodiment, the "nucleotide sequence formed by deleting, substituting, inserting, or adding one or several nucleotides" is, for example, a nucleotide sequence provided by the deletion, substitution, insertion, or addition with a sequence identity of 80% or more, 85% or more, 90% or more, 95% or more, 97% or more, 98% or more, or 99% or more with the nucleotide sequence before the deletion, substitution, insertion, or addition. Regarding the specific number of "one or several nucleotides", the deletion, substitution, insertion, and addition may occur each independently or in any combination at one position, two positions, three positions, four position, or five positions.

In the present embodiment, "stringent conditions" are such conditions that incubation is performed in a solution containing 6 × SSC (composition of 1 × SSC: 0.15 M NaCl, 0.015 M sodium citrate, pH 7.0), 0.5% SDS, 5 × Denhardt's solution, 100 μg/mL modified salmon sperm DNA, and 50% (v/v) formamide at room temperature for 12 hours, and washing is further performed with 0.5 × SSC at a temperature of 50°C or more. Furthermore included are more stringent conditions, for example, more severe conditions such as incubating at 45°C or 60°C for 12 hours, washing with 0.2 × SSC or 0.1 × SSC, and washing under a temperature condition of 60°C or 65°C or more in washing.

In an aspect of the present embodiment, it is preferable that the nucleotide sequence of the single-stranded antisense oligonucleotide be a nucleotide sequence complementary to a target region composed of continuous 15 to

25mer starting from a nucleotide at any of positions 1 to 40, 46, 51, 56, and 62 to 63 counted from a 5' end in a nucleotide sequence set forth in SEQ ID NO: 1 or a target region composed of continuous 15 to 25mer starting from a nucleotide at any of positions 108873, 108878 to 108917, 108923, 108928, 108933, and 108939 to 108940 counted from a 5' end in a nucleotide sequence set forth in SEQ ID NO: 2.

In an aspect of the present embodiment, it is more preferable that the nucleotide sequence of the single-stranded antisense oligonucleotide be a nucleotide sequence complementary to a target region composed of continuous 15 to 25mer starting from a nucleotide at any of positions 1 to 32, 34 to 40, 46, 51, and 62 to 63 counted from a 5' end in a nucleotide sequence set forth in SEQ ID NO: 1 or a target region composed of continuous 15 to 25mer starting from a nucleotide at any of positions 108878 to 108909, 108911 to 108917, 108923, 108928, and 108939 to 108940 counted from a 5' end in a nucleotide sequence set forth in SEQ ID NO: 2.

In an aspect of the present embodiment, it is further preferable that the nucleotide sequence of the single-stranded antisense oligonucleotide be a nucleotide sequence complementary to a target region composed of continuous 15 to 25mer starting from a nucleotide at any of positions 1, 3, 5 to 12, 14 to 19, 21, 25, 29, 30, 31, 34, 46, and 51 counted from a 5' end in a nucleotide sequence set forth in SEQ ID NO: 1 or a target region composed of continuous 15 to 25mer starting from a nucleotide at any of positions 108878, 108880, 108882 to 108889, 108891 to 108896, 108898, 108902, 108906 to 108908, 108911, 108923, and 108928 counted from a 5' end in a nucleotide sequence set forth in SEQ ID NO: 2.

The single-stranded antisense oligonucleotide is capable of binding to the target region of a WRN gene. Herein, "binding to the target region of a WRN gene" for the single-stranded antisense oligonucleotide of the present invention encompasses direct binding of the single-stranded antisense oligonucleotide of the present invention to an mRNA precursor for WRN.

A mode of the single-stranded antisense oligonucleotide of the present invention is a single-stranded antisense oligonucleotide that allows exon 27 of a WRN gene to be skipped and has any nucleotide sequence shown in Table 1-1 to Table 1-4, wherein the single-stranded oligonucleotide is complementary to a target region in an mRNA precursor for a human WRN gene.

The single-stranded antisense oligonucleotide may have an extension of one to five nucleotides in either one or each of the 3' side and the 5' side as long as the single-stranded antisense oligonucleotide contains a nucleotide sequence shown in Table 1-1 to Table 1-4. The target region can be said to be a region associated particularly with regulation of expression of a human WRN gene (e.g., a region having an mRNA secondary structure that allows the antisense nucleotide to bind thereto with ease) in an mRNA precursor for human WRN. In the case that Position of 5' end in SEQ ID NO: 1 is "5" and Position of 3' end therein is "24" in Table 1-1, for example, a nucleotide sequence of continuous 20mer from the 5th to the 24th counted from the 5' end in the nucleotide sequence set forth in SEQ ID NO: 1 (corresponding to exon 27 in SEQ ID NO: 2) is the target region in exon 27 for human WRN targeted by the corresponding single-stranded antisense oligonucleotide (Sequence name: "5-20"). In the case that Position of 5' end in SEQ ID NO: 1 is "(-)5" and Position of 3' end therein is "15" in Table 1-1, a nucleotide sequence of 20mer from the 5th nucleotide being in an intron region that binds to the 5' end and counted from the position that binds to exon 27 to the 15th in exon 27 in the nucleotide sequence set forth in SEQ ID NO: 1 (corresponding to exon 27 in SEQ ID NO: 2) is the target region in in exon 27 for human WRN targeted by the corresponding single-stranded antisense oligonucleotide (Sequence name: "(-)5-20").

[Table 1-1]

| Sequence name | Nucleotide sequence (5'-3') of antisense oligonucleotide | Target region of SEQ ID NO: 1 | | Target region of SEQ ID NO: 2 | |
|---|---|---|---|---|---|
| | | Position of 5' end | Position of 3' end | Position of 5' end | Position of 3' end |
| (-)10-20 | A'G'T'T'T'C'G'A'A'C'T'A'A'A'A'A'A'T'A' | -10 | 10 | 108868 | 108887 |
| (-)5-20 | G'A'T'A'C'A'G'T'T'T'C'G'A'A'C'T'A'A'A' | -5 | 15 | 108873 | 108892 |
| 1-20 | C'C'G'A'A'G'A'T'A'C'A'G'T'T'T'T'C'G'A'A' | 1 | 20 | 108878 | 108897 |
| 2-20 | C'C'C'G'A'A'G'A'T'A'C'A'G'T'T'T'T'C'G'A' | 2 | 21 | 108879 | 108898 |
| 3-20 | G'C'C'C'G'A'A'G'A'T'A'C'A'G'T'T'T'T'C'G' | 3 | 22 | 108880 | 108899 |
| 4-20 | T'G'C'C'C'G'A'A'G'A'T'A'C'A'G'T'T'T'T'C' | 4 | 23 | 108881 | 108900 |
| 5-20 | G'T'G'C'C'C'G'A'A'G'A'T'A'C'A'G'T'T'T'T' | 5 | 24 | 108882 | 108901 |
| 6-20 | G'G'T'G'C'C'C'G'A'A'G'A'T'A'C'A'G'T'T'T' | 6 | 25 | 108883 | 108902 |

(continued)

| Sequence name | Nucleotide sequence (5'-3') of antisense oligonucleotide | Target region of SEQ ID NO: 1 | | Target region of SEQ ID NO: 2 | |
|---|---|---|---|---|---|
| | | Position of 5' end | Position of 3' end | Position of 5' end | Position of 3' end |
| 7-20 | T'G'G'T'G'C'C'C'G'A'A'G'A'T'A'C'A'G'T'T' | 7 | 26 | 108884 | 108903 |
| 8-20 | T'T'G'G'T'G'C'C'C'G'A'A'G'A'T'A'C'A'G'T' | 8 | 27 | 108885 | 108904 |
| 9-20 | T'T'T'G'G'T'G'C'C'C'G'A'A'G'A'T'A'C'A'G' | 9 | 28 | 108886 | 108905 |
| 10-20 | C'T'T'T'G'G'T'G'C'C'C'G'A'A'G'A'T'A'C'A' | 10 | 29 | 108887 | 108906 |
| 11-20 | T'C'T'T'T'G'G'T'G'C'C'C'G'A'A'G'A'T'A'C' | 11 | 30 | 108888 | 108907 |
| 12-20 | C'T'C'T'T'T'G'G'T'G'C'C'C'G'A'A'G'A'T'A' | 12 | 31 | 108889 | 108908 |
| 13-20 | G'C'T'C'T'T'T'G'G'T'G'C'C'C'G'A'A'G'A'T' | 13 | 32 | 108890 | 108909 |
| 14-20 | T'G'C'T'C'T'T'T'G'G'T'G'C'C'C'G'A'A'G'A' | 14 | 33 | 108891 | 108910 |
| 15-20 | A'T'G'C'T'C'T'T'T'G'G'T'G'C'C'C'G'A'A'G' | 15 | 34 | 108892 | 108911 |
| 16-20 | A'A'T'G'C'T'C'T'T'T'G'G'T'G'C'C'C'G'A'A' | 16 | 35 | 108893 | 108912 |
| 17-20 | C'A'A'T'G'C'T'C'T'T'T'G'G'T'G'C'C'C'G'A' | 17 | 36 | 108894 | 108913 |
| 18-20 | A'C'A'A'T'G'C'T'C'T'T'T'G'G'T'G'C'C'C'G' | 18 | 37 | 108895 | 108914 |
| 19-20 | A'A'C'A'A'T'G'C'T'C'T'T'T'G'G'T'G'C'C'C' | 19 | 38 | 108896 | 108915 |
| 20-20 | T'A'A'C'A'A'T'G'C'T'C'T'T'T'G'G'T'G'C'C' | 20 | 39 | 108897 | 108916 |
| 21-20 | A'T'A'A'C'A'A'T'G'C'T'C'T'T'T'G'G'T'G'C' | 21 | 40 | 108898 | 108917 |
| 22-20 | T'A'T'A'A'C'A'A'T'G'C'T'C'T'T'T'G'G'T'G' | 22 | 41 | 108899 | 108918 |
| 23-20 | T'T'A'T'A'A'C'A'A'T'G'C'T'C'T'T'T'G'G'T' | 23 | 42 | 108900 | 108919 |

[Table 1-2]

| Sequence name | Nucleotide sequence (5'-3') of antisense oligonucleotide | Target region of SEQ ID NO: 1 | | Target region of SEQ ID NO: 2 | |
|---|---|---|---|---|---|
| | | Position of 5' end | Position of 3' end | Position of 5' end | Position of 3' end |
| 24-20 | A'T'T'A'T'A'A'C'A'A'T'G'C'T'C'T'T'T'G'G' | 24 | 43 | 108901 | 108920 |
| 25-20 | G'A'T'T'A'T'A'A'C'A'A'T'G'C'T'C'T'T'T'G' | 25 | 44 | 108902 | 108921 |
| 26-20 | T'G'A'T'T'A'T'A'A'C'A'A'T'G'C'T'C'T'T'T' | 26 | 45 | 108903 | 108922 |
| 27-20 | T'T'G'A'T'T'A'T'A'A'C'A'A'T'G'C'T'C'T'T' | 27 | 46 | 108904 | 108923 |
| 28-20 | C'T'T'G'A'T'T'A'T'A'A'C'A'A'T'G'C'T'C'T' | 28 | 47 | 108905 | 108924 |
| 29-20 | A'C'T'T'G'A'T'T'A'T'A'A'C'A'A'T'G'C'T'C' | 29 | 48 | 108906 | 108925 |
| 30-20 | T'A'C'T'T'G'A'T'T'A'T'A'A'C'A'A'T'G'C'T' | 30 | 49 | 108907 | 108926 |
| 31-20 | G'T'A'C'T'T'G'A'T'T'A'T'A'A'C'A'A'T'G'C' | 31 | 50 | 108908 | 108927 |
| 32-20 | G'G'T'A'C'T'T'G'A'T'T'A'T'A'A'C'A'A'T'G' | 32 | 51 | 108909 | 108928 |
| 33-20 | T'G'G'T'A'C'T'T'G'A'T'T'A'T'A'A'C'A'A'T' | 33 | 52 | 108910 | 108929 |
| 34-20 | C'T'G'G'T'A'C'T'T'G'A'T'T'A'T'A'A'C'A'A' | 34 | 53 | 108911 | 108930 |
| 35-20 | A'C'T'G'G'T'A'C'T'T'G'A'T'T'A'T'A'A'C'A' | 35 | 54 | 108912 | 108931 |
| 36-20 | A'A'C'T'G'G'T'A'C'T'T'G'A'T'T'A'T'A'A'C' | 36 | 55 | 108913 | 108932 |

(continued)

| Sequence name | Nucleotide sequence (5'-3') of antisense oligonucleotide | Target region of SEQ ID NO: 1 | | Target region of SEQ ID NO: 2 | |
|---|---|---|---|---|---|
| | | Position of 5' end | Position of 3' end | Position of 5' end | Position of 3' end |
| 37-20 | C'A'A'C'T'G'G'T'A'C'T'T'G'A'T'T'A'T'A'A' | 37 | 56 | 108914 | 108933 |
| 38-20 | T'C'A'A'C'T'G'G'T'A'C'T'T'G'A'T'T'A'T'A' | 38 | 57 | 108915 | 108934 |
| 39-20 | T'T'C'A'A'C'T'G'G'T'A'C'T'T'G'A'T'T'A'T' | 39 | 58 | 108916 | 108935 |
| 40-20 | A'T'T'C'A'A'C'T'G'G'T'A'C'T'T'G'A'T'T'A' | 40 | 59 | 108917 | 108936 |
| 41-20 | A'A'T'T'C'A'A'C'T'G'G'T'A'C'T'T'G'A'T'T' | 41 | 60 | 108918 | 108937 |
| 46-20 | T'A'C'T'T'A'A'T'T'C'A'A'C'T'G'G'T'A'C'T' | 46 | 65 | 108923 | 108942 |
| 51-20 | C'T'C'T'G'T'A'C'T'T'A'A'T'T'C'A'A'C'T'G' | 51 | 70 | 108928 | 108947 |
| 56-20 | T'T'C'T'T'C'T'C'T'G'T'A'C'T'T'A'A'T'T'C' | 56 | 75 | 108933 | 108952 |
| 62-20 | C'A'A'A'C'C'T'T'C'T'T'C'T'C'T'G'T'A'C'T' | 62 | 81 | 108939 | 108958 |
| 63-20 | A'C'A'A'A'C'C'T'T'C'T'T'C'T'C'T'G'T'A'C' | 63 | 82 | 108940 | 108959 |
| 66-20 | A'A'A'A'C'A'A'A'C'C'T'T'C'T'T'C'T'C'T'G' | 66 | 85 | 108943 | 108962 |
| 70-20 | C'T'T'T'A'A'A'A'C'A'A'A'C'C'T'T'C'T'T'C' | 70 | 89 | 108947 | 108966 |

[Table 1-3]

| Sequence name | Nucleotide sequence (5'-3') of antisense oligonucleotide | Target region of SEQ ID NO: 1 | | Target region of SEQ ID NO: 2 | |
|---|---|---|---|---|---|
| | | Position of 5' end | Position of 3' end | Position of 5' end | Position of 3' end |
| 1-15 | G'A'T'A'C'A'G'T'T'T'T'C'G'A'A' | 1 | 15 | 108878 | 108892 |
| 2-15 | A'G'A'T'A'C'A'G'T'T'T'T'C'G'A' | 2 | 16 | 108879 | 108893 |
| 3-15 | A'A'G'A'T'A'C'A'G'T'T'T'T'C'G' | 3 | 17 | 108880 | 108894 |
| 4-15 | G'A'A'G'A'T'A'C'A'G'T'T'T'T'C' | 4 | 18 | 108881 | 108895 |
| 5-15 | C'G'A'A'G'A'T'A'C'A'G'T'T'T'T' | 5 | 19 | 108882 | 108896 |
| 6-15 | C'C'G'A'A'G'A'T'A'C'A'G'T'T'T' | 6 | 20 | 108883 | 108897 |
| 7-15 | C'C'C'G'A'A'G'A'T'A'C'A'G'T'T' | 7 | 21 | 108884 | 108898 |
| 8-15 | G'C'C'C'G'A'A'G'A'T'A'C'A'G'T' | 8 | 22 | 108885 | 108899 |
| 9-15 | TG'C'C'C'G'A'A'G'A'T'A'C'A'G' | 9 | 23 | 108886 | 108900 |
| 10-15 | G'T'G'C'C'C'G'A'A'G'A'T'A'C'A' | 10 | 24 | 108887 | 108901 |
| 11-15 | G'G'T'G'C'C'C'G'A'A'G'A'T'A'C' | 11 | 25 | 108888 | 108902 |
| 12-15 | TG'G'T'G'C'C'C'G'A'A'G'A'T'A' | 12 | 26 | 108889 | 108903 |
| 13-15 | T'T'G'G'T'G'C'C'C'G'A'A'G'A'T' | 13 | 27 | 108890 | 108904 |
| 14-15 | T'T'T'G'G'T'G'C'C'C'G'A'A'G'A' | 14 | 28 | 108891 | 108905 |
| 15-15 | C'T'T'T'G'G'T'G'C'C'C'G'A'A'G' | 15 | 29 | 108892 | 108906 |
| 16-15 | T'C'T'T'T'G'G'T'G'C'C'C'G'A'A' | 16 | 30 | 108893 | 108907 |
| 17-15 | C'T'C'T'T'T'G'G'T'G'C'C'C'G'A' | 17 | 31 | 108894 | 108908 |
| 18-15 | G'C'T'C'T'T'T'G'G'T'G'C'C'C'G' | 18 | 32 | 108895 | 108909 |

(continued)

| Sequence name | Nucleotide sequence (5’-3’) of antisense oligonucleotide | Target region of SEQ ID NO: 1 | | Target region of SEQ ID NO: 2 | |
|---|---|---|---|---|---|
| | | Position of 5’ end | Position of 3’ end | Position of 5’ end | Position of 3’ end |
| 19-15 | T’G’C’T’C’T’T’T’G’G’T’G’C’C’C’ | 19 | 33 | 108896 | 108910 |
| 20-15 | A’T’G’C’T’C’T’T’T’G’G’T’G’C’C’ | 20 | 34 | 108897 | 108911 |
| 21-15 | A’A’T’G’C’T’C’T’T’T’G’G’T’G’C’ | 21 | 35 | 108898 | 108912 |

[Table 1-4]

| Sequence name | Nucleotide sequence (5’-3’) of antisense oligonucleotide | Target region of SEQ ID NO: 1 | | Target region of SEQ ID NO: 2 | |
|---|---|---|---|---|---|
| | | Position of 5’ end | Position of 3’ end | Position of 5’ end | Position of 3’ end |
| 1-25 | G’G’T’G’C’C’C’G’A’A’G’A’T’A’C’A’G’T’T’T’T’C’G’A’A’ | 1 | 25 | 108878 | 108902 |
| 3-25 | T’T’G’G’T’G’C’C’C’G’A’A’G’A’T’A’C’A’G’T’T’T’T’C’G’ | 3 | 27 | 108880 | 108904 |
| 5-25 | C’T’T’T’G’G’T’G’C’C’C’G’A’A’G’A’T’A’C’A’G’T’T’T’T’ | 5 | 29 | 108882 | 108906 |
| 7-25 | C’T’C’T’T’T’G’G’T’G’C’C’C’G’A’A’G’A’T’A’C’A’G’T’T’ | 7 | 31 | 108884 | 108908 |
| 9-25 | T’G’C’T’C’T’T’T’G’G’T’G’C’C’C’G’A’A’G’A’T’A’C’A’G’ | 9 | 33 | 108886 | 108910 |
| 11-25 | A’A’T’G’C’T’C’T’T’T’G’G’T’G’C’C’C’G’A’A’G’A’T’A’C’ | 11 | 35 | 108888 | 108912 |
| 15-25 | T’A’A’C’A’A’T’G’C’T’C’T’T’T’G’G’T’G’C’C’C’G’A’A’G’ | 15 | 39 | 108892 | 108916 |
| 15-24 | A’A’C’A’A’T’G’C’T’C’T’T’T’G’G’T’G’C’C’C’G’A’A’G’ | 15 | 38 | 108892 | 108915 |
| 15-23 | A’C’A’A’T’G’C’T’C’T’T’T’G’G’T’G’C’C’C’G’A’A’G’ | 15 | 37 | 108892 | 108914 |
| 15-22 | C’A’A’T’G’C’T’C’T’T’T’G’G’T’G’C’C’C’G’A’A’G’ | 15 | 36 | 108892 | 108913 |
| 15-21 | A’A’T’G’C’T’C’T’T’T’G’G’T’G’C’C’C’G’A’A’G’ | 15 | 35 | 108892 | 108912 |
| 15-19 | T’G’C’T’C’T’T’T’G’G’T’G’C’C’C’G’A’A’G’ | 15 | 33 | 108892 | 108910 |
| 15-18 | G’C’T’C’T’T’T’G’G’T’G’C’C’C’G’A’A’G’ | 15 | 32 | 108892 | 108909 |
| 15-17 | C’T’C’T’T’T’G’G’T’G’C’C’C’G’A’A’G’ | 15 | 31 | 108892 | 108908 |
| 15-16 | T’C’T’T’T’G’G’T’G’C’C’C’G’A’A’G’ | 15 | 30 | 108892 | 108907 |
| 16-19 | A’T’G’C’T’C’T’T’T’G’G’T’G’C’C’C’G’A’A’ | 16 | 34 | 108893 | 108911 |
| 16-18 | T’G’C’T’C’T’T’T’G’G’T’G’C’C’C’G’A’A’ | 16 | 33 | 108893 | 108910 |
| 16-17 | G’C’T’C’T’T’T’G’G’T’G’C’C’C’G’A’A’ | 16 | 32 | 108893 | 108909 |
| 16-16 | C’T’C’T’T’T’G’G’T’G’C’C’C’G’A’A’ | 16 | 31 | 108893 | 108908 |
| 10-19 | T’T’T’G’G’T’G’C’C’C’G’A’A’G’A’T’A’C’A’ | 10 | 28 | 108887 | 108905 |
| 10-18 | T’T’G’G’T’G’C’C’C’G’A’A’G’A’T’A’C’A’ | 10 | 27 | 108887 | 108904 |
| 10-17 | T’G’G’T’G’C’C’C’G’A’A’G’A’T’A’C’A’ | 10 | 26 | 108887 | 108903 |
| 10-16 | G’G’T’G’C’C’C’G’A’A’G’A’T’A’C’A’ | 10 | 25 | 108887 | 108902 |
| 12-19 | T’C’T’T’T’G’G’T’G’C’C’C’G’A’A’G’A’T’A’ | 12 | 30 | 108889 | 108907 |
| 12-18 | C’T’T’T’G’G’T’G’C’C’C’G’A’A’G’A’T’A’ | 12 | 29 | 108889 | 108906 |

In Table 1-1 to Table 1-4 shown above, the sign "A’", the sign "C’", the sign "G’", and the sign "T’" are each

selected from the corresponding natural nucleoside (a, c, g, or t described later) and modified nucleosides (including modified-sugar nucleosides). For the 2'-O-alkylated nucleic acid among the modified-sugar nucleosides, the sign "A'" indicates one being selected from A(M) and A(m) shown later, the sign "C'" indicates one being selected from C(M) and 5(m) shown later, the sign "G'" indicates one being selected from G(M) and G(m) shown later, and the sign "T'" indicates one being selected from U(M) and T(m) shown later. For crosslinked modified nucleosides, the sign "A'" indicates one being selected from A(L), A(Y), A(Gx), and A(S) shown later, the sign "C'" indicates one being selected from 5(x), 5(L), 5(Y), 5(Gx), and 5(S) shown later, the sign "G'" indicates one being selected from G(L), G(Y), G(Gx), and G(S) shown later, and the sign "T'" indicates one being selected from T(L), T(Y), T(Gx), and T(S) shown later. For the morpholino nucleic acid, the sign "A'" indicates A(N) shown later, the sign "C'" indicates C(N) shown later, the sign "G'" indicates G(N) shown later, and the sign "T'" indicates T(N) shown later.

<Pharmacologically acceptable salt>

The single-stranded antisense oligonucleotide according to the present embodiment may be in the form of a pharmacologically acceptable salt. Here, the "pharmacologically acceptable salt" refers to a salt of the single-stranded antisense oligonucleotide of the present invention, wherein the salt is a physiologically acceptable salt of the single-stranded antisense oligonucleotide of the present invention, specifically, a salt retaining desired biological activities of the single-stranded antisense oligonucleotide and not retaining undesired toxicological effects thereof. The same also applies to a double-stranded antisense oligonucleotide and antisense oligonucleotide complex described later.

<Pharmaceutically acceptable salt>

In an aspect of the present embodiment, the single-stranded antisense oligonucleotide may be in the form of a pharmaceutically acceptable salt. Here, the "pharmaceutically acceptable salt" refers to one that is the mentioned pharmacologically acceptable salt and is an acid addition salt or a base addition salt. Examples of the acid addition salt include inorganic acid salts such as hydrochlorides, hydrobromides, sulfates, hydroiodides, nitrates, and phosphates; and organic acid salts such as citrates, oxalates, phthalates, fumarates, maleates, succinates, malates, acetates, formates, propionates, benzoates, trifluoroacetates, methanesulfonates, benzenesulfonates, para-toluenesulfonates, and camphorsulfonates. Examples of the base addition salt include inorganic base salts such as sodium salts, potassium salts, calcium salts, magnesium salts, barium salts, and aluminum salts; and organic base salts such as trimethylamine, triethylamine, pyridine, picoline, 2,6-lutidine, ethanolamine, diethanolamine, triethanolamine, tromethamine [tris(hydroxymethyl)methylamine], tert-butylamine, cyclohexylamine, dicyclohexylamine, and N,N-dibenzylethylamine. An additional example is a salt with a basic amino acid or acidic amino acid (amino acid salt) such as arginine, lysine, ornithine, aspartic acid, or glutamic acid. The same also applies to a double-stranded antisense oligonucleotide and antisense oligonucleotide complex described later.

<Structure of single-stranded antisense oligonucleotide>

The single-stranded antisense oligonucleotide according to the present embodiment is composed of a natural oligonucleotide and/or a non-natural oligonucleotide. It is preferable that the single-stranded antisense oligonucleotide be in the form of a single chain. In an aspect of the present embodiment, the single-stranded antisense oligonucleotide may hybridize with a second-strand oligonucleotide described later to take the form of a duplex (double-stranded antisense oligonucleotide). It is preferable that the nucleotide sequence of the second-strand oligonucleotide be a nucleotide sequence having a sequence identity of 90% or more and 100% or less with a nucleotide sequence complementary to the nucleotide sequence of the single-stranded antisense oligonucleotide.

<Mechanism of exon skipping by single-stranded antisense oligonucleotide>

The single-stranded antisense oligonucleotide restores a reading frame of an abnormally spliced target RNA into the original reading frame to induce expression of the functional WRN protein through the following mechanism.

An exon is included in an mRNA only when both of the splice sites are recognized by a spliceosome complex, and hence splicing can be inhibited by targeting any of the splice sites with an antisense oligonucleotide to induce exon skipping. Exon skipping can be induced through a process in which a single-stranded antisense oligonucleotide targeting either one or both of the 5' splice site and 3' splice site of an exon or the inside of an exon binds to the exon. That is, an exon of interest can be skipped through the process in which the single-stranded antisense oligonucleotide binds to the target region of the target RNA to inhibit splicing of the exon, and translation into a functional protein is induced on the basis of the mRNA expressed.

The single-stranded antisense oligonucleotide, in the present embodiment, can be preferably used for skipping

the 27th exon of a human WRN gene with high efficiency through the mechanism as described above (including the case of acting via regulating the maturation of an mRNA precursor for WRN). More specifically, the single-stranded antisense oligonucleotide binds to exon 27, which is the target region of the target RNA (mRNA precursor of WRN gene) (top in Fig. 3) to inhibit splicing of exon 27. Accordingly, a termination codon within exon 27 that has appeared as a result of a skipping mutation in exon 26, the mutation found in Werner syndrome patients, is skipped to generate a WRN mRNA in which exon 25 and exon 28 are linked together (corrected to the reading frame of the WRN gene of healthy individuals: in-frame) (middle in Fig. 3), and translation into the functional WRN protein having a nuclear localization signal contained in exon 34 occurs on the basis of the mRNA. Furthermore, according to the present embodiment, the effect of the single-stranded antisense oligonucleotide to regulate expression of a functional WRN gene can be exerted even in transdermal administration, intravenous administration, and intradermal administration, which are routes of administration commonly used in clinical applications. Here, "skipping the 27th exon of a human WRN gene with high efficiency" at least means skipping the 27th exon of a human WRN gene with high efficiency, and at least means the resulting restoration of expression of a functional WRN protein.

Examples of natural nucleotides whose sugar moiety is deoxyribose include deoxyadenosine monophosphate, deoxyguanosine monophosphate, thymidine monophosphate, deoxycytidine monophosphate, and deoxy-5-methylcytidine monophosphate. In other words, examples of natural nucleotides constituting the single-stranded antisense oligonucleotide include those having structural formulas corresponding to the signs a, g, t, c, and 5(x) shown later.

Examples of non-natural nucleotides whose sugar moiety is deoxyribose include 2-thio-thymidine monophosphate, 2-aminoadenosine monophosphate, and 7-deazaguanosine monophosphate.

The single-stranded antisense oligonucleotide of the present invention is 10 to 30mer, preferably 15 to 25mer, more preferably 18 to 22mer, further preferably 16 to 20mer, and particularly preferably 18 to 20mer in nucleotide length. If the single-stranded antisense oligonucleotide of the present invention is 15 to 25mer, 18 to 22mer, 16 to 20mer, or 18 to 20mer in nucleotide length, the binding to an mRNA precursor for WRN is particularly strong, and high-efficient skipping of the 27th exon of a human WRN gene can be effectively controlled.

In the present embodiment, one nucleotide is bound to another via a phosphate group and/or modified phosphate group in the single-stranded antisense oligonucleotide, and it is preferable that one nucleotide be bound to another via a phosphodiester bond or a phosphorothioate bond in the single-stranded antisense oligonucleotide.

<Double-stranded antisense oligonucleotide>

The double-stranded antisense oligonucleotide according to the present embodiment is a double-stranded antisense oligonucleotide containing the single-stranded antisense oligonucleotide and a second-strand oligonucleotide hybridizing with the single-stranded antisense oligonucleotide, or a pharmaceutically acceptable salt thereof. It is preferable that the nucleotide sequence of the second-strand oligonucleotide be a nucleotide sequence having a sequence identity of 90% or more and 100% or less with a nucleotide sequence complementary to the nucleotide sequence of the single-stranded antisense oligonucleotide.

The double-stranded antisense oligonucleotide can dissociate in a solution to separate into the single-stranded antisense oligonucleotide and the second-strand oligonucleotide. The single-stranded antisense oligonucleotide separated can bind to the target RNA described above. The single-stranded antisense oligonucleotide may be regarded as the "first-strand oligonucleotide" in relation to the second-strand oligonucleotide. Of the oligonucleotides constituting the double-stranded antisense oligonucleotide, the first-strand oligonucleotide has an antisense strand to the target RNA described above; however, the double-stranded oligonucleotide consisting of the first-strand oligonucleotide and the second-strand oligonucleotide is referred to as the "double-stranded antisense oligonucleotide" for convenience.

<<Method for producing single-stranded antisense oligonucleotide>>

The single-stranded antisense oligonucleotide of the present invention can be produced through solid-phase synthesis by a phosphoramidite method. For example, a single-stranded oligonucleotide having a specific nucleotide sequence is first synthesized on a solid-phase carrier by using a commercially available automated nucleic acid synthesizer. Next, the single-stranded oligonucleotide synthesized is cut out of the solid-phase carrier by using a basic substance or the like, and deprotected to give a crude form of the single-stranded oligonucleotide. Thereafter, the resulting crude form of the single-stranded oligonucleotide is purified by using HPLC or the like. Production of the single-stranded antisense oligonucleotide of the present invention is not limited to the described production method, and the single-stranded antisense oligonucleotide of the present invention can be produced with appropriate modification of, for example, the nucleotide sequence or modification sites of the nucleic acid according to a method known to those skilled in the art. AmNA, GuNA, and scpBNA can be produced with methods described in WO 2011/052436 (PTL 1), WO 2014/046212 (PTL 2), and WO 2015/125783 (PTL 3), respectively. Morpholino oligonucleic acids (PMO) can be produced according to a method described in WO 1991/009033 (PTL 4), WO 2009/064471 (PTL 5), or NPL 8.

<<Method for producing double-stranded antisense oligonucleotide>>

The double-stranded antisense oligonucleotide of the present invention can be produced as follows: first, an oligonucleotide having a specific sequence identity with a nucleotide sequence complementary to the single-stranded antisense oligonucleotide (second-strand oligonucleotide) is produced by using the same production method as for the single-stranded antisense oligonucleotide; thereafter, the single-stranded antisense oligonucleotide and the second-strand oligonucleotide are allowed to hybridize with each other.

«Antisense oligonucleotide complex»

The antisense oligonucleotide complex according to the present embodiment contains:

the oligonucleotide (preferably, the single-stranded antisense oligonucleotide) or a pharmaceutically acceptable salt thereof, or the double-stranded antisense oligonucleotide or a pharmaceutically acceptable salt thereof; and
an additive substance bound to the oligonucleotide or the second-strand oligonucleotide.

The additive substance is selected from the group consisting of polyethylene glycol, a peptide, an alkyl chain (e.g., saturated aliphatic hydrocarbon), a ligand compound, an antibody, a protein, and a sugar chain (e.g., carbohydrate, polysaccharide).

In an aspect of the present embodiment, the oligonucleotide complex is an oligonucleotide complex or a pharmaceutically acceptable salt thereof, the oligonucleotide complex containing:

the oligonucleotide or a pharmaceutically acceptable salt thereof, or the double-stranded antisense oligonucleotide or a pharmaceutically acceptable salt thereof; and
an additive substance bound to the oligonucleotide or the second-strand oligonucleotide directly or via a linker bond, wherein
the additive substance is selected from the group consisting of polyethylene glycol, a peptide, an alkyl chain, a ligand compound, an antibody, a protein, and a sugar chain, and
the linker bond is independently in each occurrence a phosphodiester bond, a phosphorothioate bond, a phosphorodithioate bond, a phosphoamidate bond, a boranophosphate bond, an alkylphosphonate bond, a phosphorodiamidate bond, a phosphorodiamidothioate bond, or a phosphorodiamidodithioate bond.

In an aspect of the present embodiment, the antisense oligonucleotide complex contains:

the oligonucleotide (preferably, the single-stranded antisense oligonucleotide) or a pharmaceutically acceptable salt thereof; and
an additive substance bound to the oligonucleotide, wherein the additive substance is selected from the group consisting of polyethylene glycol, a peptide, an alkyl chain, a ligand compound, an antibody, a protein, and a sugar chain.

In another aspect of the present embodiment, the antisense oligonucleotide complex contains:

the double-stranded antisense oligonucleotide or a pharmaceutically acceptable salt thereof; and
an additive substance bound to the single-stranded antisense oligonucleotide or the second-strand oligonucleotide, wherein the additive substance is selected from the group consisting of polyethylene glycol, a peptide, an alkyl chain (e.g., saturated aliphatic hydrocarbon), a ligand compound, an antibody, a protein, and a sugar chain (e.g., carbohydrate, polysaccharide).

In the present embodiment, the "additive substance" refers to a substance that is bound to the single-stranded antisense oligonucleotide or the second-strand oligonucleotide and used for imparting a specific effect. The additive substance may be bound to the 5' end, the 3' end, or each of the 5' end and 3' end of the single-stranded antisense oligonucleotide. The additive substance may be bound to the 5' end, the 3' end, or each of the 5' end and 3' end of the second-strand oligonucleotide. In an aspect of the present embodiment, it is preferable that the additive substance be bound to either one of the 5' end and 3' end of the single-stranded antisense oligonucleotide or the second-strand oligonucleotide. The additive substance may be directly bound to the single-stranded antisense oligonucleotide or the second-strand oligonucleotide via a covalent bond. The additive substance may be bound to the single-stranded antisense oligonucleotide or the second-strand oligonucleotide via a linker substance. Examples of the linker substance include an alkyl, polyethylene glycol, a peptide, a disulfide, a phosphate bond, and/or a linker composed of any combination of

them. The linker substance may be regarded as a linker bond.

Examples of the peptide to be used as the additive substance include, but are not limited to, the followings: CPPs (Cell Penetrating Peptides), nuclear-localized peptides, TAT (Trans-Activator of Transcription protein), polyarginine, glucagon-like peptide 1 analog peptide, synthetic cyclic RGD peptide, and skin penetrating peptides.

Examples of the ligand compound to be used as the additive substance include, but are not limited to, the followings: N-acetylgalactosamine (GalNAc), saccharides (e.g., glucose, mannose), lipids (e.g., cholesterol), vitamins (e.g., folic acid, vitamin A, vitamin E), and amino acids.

Examples of the antibody to be used as the additive substance include, but are not limited to, the followings: an anti-insulin receptor antibody, an anti-transferrin receptor antibody, an anti-LDL receptor-associated protein antibody, an anti-CD22 antibody, an anti-CD30 antibody, and an anti-HER2 antibody.

Examples of the protein to be used as the additive substance include, but are not limited to, the following: albumin.

Examples of combinations of the additive substance and the linker substance in morpholino oligonucleic acids include, but are not limited to, those represented by the following structural formula:

[Formula 9]

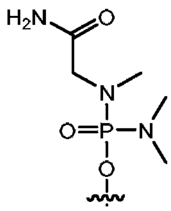

<<Skipping agent for 27th exon of human WRN gene>>

The skipping agent for the 27th exon of a human WRN gene according to the present embodiment contains the single-stranded antisense oligonucleotide of the present invention, the double-stranded antisense oligonucleotide, or the antisense oligonucleotide complex as an active ingredient.

The single-stranded antisense oligonucleotide of the present invention enables skipping of the 27th exon of a human WRN gene by binding to an mRNA precursor for WRN. Any administration method and formulation known in the art are available as an administration method and formulation for the skipping agent.

«Functional WRN recovery agent»

The functional WRN recovery agent according to the present embodiment is a functional WRN recovery agent that possesses a nuclear localization signal by virtue of skipping of the 27th exon of a human WRN gene, the functional WRN recovery agent containing the oligonucleotide or a pharmaceutically acceptable salt thereof, the double-stranded antisense oligonucleotide or a pharmaceutically acceptable salt thereof, or the oligonucleotide complex or a pharmaceutically acceptable salt thereof as an active ingredient. The single-stranded antisense oligonucleotide of the present invention enables skipping of the 27th exon of a human WRN gene by binding to an mRNA precursor for WRN. In particular, if the human WRN gene has a skipping mutation in exon 26, the mutation found in Werner syndrome patients, a termination codon within exon 27 that has appeared in the human WRN gene is skipped to generate a WRN mRNA in which exon 25 and exon 28 are linked together, and translation into a functional WRN protein having a nuclear localization signal contained in exon 34 occurs on the basis of the mRNA.

«Pharmaceutical composition containing single-stranded antisense oligonucleotide or the like as active ingredient»

The pharmaceutical composition according to the present embodiment contains the single-stranded antisense oligonucleotide of the present invention or a pharmaceutically acceptable salt thereof, the double-stranded antisense oligonucleotide or a pharmaceutically acceptable salt thereof, or the antisense oligonucleotide complex or a pharmaceutically acceptable salt thereof as an active ingredient. Any administration method and formulation known in the art are available as an administration method and formulation for the pharmaceutical composition of the present embodiment. Hereinafter, the pharmaceutical composition is occasionally referred to as "the pharmaceutical composition of the antisense oligonucleotide or the like".

The pharmaceutical composition is used for treating or preventing Werner syndrome. In other words, the pharmaceutical composition can be used for treatment or prevention expected to ameliorate symptoms of Werner syndrome by skipping the 27th exon of a human WRN gene with high efficiency.

<<Therapeutic agent and prophylactic agent for Werner syndrome>>

The therapeutic agent for Werner syndrome according to the present embodiment contains the single-stranded antisense oligonucleotide or a pharmaceutically acceptable salt thereof, the double-stranded antisense oligonucleotide or a pharmaceutically acceptable salt thereof, or the antisense oligonucleotide complex or a pharmaceutically acceptable salt thereof as an active ingredient. The prophylactic agent for Werner syndrome according to the present embodiment contains the single-stranded antisense oligonucleotide or a pharmaceutically acceptable salt thereof, the double-stranded antisense oligonucleotide or a pharmaceutically acceptable salt thereof, or the antisense oligonucleotide complex or a pharmaceutically acceptable salt thereof as an active ingredient.

<Individuals>

An individual in the present invention refers to a mammal. Preferred are humans, monkeys, marmosets, dogs, pigs, rabbits, guinea pigs, rats, and mice. More preferred are humans.

In administering the single-stranded antisense oligonucleotide of the present invention or the pharmaceutical composition (including the therapeutic agent and prophylactic agent for Werner syndrome), the administration method and dosage form are not limited. That is, any administration method and formulation known in the art can be used as an administration method and formulation for the antisense oligonucleotide or the like of the present invention. Examples of the administration method include oral administration and parenteral administration. Examples of the parenteral administration include ophthalmic administration, intravaginal administration, intrarectal administration, intranasal administration, transdermal administration, intravenous injection, drip infusion, subcutaneous administration, intraperitoneal administration or intramuscular infusion, pulmonary administration by aspiration or inhalation, intrathecal administration, and intracerebroventricular administration.

As necessary, various pharmaceutical excipients such as diluents, binders, wetting agents, disintegrators, lubricants, diluting agents, corrigents, aromatic substances, solubilizers, suspending agents, emulsifying agents, stabilizers, preservatives, and isotonic agents can be mixed into the formulation of the antisense oligonucleotide or the like of the present invention.

In topically administering the pharmaceutical composition of the antisense oligonucleotide or the like of the present invention, formulations including transdermal patches, ointments, lotions, creams, gels, drops, suppositories, sprays, liquids, and powders are available.

In orally administering the pharmaceutical composition of the antisense oligonucleotide or the like of the present invention, formulations including powders, granules, suspensions or solutions with water or nonaqueous medium for dissolution, capsules, dry powders, and tablets are available.

In parenterally, intravenously, subcutaneously, or intradermally administering the pharmaceutical composition of the antisense oligonucleotide or the like of the present invention, formulations including sterile aqueous solutions are available.

The effective dose of the single-stranded antisense oligonucleotide of the present invention can be arbitrarily set, for example, according to the sex, age, and body weight of and symptoms in an individual to receive the dose. Additionally, the effective dose can be arbitrarily set, for example, according to the method, route, and frequency of administration. An example of the dose is 0.01 to 100 mg/kg. The dose is preferably 0.1 to 50 mg/kg, and further preferably 0.1 to 10 mg/kg.

<<Method for skipping 27th exon of human WRN gene with high efficiency>>

The method for skipping the 27th exon of a human WRN gene in the present embodiment includes administering the single-stranded antisense oligonucleotide or a pharmaceutically acceptable salt thereof, the double-stranded antisense oligonucleotide or a pharmaceutically acceptable salt thereof, or the antisense oligonucleotide complex or a pharmaceutically acceptable salt thereof as an active ingredient to cells, tissue, or an individual expressing such a WRN gene.

In the present embodiment, administering the single-stranded antisense oligonucleotide or the like to cells, tissue, or an individual may be performed with an in vitro or in vivo method. For in vivo administration, any of the routes of administration mentioned above is used as a route of administration therefor.

Examples of the "cells expressing a WRN gene" in the present embodiment include fibroblasts, adipocytes, mesenchymal stem cells, keratinocytes, myocytes, vascular endothelial cells, and smooth muscle cells.

The method for treating or preventing Werner syndrome in the present embodiment includes administering the single-stranded antisense oligonucleotide or a pharmaceutically acceptable salt thereof, the double-stranded antisense oligonucleotide or a pharmaceutically acceptable salt thereof, or the antisense oligonucleotide complex or a pharmaceutically acceptable salt thereof as an active ingredient to an individual affected by Werner syndrome.

Thus, the antisense oligonucleotide according to the present embodiment has been described. The single-stranded antisense oligonucleotide having the configuration described above enables skipping of the 27th exon of a human WRN gene with high efficiency.

<<Method for evaluating activity for skipping of 27th exon of human WRN gene>>

introduction of antisense oligonucleotide into cells>

Observation of "cells expressing a WRN gene" becomes possible by treating them with the antisense oligonucleotide by means of introduction by, for example, lipofection, electroporation, or direct addition for 6 hours to a month. It is preferable that the treatment time be 48 hours to 7 days. The cells to be used can be any cells expressing a WRN gene, and are, for example, HEK293T cells or fibroblasts derived from a Werner syndrome patient (hereinafter, referred to as "Werner syndrome patient fibroblasts"). The cells treated with the antisense oligonucleotide may be collected immediately after the treatment, or continuously cultured with the antisense oligonucleotide removed.

<Evaluation of 27th exon skipping for human WRN gene>

Reverse transcription reaction is performed for the total RNA extracted from the cells collected, and a region around exon 27 of a WRN gene of the resulting cDNA is amplified through PCR. The 27th exon skipping for the WRN gene can be confirmed by quantification of the amounts of polynucleotides or sequence analysis for the PCR amplification products. To calculate the skipping efficiency (%), the amounts of the polynucleotides of PCR products with exon 27 skipped, "A", and the amounts of the polynucleotides of PCR products without exon 27 skipped, "B", are quantified, and the measurements of "A" and "B" are applied to the following calculation formula. Skipping efficiency (%) = $100 \times A / (A + B)$

The present invention is not limited to the embodiment described above. For example, the following modes of implementation are included as the single-stranded antisense oligonucleotide.

A mode of the single-stranded antisense oligonucleotide of the present invention is an oligonucleotide that expresses a functional human WRN protein against a skip mutation in the 26th or 28th exon of a human WRN gene, or a pharmaceutically acceptable salt thereof, wherein

one nucleotide is bound to another via a phosphate group and/or modified phosphate group in the oligonucleotide,
the oligonucleotide contains a modified nucleic acid having at least one modified sugar,
the oligonucleotide is 10 to 30mer in nucleotide length, and
the nucleotide sequence of the oligonucleotide is:

a nucleotide sequence having a sequence identity of 90% or more and 100% or less with a nucleotide sequence complementary to at least one target region having the same nucleotide length as the oligonucleotide in a nucleotide sequence set forth in SEQ ID NO: 1;
a nucleotide sequence complementary to a nucleotide sequence formed by deleting, substituting, inserting, or adding one or several nucleotides in the target region; or
a nucleotide sequence hybridizable with an oligonucleotide having the target region under stringent conditions.

In another mode of the single-stranded antisense oligonucleotide of the present invention, the nucleotide sequence of the single-stranded antisense oligonucleotide is:
a nucleotide sequence having a sequence identity of 95% or more and 100% or less with a nucleotide sequence complementary to at least one target region having the same nucleotide length as the single-stranded antisense oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6.

In another mode of the single-stranded antisense oligonucleotide of the present invention, the nucleotide sequence of the single-stranded antisense oligonucleotide is a nucleotide sequence complementary to at least one target region having the same nucleotide length as the single-stranded antisense oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6.

In another mode of the single-stranded antisense oligonucleotide of the present invention, a sugar constituting the oligonucleotide is D-ribofuranose, and modification of a sugar is sugar modification on a hydroxy group at position 2' of D-ribofuranose.

The oligonucleotide is 15 to 25mer in nucleotide length.

In another mode of the single-stranded antisense oligonucleotide of the present invention, modification of a sugar constituting the oligonucleotide is made to form a group represented by the following formula (A1):

[Formula 10]

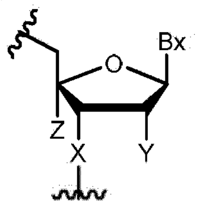

(A1)

wherein Bx is a nucleobase, being independently in each occurrence a group formed from adenine, guanine, cytosine, 5-methylcytosine, thymine, or uracil; X is a phosphate bond, being independently in each occurrence a phosphodiester bond, a phosphorothioate bond, a phosphorodithioate bond, a phosphoamidate bond, a boranophosphate bond, or an alkylphosphonate bond; Y is independently in each occurrence a hydrogen atom, a hydroxy group, a fluorine atom, an optionally substituted alkoxy group having one to six carbon atoms, or an optionally substituted amino group; and Z is independently in each occurrence a hydrogen atom, or an alkyl group having one to five carbon atoms and optionally having a carbon-oxygen double bond or a cyclic structure, or a certain carbon atom in the alkyl group and Y are taken together to form a ring. Examples of the "carbon-oxygen double bond" possessed by the alkyl group having one to five carbon atoms include a carbonyl group. Examples of the "cyclic structure" possessed by the alkyl group having one to five carbon atoms include a cyclopropane structure.

In another mode of the single-stranded antisense oligonucleotide of the present invention, at least one intemucleotide bond in the oligonucleotide is a phosphorothioate bond.

In another mode of the single-stranded antisense oligonucleotide of the present invention, at least one internucleotide bond in the oligonucleotide is a phosphodiester bond.

In another mode of the single-stranded antisense oligonucleotide of the present invention, the nucleotide sequence of the oligonucleotide is:

a nucleotide sequence having a sequence identity of 90% or more and 100% or less with a nucleotide sequence complementary to a target region composed of continuous 15 to 25mer starting from a nucleotide at any of positions 1 to 12, 14 to 21, 24, 25, 29 to 31, 34, 36, 41, 46, 51, and 62 counted from a 5' end in a nucleotide sequence set forth in SEQ ID NO: 1;
a nucleotide sequence complementary to a nucleotide sequence formed by deleting, substituting, inserting, or adding one or several nucleotides in the target region; or
a nucleotide sequence hybridizable with an oligonucleotide having the target region under stringent conditions.

In another mode of the single-stranded antisense oligonucleotide of the present invention, the nucleotide sequence of the oligonucleotide is:

a nucleotide sequence having a sequence identity of 90% or more and 100% or less with a nucleotide sequence complementary to a target region composed of continuous 15 to 25mer starting from a nucleotide at any of positions 1 to 40, 46, 51, 56, and 62 to 63 counted from a 5' end in a nucleotide sequence set forth in SEQ ID NO: 1 or a target region composed of continuous 15 to 25mer starting from a nucleotide at any of positions 108873, 108878 to 108917, 108923, 108928, 108933, and 108939 to 108940 counted from a 5' end in a nucleotide sequence set forth in SEQ ID NO: 2;
a nucleotide sequence complementary to a nucleotide sequence formed by deleting, substituting, inserting, or adding one or several nucleotides in the target region; or
a nucleotide sequence hybridizable with an oligonucleotide having the target region under stringent conditions.

In another mode of the single-stranded antisense oligonucleotide of the present invention, the nucleotide sequence of the oligonucleotide is a nucleotide sequence having a sequence identity of 90% or more and 100% or less with a nucleotide sequence complementary to a target region composed of continuous 15 to 25mer starting from a nucleotide at any of positions 1, 3 to 12, 14 to 19, 21, 25, 29, 30, 31, 34, 46, and 51 counted from a 5' end in a nucleotide sequence set forth in SEQ ID NO: 1.

In another mode of the single-stranded antisense oligonucleotide of the present invention, the nucleotide sequence of the oligonucleotide is a nucleotide sequence having a sequence identity of 90% or more and 100% or less with a nucleotide sequence complementary to a target region composed of continuous 15 to 25mer starting from a nucleotide at any of positions 1 to 32, 34 to 40, 46, 51, and 62 to 63 counted from a 5' end in a nucleotide sequence set forth in SEQ ID NO: 1 or a target region composed of continuous 15 to 25mer starting from a nucleotide at any of positions

108878 to 108909, 108911 to 108917, 108923, 108928, and 108939 to 108940 counted from a 5' end in a nucleotide sequence set forth in SEQ ID NO: 2.

In another mode of the single-stranded antisense oligonucleotide of the present invention, the nucleotide sequence of the oligonucleotide is a nucleotide sequence having a sequence identity of 90% or more and 100% or less with a nucleotide sequence complementary to a target region composed of continuous 15 to 25mer starting from a nucleotide at any of positions 1, 3, 6 to 12, 14 to 19, 21, 25, 29, 30, 31, 34, 46, and 51 counted from a 5' end in a nucleotide sequence set forth in SEQ ID NO: 1.

In another mode of the single-stranded antisense oligonucleotide of the present invention, the nucleotide sequence of the oligonucleotide is a nucleotide sequence having a sequence identity of 90% or more and 100% or less with a nucleotide sequence complementary to a target region composed of continuous 15 to 25mer starting from a nucleotide at any of positions 1, 3, 5 to 12, 14 to 19, 21, 25, 29, 30, 31, 34, 46, and 51 counted from a 5' end in a nucleotide sequence set forth in SEQ ID NO: 1 or a target region composed of continuous 15 to 25mer starting from a nucleotide at any of positions 108878, 108880, 108882 to 108889, 108891 to 108896, 108898, 108902, 108906 to 108908, 108911, 108923, and 108928 counted from a 5' end in a nucleotide sequence set forth in SEQ ID NO: 2.

In another mode of the single-stranded antisense oligonucleotide of the present invention, modification of a sugar constituting the oligonucleotide is made to form a group represented by the following formula (A2) or formula (A3):

[Formula 11]

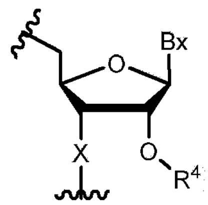

(A2)

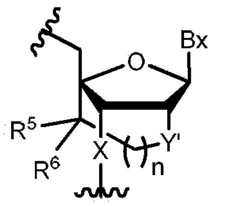

(A3)

wherein Bx is a nucleobase, being independently in each occurrence a group formed from adenine, guanine, cytosine, 5-methylcytosine, thymine, or uracil; X is a phosphate bond, being independently in each occurrence a phosphodiester bond, a phosphorothioate bond, a phosphorodithioate bond, a phosphoamidate bond, a boranophosphate bond, or an alkylphosphonate bond; $R^4$ is independently in each occurrence a hydrogen atom or an optionally substituted alkyl group having one to six carbon atoms; Y' is an oxygen atom or an optionally substituted nitrogen atom; $R^5$ and $R^6$ are each independently in each occurrence a hydrogen atom or an optionally substituted alkyl group having one to six carbon atoms, or $R^5$ and $R^6$ are taken together to form a carbonyl group or a ring; and n is 0 to 1.

In another mode of the single-stranded antisense oligonucleotide of the present invention, the nucleotide sequence of the oligonucleotide is one nucleotide sequence selected from the group consisting of nucleotide sequences set forth in SEQ ID NOs: 9, 11, 14 to 22, 24 to 29, 31, 35, 39 to 41, 44, 52, and 53.

In another mode of the single-stranded antisense oligonucleotide of the present invention, the nucleotide sequence of the oligonucleotide is one nucleotide sequence selected from the group consisting of nucleotide sequences set forth in SEQ ID NOs: 9 to 14, 16 to 26, 28 to 39, 41 to 42, 44 to 50, 52, 55 to 56, 69, 73, 80 to 105, 107 to 108, 110, 112, 114, 116, 118 to 131, 133, and 135.

In another mode of the single-stranded antisense oligonucleotide of the present invention, the nucleotide sequence of the oligonucleotide is a single-stranded antisense oligonucleotide.

EXAMPLES

Hereinafter, Examples according to the present invention will be described, but the present invention is not limited thereto.

«Preparation of single-stranded antisense oligonucleotides for WRN gene»

Single-stranded antisense oligonucleotides for a WRN gene were prepared in the following procedure.

Single-stranded antisense oligonucleotides containing any of 2'-OMe (O-methyl), 2'-MOE (O-methoxyethyl), AmNA, scpBNA, and GuNA were synthesized by using an automated nucleic acid synthesizer (model nS-8, manufactured by GeneDesign, Inc.) at a scale of 0.2 μmol. Chain length elongation was performed in accordance with a standard phosphoramidite protocol. At that time, CPG resin was used as a solid-phase carrier. In sulfidation for phosphorothioated skeleton (PS) formation, DDTT (((dimethylamino-methylidene)amino)-3H-1,2,4-dithiazaoline-3-thione) or the like was used. Antisense oligonucleotides containing any of AmNA and scpBNA were obtained as ones each of which had the hydroxy group at position 5' of an end not protected with a DMTr (4,4'-dimethoxytrityl) group and was supported at position 3' by the solid phase. Each single-stranded antisense oligonucleotide was cut out of the solid-phase carrier through subsequent alkali treatment, and collected in a state of solution. Thereafter, the solvent was distilled off from the collected solution to give a crude product. The crude products obtained were purified by reversed-phase HPLC to give purified single-stranded antisense oligonucleotides. The purities and structures of single-stranded antisense oligonucleotides obtained were checked by LC-MS (manufactured by Waters Corporation). Morpholino oligonucleic acids containing morpholino nucleic acid were synthesized according to WO 2009/064471 (PTL 5) or J. Am. Chem. Soc (2016) 138: 15663-15672 (NPL 8).

The single-stranded antisense oligonucleotides produced with the method described above are listed in Table 2-1 to Table 2-7 in the following.

[Table 2-1]

| Example | Sequence name | Sequence (5' → 3') | Molecular weight (found) | SEQ ID NO |
|---|---|---|---|---|
| 1 | (-)10-20-A | A(M)^G(M)^U(M)^U(M)^U(M)^U(M)^C(M)^G(M)^A(M)^A(M)^C(M)^U(M)^A(M)^A(M)^A(M)^A(M)^A(M)^A(M)^U(M)^A(M) | 6951.82 | 7 |
| 2 | (-)5-20-A | G(M)^A(M)^U(M)^A(M)^C(M)^A(M)^G(M)^U(M)^U(M)^U(M)^U(M)^C(M)^G(M)^A(M)^A(M)^C(M)^U(M)^A(M)^A(M)^A(M) | 6942.94 | 8 |
| 3 | 1-20-A | C(M)^C(M)^G(M)^A(M)^A(M)^G(M)^A(M)^U(M)^A(M)^C(M)^A(M)^G(M)^U(M)^U(M)^U(M)^U(M)^C(M)^G(M)^A(M)^A(M) | 6958.53 | 9 |
| 4 | 2-20-A | C(M)^C(M)^C(M)^G(M)^A(M)^A(M)^G(M)^A(M)^U(M)^A(M)^C(M)^A(M)^G(M)^U(M)^U(M)^U(M)^U(M)^C(M)^G(M)^A(M) | 6935.82 | 10 |
| 5 | 3-20-A | G(M)^C(M)^C(M)^C(M)^G(M)^A(M)^A(M)^G(M)^A(M)^U(M)^A(M)^C(M)^A(M)^G(M)^U(M)^U(M)^U(M)^U(M)^C(M)^G(M) | 6952.07 | 11 |
| 6 | 4-20-A | U(M)^G(M)^C(M)^C(M)^C(M)^G(M)^A(M)^A(M)^G(M)^A(M)^U(M)^A(M)^C(M)^A(M)^G(M)^U(M)^U(M)^U(M)^U(M)^C(M) | 6910.14 | 12 |
| 7 | 5-20-A | G(M)^U(M)^G(M)^C(M)^C(M)^C(M)^G(M)^A(M)^A(M)^G(M)^A(M)^U(M)^A(M)^C(M)^A(M)^G(M)^U(M)^U(M)^U(M)^U(M) | 6951.16 | 13 |
| 8 | 6-20-A | G(M)^G(M)^U(M)^G(M)^C(M)^C(M)^C(M)^G(M)^A(M)^A(M)^G(M)^A(M)^U(M)^A(M)^C(M)^A(M)^G(M)^U(M)^U(M)^U(M) | 6988.32 | 14 |
| 9 | 7-20-A | U(M)^G(M)^G(M)^U(M)^G(M)^C(M)^C(M)^C(M)^G(M)^A(M)^A(M)^G(M)^A(M)^U(M)^A(M)^C(M)^A(M)^G(M)^U(M)^U(M) | 6992.24 | 15 |

(continued)

| Example | Sequence name | Sequence (5' → 3') | Molecular weight (found) | SEQ ID NO |
|---|---|---|---|---|
| 10 | 8-20-A | U(M)^U(M)^G(M)^G(M)^U(M)^G(M)^C(M)^C(M)^C(M)^G(M)"A(M)^A(M)^G(M)^A(M)^U(M)^A(M)^C(M)^A(M)^G(M)^U(M) | 6990.45 | 16 |
| 11 | 8-20-B | T(Y)^U(M)^G(M)^G(Y)^U(M)^G(Y)^C(M)^C(M)^5(Y)^G(M)^A(M)^A(Y)^G(M)^A(M)^T(Y)^A(M)^C(M)^A(Y)^G(M)^U(M) | 7208.20 | 17 |
| 12 | 8-20-C | T(Y)-T(Y)^G(M)^G(M)^T(Y)-G(Y)^C(M)^C(M)^5(Y)-G(Y)^A(M)^A(M)^G(Y)-A(Y)^U(M)^A(M)^5(Y)-A(Y)^G(M)^(M) | 7231.05 | 18 |
| 13 | 9-20-A | U(M)^U(M)^U(M)^G(M)^G(M)^U(M)^G(M)^C(M)^C(M)^C(M)^G(M)^A(M)^A(M)^G(M)^A(M)^U(M)^A(M)^C(M)^A(M)^G(M) | 6992.05 | 19 |
| 14 | 10-20-A | C(M)^U(M)^U(M)^U(M)^G(M)^G(M)^U(M)^G(M)^C(M)^C(M)^C(M)^G(M)^A(M)^A(M)^G(M)^A(M)^U(M)^A(M)^C(M)^A(M) | 6953.70 | 20 |
| 15 | 11-20-A | U(M)^C(M)^U(M)^U(M)^U(M)^G(M)^G(M)^U(M)^G(M)^C(M)^C(M)^C(M)^G(M)^A(M)^A(M)^G(M)^A(M)^U(M)^A(M)^C(M) | 6928.27 | 21 |
| 16 | 12-20-A | C(M)^U(M)^C(M)^U(M)^U(M)^U(M)^G(M)^G(M)^U(M)^G(M)^C(M)^C(M)^C(M)^G(M)^A(M)^A(M)^G(M)^A(M)^U(M)^A(M) | 6927.48 | 22 |
| 17 | 13-20-A | G(M)^C(M)^U(M)^C(M)^U(M)^U(M)^U(M)^G(M)^G(M)^U(M)^G(M)^C(M)^C(M)^C(M)^G(M)^A(M)^A(M)^G(M)^A(M)^U(M) | 6945.10 | 23 |
| 18 | 14-20-A | U(M)^G(M)^C(M)^U(M)^C(M)^U(M)^U(M)^U(M)^G(M)^G(M)^U(M)^G(M)^C(M)^C(M)^C(M)^G(M)^A(M)^A(M)^G(M)^A(M) | 6943.98 | 24 |
| 19 | 15-20-A | A(M)^U(M)^G(M)^C(M)^U(M)^C(M)^U(M)^U(M)^U(M)^G(M)^G(M)^U(M)^G(M)^C(M)^C(M)^C(M)^G(M)^A(M)^A(M)^G(M) | 6944.03 | 25 |
| 20 | 16-20-A | A(M)^A(M)^U(M)^G(M)^C(M)^U(M)^C(M)^U(M)^U(M)^U(M)^G(M)^G(M)^U(M)^G(M)^C(M)^C(M)^C(M)^G(M)^A(M)^A(M) | 6927.74 | 26 |

[Table 2-2]

| Example | Sequence name | Sequence (5' → 3') | Molecular weight (found) | SEQ ID NO |
|---|---|---|---|---|
| 21 | 17-20-A | C(M)^A(M)^A(M)^U(M)^G(M)^C(M)^U(M)^C(M)^U(M)^U(M)^U(M)^G(M)^G(M)^U(M)^G(M)^C(M)^C(M)^C(M)^G(M)^A(M) | 6902.92 | 27 |
| 22 | 18-20-A | A(M)^C(M)^A(M)^A(M)^U(M)^G(M)^C(M)^U(M)^C(M)^U(M)^U(M)^U(M)^G(M)^G(M)^U(M)^G(M)^C(M)^C(M)^C(M)^G(M) | 6903.00 | 28 |

(continued)

| Example | Sequence name | Sequence (5' → 3') | Molecular weight (found) | SEQ ID NO |
|---|---|---|---|---|
| 23 | 19-20-A | A(M)^A(M)^C(M)^A(M)^A(M)^U(M)^G(M)^C(M)^U(M)^C(M)^U(M)^U(M)^U(M)^G(M)^G(M)^U(M)^G(M)^C(M)^C(M)^C(M) | 6889.93 | 29 |
| 24 | 20-20-A | U(M)^A(M)^A(M)^C(M)^A(M)^A(M)^U(M)^G(M)^C(M)^U(M)^C(M)^U(M)^U(M)^U(M)^G(M)^G(M)^U(M)^G(M)^C(M)^C(M) | 6890.72 | 30 |
| 25 | 21-20-A | A(M)^U(M)^A(M)^A(M)^C(M)^A(M)^A(M)^U(M)^G(M)^C(M)^U(M)^C(M)^U(M)^U(M)^U(M)^G(M)^G(M)^U(M)^G(M)^C(M) | 6914.48 | 31 |
| 26 | 22-20-A | U(M)^A(M)^U(M)^A(M)^A(M)^C(M)^A(M)^A(M)^U(M)^G(M)^C(M)^U(M)^C(M)^U(M)^U(M)^U(M)^G(M)^G(M)^U(M)^G(M) | 6918.16 | 32 |
| 27 | 23-20-A | U(M)^U(M)^A(M)^U(M)^A(M)^A(M)^C(M)^A(M)^A(M)^U(M)^G(M)^C(M)^U(M)^C(M)^U(M)^U(M)^U(M)^G(M)^G(M)^U(M) | 6877.29 | 33 |
| 28 | 24-20-A | A(M)^U(M)^U(M)^A(M)^U(M)^A(M)^A(M)^C(M)^A(M)^A(M)^U(M)^G(M)^C(M)^U(M)^C(M)^U(M)^U(M)^U(M)^G(M)^G(M) | 6902.98 | 34 |
| 29 | 25-20-A | G(M)^A(M)^U(M)^U(M)^A(M)^U(M)^A(M)^A(M)^C(M)^A(M)^A(M)^U(M)^G(M)^C(M)^U(M)^C(M)^U(M)^U(M)^U(M)^G(M) | 6899.26 | 35 |
| 30 | 26-20-A | U(M)^G(M)^A(M)^U(M)^U(M)^A(M)^U(M)^A(M)^A(M)^C(M)^A(M)^A(M)^U(M)^G(M)^C(M)^U(M)^C(M)^U(M)^U(M)^U(M) | 6861.06 | 36 |
| 31 | 27-20-A | U(M)^U(M)^G(M)^A(M)^U(M)^U(M)^A(M)^U(M)^A(M)^A(M)^C(M)^A(M)^A(M)^U(M)^G(M)^C(M)^U(M)^C(M)^U(M)^U(M) | 6863.93 | 37 |
| 32 | 28-20-A | C(M)^U(M)^U(M)^G(M)^A(M)^U(M)^U(M)^A(M)^U(M)^A(M)^A(M)^C(M)^A(M)^A(M)^U(M)^G(M)^C(M)^U(M)^C(M)^U(M) | 6861.32 | 38 |
| 33 | 29-20-A | A(M)^C(M)^U(M)^U(M)^G(M)^A(M)^U(M)^U(M)^A(M)^U(M)^A(M)^A(M)^C(M)^A(M)^A(M)^U(M)^G(M)^C(M)^U(M)^C(M) | 6882.53 | 39 |
| 34 | 30-20-A | U(M)^A(M)^C(M)^U(M)^U(M)^G(M)^A(M)^U(M)^U(M)^A(M)^U(M)^A(M)^A(M)^C(M)^A(M)^A(M)^U(M)^G(M)^C(M)^U(M) | 6884.23 | 40 |
| 35 | 31-20-A | G(M)^U(M)^A(M)^C(M)^U(M)^U(M)^G(M)^A(M)^U(M)^U(M)^A(M)^U(M)^A(M)^A(M)^C(M)^A(M)^A(M)^U(M)^G(M)^C(M) | 6923.75 | 41 |
| 36 | 32-20-A | G(M)^G(M)^U(M)^A(M)^C(M)^U(M)^U(M)^G(M)^A(M)^U(M)^U(M)^A(M)^U(M)^A(M)^A(M)^C(M)^A(M)^A(M)^U(M)^G(M) | 6963.70 | 42 |
| 37 | 33-20-A | U(M)^G(M)^G(M)^U(M)^A(M)^C(M)^U(M)^U(M)^G(M)^A(M)^U(M)^U(M)^A(M)^U(M)^A(M)^A(M)^C(M)^A(M)^A(M)^U(M) | 6924.62 | 43 |

(continued)

| Example | Sequence name | Sequence (5' → 3') | Molecular weight (found) | SEQ ID NO |
|---|---|---|---|---|
| 38 | 34-20-A | C(M)^U(M)^G(M)^G(M)^U(M)^A(M)^C(M)^U(M)^U(M)^G(M)^A(M)^U(M)^U(M)^A(M)^U(M)^A(M)^A(M)^C(M)^A(M)^A(M) | 6923.60 | 44 |
| 39 | 35-20-A | A(M)^C(M)^U(M)^G(M)^G(M)^U(M)^A(M)^C(M)^U(M)^U(M)^G(M)^A(M)^U(M)^U(M)^A(M)^U(M)^A(M)^A(M)^C(M)^A(M) | 6924.83 | 45 |
| 40 | 36-20-A | A(M)^A(M)^C(M)^U(M)^G(M)^G(M)^U(M)^A(M)^C(M)^U(M)^U(M)^G(M)^A(M)^U(M)^U(M)^A(M)^U(M)^A(M)^A(M)^C(M) | 6921.14 | 46 |

[Table 2-3]

| Example | Sequence name | Sequence (5' → 3') | Molecular weight (found) | SEQ ID NO |
|---|---|---|---|---|
| 41 | 37-20-A | C(M)^A(M)^A(M)^C(M)^U(M)^G(M)^G(M)^U(M)^A(M)^C(M)^U(M)^U(M)^G(M)^A(M)^U(M)^U(M)^A(M)^U(M)^A(M)^A(M) | 6923.22 | 47 |
| 42 | 38-20-A | U(M)^C(M)^A(M)^A(M)^C(M)^U(M)^G(M)^G(M)^U(M)^A(M)^C(M)^U(M)^U(M)^G(M)^A(M)^U(M)^U(M)^A(M)^U(M)^A(M) | 6900.02 | 48 |
| 43 | 39-20-A | U(M)^U(M)^C(M)^A(M)^A(M)^C(M)^U(M)^G(M)^G(M)^U(M)^A(M)^C(M)^U(M)^U(M)^G(M)^A(M)^U(M)^U(M)^A(M)^U(M) | 6877.81 | 49 |
| 44 | 40-20-A | A(M)^U(M)^U(M)^C(M)^A(M)^A(M)^C(M)^U(M)^G(M)^G(M)^U(M)^A(M)^C(M)^U(M)^U(M)^G(M)^A(M)^U(M)^U(M)^A(M) | 6899.32 | 50 |
| 45 | 41-20-A | A(M)^A(M)^U(M)^U(M)^C(M)^A(M)^A(M)^C(M)^U(M)^G(M)^G(M)^U(M)^A(M)^C(M)^U(M)^U(M)^G(M)^A(M)^U(M)^U(M) | 6895.25 | 51 |
| 46 | 46-20-A | U(M)^A(M)^C(M)^U(M)^U(M)^A(M)^A(M)^U(M)^U(M)^C(M)^A(M)^A(M)^C(M)^U(M)^G(M)^G(M)^U(M)^A(M)^C(M)^U(M) | 6857.81 | 52 |
| 47 | 51-20-A | C(M)^U(M)^C(M)^U(M)^G(M)^U(M)^A(M)^C(M)^U(M)^U(M)^A(M)^A(M)^U(M)^U(M)^C(M)^A(M)^A(M)^C(M)^U(M)^G(M) | 6834.64 | 53 |
| 48 | 56-20-A | U(M)^U(M)^C(M)^U(M)^U(M)^C(M)^U(M)^C(M)^U(M)^G(M)^U(M)^A(M)^C(M)^U(M)^U(M)^A(M)^A(M)^U(M)^U(M)^C(M) | 6751.36 | 54 |
| 49 | 62-20-A | C(M)^A(M)^A(M)^A(M)^C(M)^C(M)^U(M)^U(M)^C(M)^U(M)^U(M)^C(M)^U(M)^C(M)^U(M)^G(M)^U(M)^A(M)^C(M)^U(M) | 6769.19 | 55 |
| 50 | 63-20-A | A(M)^C(M)^A(M)^A(M)^A(M)^C(M)^C(M)^U(M)^U(M)^C(M)^U(M)^U(M)^C(M)^U(M)^C(M)^U(M)^G(M)^U(M)^A(M)^C(M) | 6793.63 | 56 |

(continued)

| Example | Sequence name | Sequence (5' → 3') | Molecular weight (found) | SEQ ID NO |
|---|---|---|---|---|
| 51 | 66-20-A | A(M)^A(M)^A(M)^A(M)^C(M)^A(M)^A(M)^A(M)^C(M) ^C(M)^U(M)^U(M)^C(M)^U(M)^U(M)^C(M)^U(M)^C (M)^U(M)^G(M) | 6840.57 | 57 |
| 52 | 70-20-A | C(M)^U(M)^U(M)^U(M)^A(M)^A(M)^A(M)^A(M)^C(M) ^A(M)^A(M)^A(M)^C(M)^C(M)^U(M)^U(M)^C(M)^U (M)^U(M)^C(M) | 6800.68 | 58 |
| 53 | 1-15-A | G(M)^A(M)^U(M)^A(M)^C(M)^A(M)^G(M)^U(M)^U(M) ^U(M)^U(M)^C(M)^G(M)^A(M)^A(M) | 5194.90 | 59 |
| 54 | 2-15-A | A(M)^G(M)^A(M)^U(M)^A(M)^C(M)^A(M)^G(M)^U(M) ^U(M)^U(M)^U(M)^C(M)^G(M)^A(M) | 5195.06 | 60 |
| 55 | 3-15-A | A(M)^A(M)^G(M)^A(M)^U(M)^A(M)^C(M)^A(M)^G(M) ^U(M)^U(M)^U(M)^U(M)^C(M)^G(M) | 5194.48 | 61 |
| 56 | 4-15-A | G(M)^A(M)^A(M)^G(M)^A(M)^U(M)^A(M)^C(M)^A(M) ^G(M)^U(M)^U(M)^U(M)^U(M)^C(M) | 5194.45 | 62 |
| 57 | 5-15-A | C(M)^G(M)^A(M)^A(M)^G(M)^A(M)^U(M)^A(M)^C(M) ^A(M)^G(M)^U(M)^U(M)^U(M)^U(M) | 5194.46 | 63 |
| 58 | 6-15-A | C(M)^C(M)^G(M)^A(M)^A(M)^G(M)^A(M)^U(M)^A(M) ^C(M)^A(M)^G(M)^U(M)^U(M)^U(M) | 5196.79 | 64 |
| 59 | 7-15-A | C(M)^C(M)^C(M)^G(M)^A(M)^A(M)^G(M)^A(M)^U(M) ^A(M)^C(M)^A(M)^G(M)^U(M)^U(M) | 5195.64 | 65 |
| 60 | 8-15-A | G(M)^C(M)^C(M)^C(M)^G(M)^A(M)^A(M)^G(M)^A(M) ^U(M)^A(M)^C(M)^A(M)^G(M)^U(M) | 5234.46 | 66 |

[Table 2-4]

| Example | Sequence name | Sequence (5' → 3') | Molecular weight (found) | SEQ ID NO |
|---|---|---|---|---|
| 61 | 9-15-A | U(M)^G(M)^C(M)^C(M)^C(M)^G(M)^A(M)^A(M)^G(M) ^A(M)^U(M)^A(M)^C(M)^A(M)^G(M) | 5233.92 | 67 |
| 62 | 10-15-A | G(M)^U(M)^G(M)^C(M)^C(M)^C(M)^G(M)^A(M)^A(M) ^G(M)^A(M)^U(M)^A(M)^C(M)^A(M) | 5234.25 | 68 |
| 63 | 11-15-A | G(M)^G(M)^U(M)^G(M)^C(M)^C(M)^C(M)^G(M)^A(M) ^A(M)^G(M)^A(M)^U(M)^A(M)^C(M) | 5250.48 | 69 |
| 64 | 12-15-A | U(M)^G(M)^G(M)^U(M)^G(M)^C(M)^C(M)^C(M)^G(M) ^A(M)^A(M)^G(M)^A(M)^U(M)^A(M) | 5251.03 | 70 |
| 65 | 13-15-A | U(M)^U(M)^G(M)^G(M)^U(M)^G(M)^C(M)^C(M)^C(M) ^G(M)^A(M)^A(M)^G(M)^A(M)^U(M) | 5227.01 | 71 |
| 66 | 14-15-A | U(M)^U(M)^U(M)^G(M)^G(M)^U(M)^G(M)^C(M)^C(M) ^C(M)^G(M)^A(M)^A(M)^G(M)^A(M) | 5227.14 | 72 |
| 67 | 15-15-A | C(M)^U(M)^U(M)^U(M)^G(M)^G(M)^U(M)^G(M)^C(M) ^C(M)^C(M)^G(M)^A(M)^A(M)^G(M) | 5201.97 | 73 |
| 68 | 16-15-A | U(M)^C(M)^U(M)^U(M)^U(M)^G(M)^G(M)^U(M)^G(M) ^C(M)^C(M)^C(M)^G(M)^A(M)^A(M) | 5163.96 | 74 |

(continued)

| Example | Sequence name | Sequence (5' → 3') | Molecular weight (found) | SEQ ID NO |
|---|---|---|---|---|
| 69 | 17-15-A | C(M)^U(M)^C(M)^U(M)^U(M)^U(M)^G(M)^G(M)^U(M) ^G(M)^C(M)^C(M)^C(M)^G(M)^A(M) | 5139.28 | 75 |
| 70 | 18-15-A | G(M)^C(M)^U(M)^C(M)^U(M)^U(M)^U(M)^G(M)^G(M) ^U(M)^G(M)^C(M)^C(M)^C(M)^G(M) | 5155.50 | 76 |
| 71 | 19-15-A | U(M)^G(M)^C(M)^U(M)^C(M)^U(M)^U(M)^U(M)^G(M) ^G(M)^U(M)^G(M)^C(M)^C(M)^C(M) | 5118.85 | 77 |
| 72 | 20-15-A | A(M)^U(M)^G(M)^C(M)^U(M)^C(M)^U(M)^U(M)^U(M) ^G(M)^G(M)^U(M)^G(M)^C(M)^C(M) | 5142.00 | 78 |
| 73 | 21-15-A | A(M)^A(M)^U(M)^G(M)^C(M)^U(M)^C(M)^U(M)^U(M) ^U(M)^G(M)^G(M)^U(M)^G(M)^C(M) | 5164.34 | 79 |
| 74 | 1-25-A | G(M)^G(M)^U(M)^G(M)^C(M)^C(M)^C(M)^G(M)^A(M) ^A(M)^G(M)^A(M)^U(M)^A(M)^C(M)^A(M)^G(M)^U(M)^ U(M)^U(M)^U(M)^C(M)^G(M)^A(M)^A(M) | 8759.74 | 80 |
| 75 | 3-25-A | U(M)^U(M)^G(M)^G(M)^U(M)^G(M)^C(M)^C(M)^C(M) ^G(M)^A(M)^A(M)^G(M)^A(M)^U(M)^A(M)^C(M)^A(M)^ G(M)^U(M)^U(M)^U(M)^U(M)^C(M)^G(M) | 8715.63 | 81 |
| 76 | 5-25-A | C(M)^U(M)^U(M)^U(M)^G(M)^G(M)^U(M)^G(M)^C(M) ^C(M)^C(M)^G(M)^A(M)^A(M)^G(M)^A(M)^U(M)^A(M)^ C(M)^A(M)^G(M)^U(M)^U(M)^U(M)^U(M) | 8674.34 | 82 |
| 77 | 7-25-A | C(M)^U(M)^C(M)"U(M)^U(M)^U(M)^G(M)^G(M)^U(M) ^G(M)^C(M)^C(M)^C(M)^G(M)^A(M)^A(M)^G(M)^A(M)^ U(M)^A(M)^C(M)^A(M)^G(M)^U(M)^U(M) | 8671.30 | 83 |
| 78 | 9-25-A | U(M)^G(M)^C(M)^U(M)^C(M)^U(M)^U(M)^U(M)^G(M) ^G(M)^U(M)^G(M)^C(M)^C(M)^C(M)^G(M)^A(M)^A(M)^ G(M)^A(M)^U(M)^A(M)^C(M)^A(M)^G(M) | 8709.38 | 84 |
| 79 | 11-25-A | A(M)^A(M)^U(M)^G(M)^C(M)^U(M)^C(M)^U(M)^U(M) ^U(M)^G(M)^G(M)^U(M)^G(M)^C(M)^C(M)^C(M)^G(M) ^ A(M)^A(M)^G(M)^A(M)^U(M)^A(M)^C(M) | 8694.16 | 85 |
| 80 | 1-20-B | 5(m)^5(m)^G(m)^A(m)^A(m)^G(m)^A(m)^T(m)^A(m)^5 (m)^A(m)^G(m)^T(m)^T(m)^T(m)^T(m)^5(m)^G(m)^A ( m)^A(m) | 7968.6 | 107 |

[Table 2-5]

| Example | Sequence name | Sequence (5' → 3') | Molecular weight (found) | SEQ ID NO |
|---|---|---|---|---|
| 81 | 6-20-B | G(m)^G(m)^T(m)^G(m)^5(m)^5(m)^5(m)^G(m)^A(m)^A(m) ^G(m)^A(m)^T(m)^A(m)^5(m)^A(m)^G(m)^T(m)^T(m)^T(m) | 7998.45 | 129 |
| 82 | 7-20-B | T(m)^G(m)^G(m)^T(m)^G(m)^5(m)^5(m)^5(m)^G(m)^A(m) ^A(m)^G(m)^A(m)^T(m)^A(m)^5(m)^A(m)^G(m)^T(m)^T (m) | 7999.87 | 131 |
| 83 | 8-20-D | T(m)^T(m)^G(m)^G(m)^T(m)^G(m)^5(m)^5(m)^5(m)^G(m) ^A(m)^A(m)^G(m)^A(m)^T(m)^A(m)^5(m)^A(m)^G(m)^T (m) | 7998.59 | 133 |

(continued)

| Example | Sequence name | Sequence (5' → 3') | Molecular weight (found) | SEQ ID NO |
|---|---|---|---|---|
| 84 | 9-20-B | T(m)^T(m)^T(m)^G(m)^G(m)^T(m)^G(m)^5(m)^5(m)^5(m)^G(m)^A(m)^A(m)^G(m)^A(m)^T(m)^A(m)^5(m)^A(m)^G(m) | 7999.78 | 135 |
| 85 | 10-20-B | 5(m)^T(m)^T(m)^T(m)^G(m)^G(m)^T(m)^G(m)^5(m)^5(m)^5(m)^G(m)^A(m)^A(m)^G(m)^A(m)^T(m)^A(m)^5(m)^A(m) | 7972.81 | 105 |
| 86 | 12-20-B | 5(m)^T(m)^5(m)^T(m)^T(m)^T(m)^G(m)^G(m)^T(m)^G(m)^5(m)^5(m)^5(m)^G(m)^A(m)^A(m)^G(m)^A(m)^T(m)^A(m) | 7962.51 | 108 |
| 87 | 14-20-B | T(m)^G(m)^5(m)^T(m)^5(m)^T(m)^T(m)^T(m)^G(m)^G(m)^T(m)^G(m)^5(m)^5(m)^5(m)^G(m)^A(m)^A(m)^G(m)^A(m) | 7981.56 | 110 |
| 88 | 15-25-A | T(m)^A(m)^A(m)^5(m)^A(m)^A(m)^T(m)^G(m)^5(m)^T(m)^5(m)^T(m)^T(m)^T(m)^G(m)^G(m)^T(m)^G(m)^5(m)^5(m)^ 5(m)^G(m)^A(m)^A(m)^G(m) | 9981.03 | 86 |
| 89 | 15-24-A | A(m)^A(m)^5(m)^A(m)^A(m)^T(m)^G(m)^5(m)^T(m)^5(m)^T(m)^T(m)^T(m)^G(m)^G(m)^T(m)^G(m)^5(m)^5(m)^5(m)^ G(m)^A(m)^A(m)^G(m) | 9584.24 | 87 |
| 90 | 15-23-A | A(m)^5(m)^A(m)^A(m)^T(m)^G(m)^5(m)^T(m)^5(m)^T(m)^T(m)^T(m)^G(m)^G(m)^T(m)^G(m)^5(m)^5(m)^5(m)^G(m)^ A(m)^A(m)^G(m) | 9181.7 | 88 |
| 91 | 15-22-A | 5(m)^A(m)^A(m)^T(m)^G(m)^5(m)^T(m)^5(m)^T(m)^T(m)^T(m)^G(m)^G(m)^T(m)^G(m)^5(m)^5(m)^5(m)^G(m)^A(m)^ A(m)^G(m) | 8777.73 | 89 |
| 92 | 15-21-A | A(m)^A(m)^T(m)^G(m)^5(m)^T(m)^5(m)^T(m)^T(m)^T(m)^G(m)^G(m)^T(m)^G(m)^5(m)^5(m)^5(m)^G(m)^A(m)^A(m)^ G(m) | 8386.13 | 90 |
| 93 | 15-20-B | A(m)^T(m)^G(m)^5(m)^T(m)^5(m)^T(m)^T(m)^T(m)^G(m)^G(m)^T(m)^G(m)^5(m)^5(m)^5(m)^G(m)^A(m)^A(m)^G(m) | 7982.63 | 112 |
| 94 | 15-19-A | T(m)^G(m)^5(m)^T(m)^5(m)^T(m)^T(m)^T(m)^G(m)^G(m)^T(m)^G(m)^5(m)^5(m)^5(m)^G(m)^A(m)^A(m)^G(m) | 7579.98 | 91 |
| 95 | 15-18-A | G(m)^5(m)^T(m)^5(m)^T(m)^T(m)^T(m)^G(m)^G(m)^T(m)^G(m)^5(m)^5(m)^5(m)^G(m)^A(m)^A(m)^G(m) | 7180.3 | 92 |
| 96 | 15-17-A | 5(m)^T(m)^5(m)^T(m)^T(m)^T(m)^G(m)^G(m)^T(m)^G(m)^5(m)^5(m)^5(m)^G(m)^A(m)^A(m)^G(m) | 6760.06 | 93 |
| 97 | 15-16-A | T(m)^5(m)^T(m)^T(m)^T(m)^G(m)^G(m)^T(m)^G(m)^5(m)^5(m)^5(m)^G(m)^A(m)^A(m)^G(m) | 6370.01 | 94 |
| 98 | 16-20-B | A(m)^A(m)^T(m)^G(m)^5(m)^T(m)^5(m)^T(m)^T(m)^T(m)^G(m)^G(m)^T(m)^G(m)^5(m)^5(m)^5(m)^G(m)^A(m)^A(m) | 7967.47 | 114 |
| 99 | 16-19-A | A(m)^T(m)^G(m)^5(m)^T(m)^5(m)^T(m)^T(m)^T(m)^G(m)^G(m)^T(m)^G(m)^5(m)^5(m)^5(m)^G(m)^A(m)^A(m) | 7563.25 | 95 |
| 100 | 16-18-A | T(m)^G(m)^5(m)^T(m)^5(m)^T(m)^T(m)^T(m)^G(m)^G(m)^T(m)^G(m)^5(m)^5(m)^5(m)^G(m)^A(m)^A(m) | 7160.51 | 96 |

[Table 2-6]

| Example | Sequence name | Sequence (5' → 3') | Molecular weight (found) | SEQ ID NO |
|---|---|---|---|---|
| 101 | 16-17-A | G(m)^5(m)^T(m)^5(m)^T(m)^T(m)^T(m)^G(m)^G(m)^T(m)^G(m)^5(m)^5(m)^5(m)^G(m)^A(m)^A(m) | 6763.91 | 97 |
| 102 | 16-16-A | 5(m)^T(m)^5(m)^T(m)^T(m)^T(m)^G(m)^G(m)^T(m)^G(m)^5(m)^5(m)^5(m)^G(m)^A(m)^A(m) | 6348.15 | 98 |
| 103 | 17-20-B | 5(m)^A(m)^A(m)^T(m)^G(m)^5(m)^T(m)^5(m)^T(m)^T(m)^T(m)^G(m)^G(m)^T(m)^G(m)^5(m)^5(m)^5(m)^G(m)^A(m) | 7954.62 | 116 |
| 104 | 18-20-B | A(m)^5(m)^A(m)^A(m)^T(m)^G(m)^5(m)^T(m)^5(m)^T(m)^T(m)^T(m)^G(m)^G(m)^T(m)^G(m)^5(m)^5(m)^5(m)^G(m) | 7951.6 | 118 |
| 105 | 19-20-B | A(m)^A(m)^5(m)^A(m)^A(m)^T(m)^G(m)^5(m)^T(m)^5(m)^T(m)^T(m)^T(m)^G(m)^G(m)^T(m)^G(m)^5(m)^5(m)^5(m) | 7934.75 | 119 |
| 106 | 21-20-B | A(m)^T(m)^A(m)^A(m)^5(m)^A(m)^A(m)^T(m)^G(m)^5(m)^T(m)^5(m)^T(m)^T(m)^T(m)^G(m)^G(m)^T(m)^G(m)^5(m) | 7947.37 | 120 |
| 107 | 25-20-B | G(m)^A(m)^T(m)^T(m)^A(m)^T(m)^A(m)^A(m)^5(m)^A(m)^A(m)^T(m)^G(m)^5(m)^T(m)^5(m)^T(m)^T(m)^T(m)^G(m) | 7935.52 | 121 |
| 108 | 29-20-B | A(m)^5(m)^T(m)^T(m)^G(m)^A(m)^T(m)^T(m)^A(m)^T(m)^A(m)^A(m)^5(m)^A(m)^A(m)^T(m)^G(m)^5(m)^T(m)^5(m) | 7916.1 | 122 |
| 109 | 30-20-B | T(m)^A(m)^5(m)^T(m)^T(m)^G(m)^A(m)^T(m)^T(m)^A(m)^T(m)^A(m)^A(m)^5(m)^A(m)^A(m)^T(m)^G(m)^5(m)^T(m) | 7918.18 | 123 |
| 110 | 31-20-B | G(m)^T(m)^A(m)^5(m)^T(m)^T(m)^G(m)^A(m)^T(m)^T(m)^A(m)^T(m)^A(m)^A(m)^5(m)^A(m)^A(m)^T(m)^G(m)^5(m) | 7946.8 | 124 |
| 111 | 34-20-B | 5(m)^T(m)^G(m)^G(m)^T(m)^A(m)^5(m)^T(m)^T(m)^G(m)^A(m)^T(m)^T(m)^A(m)^T(m)^A(m)^A(m)^5(m)^A(m)^A(m) | 7943.99 | 125 |
| 112 | 36-20-B | A(m)^A(m)^5(m)^T(m)^G(m)^G(m)^T(m)^A(m)^5(m)^T(m)^T(m)^G(m)^A(m)^T(m)^T(m)^A(m)^T(m)^A(m)^A(m)^5(m) | 7944.95 | 126 |
| 113 | 46-20-B | T(m)^A(m)^5(m)^T(m)^T(m)^A(m)^A(m)^T(m)^T(m)^5(m)^A(m)^A(m)^5(m)^T(m)^G(m)^G(m)^T(m)^A(m)^5(m)^T(m) | 7911.45 | 127 |
| 114 | 51-20-B | 5(m)^T(m)^5(m)^T(m)^G(m)^T(m)^A(m)^5(m)^T(m)^T(m)^A(m)^A(m)^T(m)^T(m)^5(m)^A(m)^A(m)^5(m)^T(m)^G(m) | 7899.54 | 128 |
| 115 | 8-20-E | T(m)^T(m)^G(m)^G(m)^T(m)^G(m)^5(m)^5(m)-5(m)^G(m)^A(m)^A(m)^G(m)^A(m)^T(m)^A(m)^5(m)^A(m)^G(m)^T(m) | 7980.98 | 133 |

(continued)

| Example | Sequence name | Sequence (5' → 3') | Molecular weight (found) | SEQ ID NO |
|---|---|---|---|---|
| 116 | 10-20-C | 5(m)^T(m)^T(m)^T(m)^G(m)^G(m)^T(m)^G(m)^5(m)^5 (m)^5(m)-G(m)^A(m)^A(m)^G(m)^A(m)^T(m)^A(m)^5 (m)^A(m) | 7954.93 | 105 |
| 117 | 12-20-C | 5(m)^T(m)^5(m)^T(m)^T(m)^T(m)^G(m)^G(m)^T(m)^G (m)-5(m)^5(m)^5(m)^G(m)^A(m)^A(m)^G(m)^A(m)^T (m)^A(m) | 7943.55 | 108 |
| 118 | 14-20-C | T(m)^G(m)^5(m)^T(m)^5(m)^T(m)^T(m)^T(m)^G(m)-G (m)^T(m)^G(m)^5(m)^5(m)^5(m)^G(m)^A(m)^A(m)^G (m)^A(m) | 7959.30 | 110 |
| 119 | 16-20-C | A(N)*A(N)*T(N)*G(N)*C(N)*T(N)*C(N)*T(N)*T(N)*T(N) *G(N)*G(N)*T(N)*G(N)*C(N)*C(N)*C(N)*G(N)*A(N)*A (N) | 6782.40 | 115 |
| 120 | 9-20-C | T(N)*T(N)*T(N)*G(N)*G(N)*T((N)*G(N)*C(N)*C(N)*C(N) *G(N)*A(N)*A(N)*G(N)*A(N)*T(N)*A(N)*C(N)*A(N)*G (N) | 6831.45 | 136 |

[Table 2-7]

| Example | Sequence name | Sequence (5' → 3') | Molecular weight (found) | SEQ ID NO |
|---|---|---|---|---|
| 121 | 9-20-D | T(N)*T(N)*T(N)*C(N)*G(N)*T(N)*C(N)*C(N)*C(N)*C(N) *C(N)*A(N)*A(N)*C(N)*A(N)*T(N)*A(N)*G(N)*A(N)*G(N) | 6710.50 | 137 |
| 122 | 6-20-C | G(N)*G(N)*T(N)*G(N)*C(N)*C(N)*C(N)*G(N)*A(N)*A(N) *G(N)*A(N)*T(N)*A(N)*C(N)*A(N)*G(N)*T(N)*T(N)*T(N) | 6828.90 | 130 |
| 123 | 7-20-C | T(N)*G(N)*G(N)*T(N)*G(N)*C(N)*C(N)*C(N)*G(N)*A(N) *A(N)*G(N)*A(N)*T(N)*A(N)*C(N)*A(N)*G(N)*T(N)*T(N) | 6830.90 | 132 |
| 124 | 8-20-F | T(N)*T(N)*G(N)*G(N)*T(N)*G(N)*C(N)*C(N)*C(N)*G(N) *A(N)*A(N)*G(N)*A(N)*T(N)*A(N)*C(N)*A(N)*G(N)*T(N) | 6832.20 | 134 |
| 125 | 10-20-D | C(N)*T(N)*T(N)*T(N)*G(N)*G(N)*T(N)*G(N)*C(N)*C(N) *C(N)*G(N)*A(N)*A(N)*G(N)*A(N)*T(N)*A(N)*C(N)*A(N) | 6792.40 | 106 |
| 126 | 12-20-D | C(N)*T(N)*C(N)*T(N)*T(N)*T(N)*G(N)*G(N)*T(N)*G(N) *C(N)*C(N)*C(N)*G(N)*A(N)*A(N)*G(N)*A(N)*T(N)*A(N) | 6782.40 | 109 |
| 127 | 14-20-D | T(N)*G(N)*C(N)*T(N)*C(N)*T(N)*T(N)*T(N)*G(N)*G(N) *T(N)*G(N)*C(N)*C(N)*C(N)*G(N)*A(N)*A(N)*G(N)*A(N) | 6798.20 | 111 |
| 128 | 15-20-C | A(N)*T(N)*G(N)*C(N)*T(N)*C(N)*T(N)*T(N)*T(N)*G(N) *G(N)*T(N)*G(N)*C(N)*C(N)*C(N)*G(N)*A(N)*A(N)*G (N) | 6798.50 | 113 |
| 129 | 17-20-C | C(N)*A(N)*A(N)*T(N)*G(N)*C(N)*T(N)*C(N)*T(N)*T(N) *T(N)*G(N)*G(N)*T(N)*G(N)*C(N)*C(N)*C(N)*G(N)*A (N) | 6758.60 | 117 |
| 130 | 10-19-A | T(m)^T(m)^T(m)^G(m)^G(m)^T(m)^G(m)^5(m)^5(m)^5 (m)^G(m)^A(m)^A(m)^G(m)^A(m)^T(m)^A(m)^5(m)^A (m ) | 7576.06 | 99 |

(continued)

| Example | Sequence name | Sequence (5' → 3') | Molecular weight (found) | SEQ ID NO |
|---|---|---|---|---|
| 131 | 10-18-A | T(m)^T(m)^G(m)^G(m)^T(m)^G(m)^5(m)^5(m)^5(m)^G (m)^A(m)^A(m)^G(m)^A(m)^T(m)^A(m)^5(m)^A(m) | 7182.37 | 100 |
| 132 | 10-17-A | T(m)^G(m)^G(m)^T(m)^G(m)^5(m)^5(m)^5(m)^G(m)^A (m)^A(m)^G(m)^A(m)^T(m)^A(m)^5(m)^A(m) | 6789.10 | 101 |
| 133 | 10-16-A | G(m)^G(m)^T(m)^G(m)^5(m)^5(m)^5(m)^G(m)^A(m)^A (m)^G(m)^A(m)^T(m)^A(m)^5(m)^A(m) | 6394.41 | 102 |
| 134 | 12-19-A | T(m)^5(m)^T(m)^T(m)^T(m)^G(m)^G(m)^T(m)^G(m)^5 (m)^5(m)^5(m)^G(m)^A(m)^A(m)^G(m)^A(m)^T(m)^A (m ) | 7568.76 | 103 |
| 135 | 12-18-A | 5(m)^T(m)^T(m)^T(m)^G(m)^G(m)^T(m)^G(m)^5(m)^5 (m)^5(m)^G(m)^A(m)^A(m)^G(m)^A(m)^T(m)^A(m) | 7174.73 | 104 |

Herein, any of the following signs or representations is occasionally used to represent the corresponding structure.

[Formula 12]

—

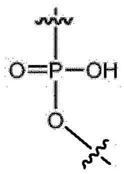

^

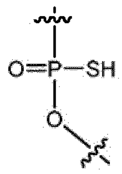

*

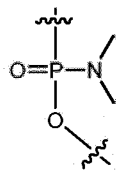

[Formula 13]

a
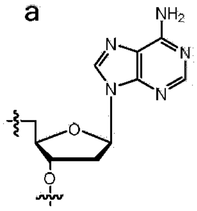
A
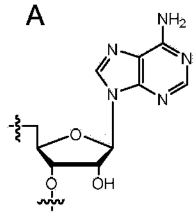
A(M)
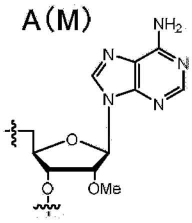
A(m)
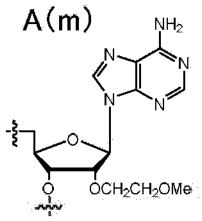
A(L)
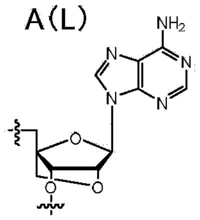

A(Y)
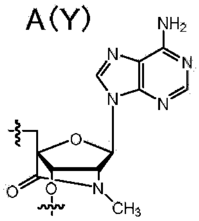
A(Gx)
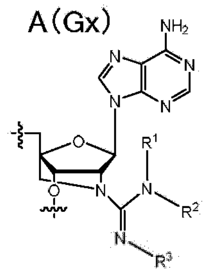
A(S)
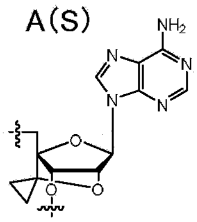
A(Mx)
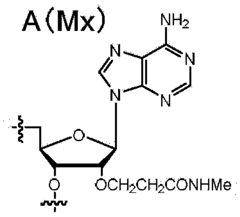

c
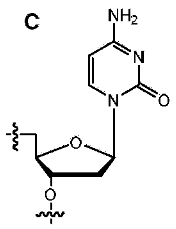
5(x)
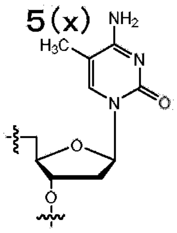
C
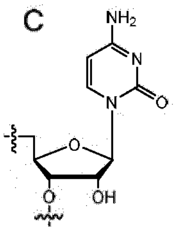
C(M)
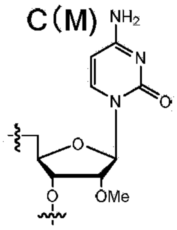
5(m)
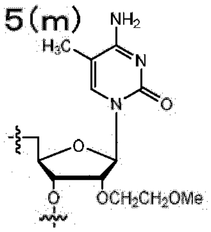

5(L)
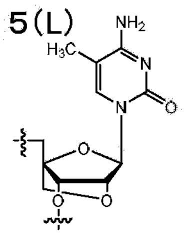
5(Y)
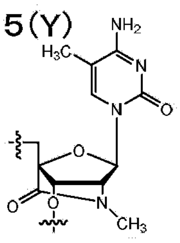
5(Gx)
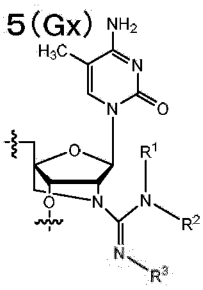
5(S)
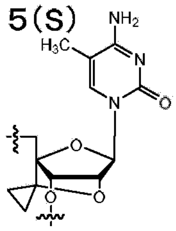
C(Mx)
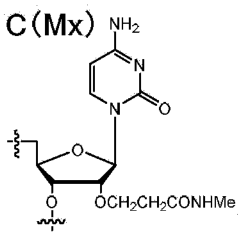

[Formula 14]

g 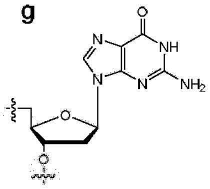 G 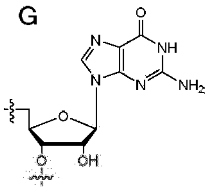 G(M) 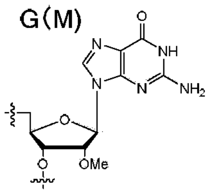 G(m) 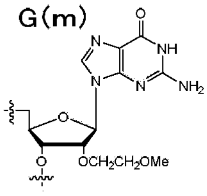 G(L) 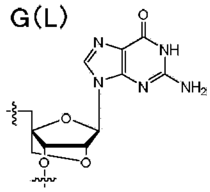

G(Y) 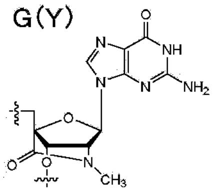 G(Gx) 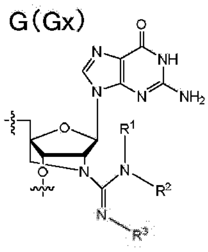 G(S) 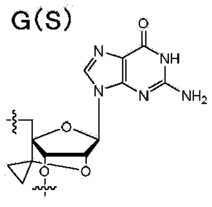 G(Mx) 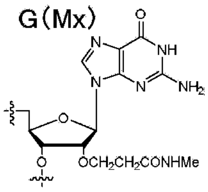

t 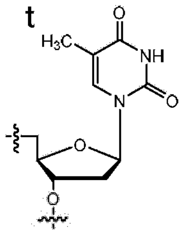 U 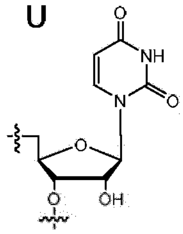 U(M) 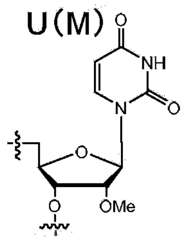 T(m) 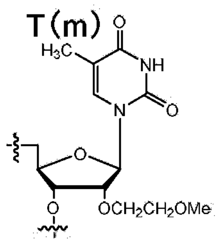 T(L) 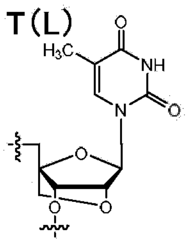

T(Y) 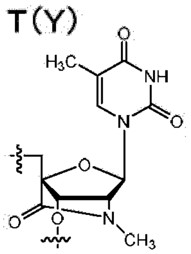 T(Gx) 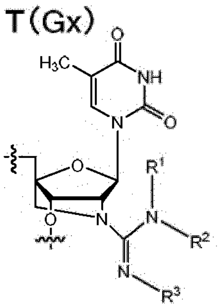 T(S) 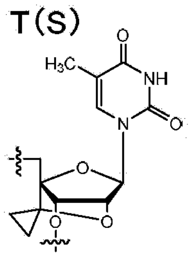 T(Mx) 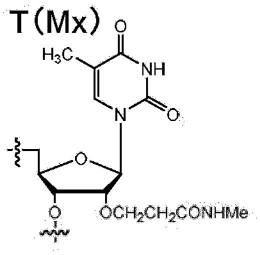

[Formula 15]

A(N) 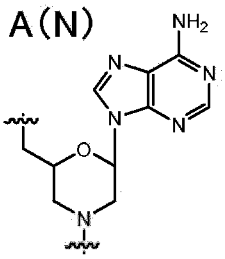 C(N) 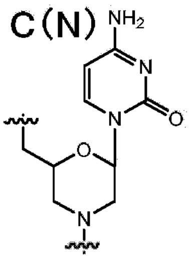 G(N) 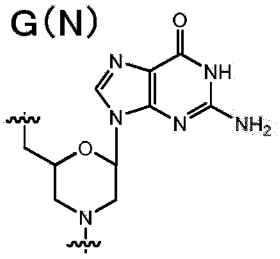 T(N) 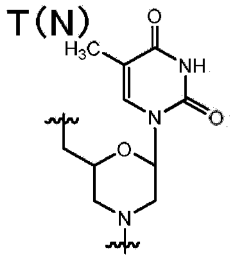

In the structural formulas shown above, $R^1$, $R^2$, and $R^3$ each independently represent a hydrogen atom, a linear or branched alkyl group having one to six carbon atoms, or a cycloalkyl group having three to seven carbon atoms. Here, for GuNA indicated by "Gx" illustrated above, the case that $R^1$ and $R^3$ are each a hydrogen atom and $R^2$ is a methyl group is indicated by "Gm", the case that $R^1$ is a hydrogen atom and $R^2$ and $R^3$ are each a methyl group is indicated by "Gdm", and the case that $R^1$ and $R^3$ are each a hydrogen atom and $R^2$ is a tert-butyl group is indicated by "GtB".

«Evaluation of 27th exon skipping for human WRN gene»

Evaluation of 27th exon skipping for a human WRN gene was performed by checking induction of exon 27

skipping in HEK293T cells and Werner syndrome patient fibroblasts with exon 26 deleted.

Gene expression evaluation in the present Examples is evaluation of the sequence structure of the 27th exon of a WRN gene by measuring the amount of amplification with use of a PCR method for a region ranging from exons 26 (for HEK293T having a normal WRN gene) or 25 (for Werner syndrome patient fibroblasts with exon 26 deleted) to 29 of the WRN gene in complementary DNA (cDNA) given by reverse transcription reaction. Specific procedures of expression evaluations will be described in the following.

(Introduction of antisense oligonucleotide into cells)

"Cells expressing a human WRN gene" were treated with an antisense oligonucleotide by means of introduction by, for example, lipofection or direct addition for 2 days. The cells used were cells expressing a WRN gene (e.g., HEK293T cells or Werner syndrome patient fibroblasts with exon 26 deleted). The cells treated with the antisense oligonucleotide were collected immediately after the treatment, or collected after being continuously cultured with the antisense oligonucleotide removed.

(Analysis of WRN cDNA)

Reverse transcription reaction was performed for the total RNA extracted from the cells collected, and, for the resulting cDNA, a region ranging from exon 26 to exon 29 of a WRN gene (in using HEK293T cells having a normal WRN gene) or a region ranging from exon 25 to exon 29 of a WRN gene (in using Werner syndrome patient fibroblasts with exon 26 deleted) was amplified by using a PCR method. In this amplification, the annealing temperature was set to 60°C.

(Confirmation of skipping of exon 27)

Amplification of the region from exons 26 to 29 of WRN cDNA from HEK293T having a normal WRN gene in the described manner without addition of an antisense oligonucleotide gave a band of 290 base pairs. Determination of the nucleotide sequence of this amplification product by a conventional method showed that the amplification product consisted of exon 26, exon 27, exon 28, and exon 29.

Likewise, amplification of a region from exon 25 to exon 29 of WRN cDNA from Werner syndrome patient fibroblasts with exon 26 deleted without addition of an antisense oligonucleotide gave a band of 290 base pairs. Determination of the nucleotide sequence of this amplification product by a conventional method showed that the amplification product consisted of exon 25, exon 27, exon 28, and exon 29. This result agreed quite well with a result of gene analysis for the patient.

For cDNA from the HEK293T cells with introduction of an antisense oligonucleotide therein, on the other hand, not only an amplification product having the same size as the amplification product given by the cells without introduction of an antisense oligonucleotide therein was given, but also an amplification product of small size was given after culturing for 48 hours. Determination of the nucleotide sequence of this amplification product by a conventional method showed that the amplification product consisted of exon 26, exon 28, and exon 29. Under those conditions, only a transcript of small size, that is, with exon 27 skipped was given.

For cDNA from the Werner syndrome patient fibroblasts described above with introduction of an antisense oligonucleotide therein, an amplification product of smaller size than the amplification product given by the cells without introduction of an antisense oligonucleotide therein was given. Determination of the nucleotide sequence of this amplification product of small size found that the sequence of exon 25 was directly connecting to the sequence of exon 28, revealing deletion of exon 26 and exon 27. This suggests that the antisense oligonucleotide treatment allowed exon 27 to be skipped.

Also for an mRNA precursor transcribed from a gene having the normal human WRN gene structure in splicing reactions, the present inventors confirmed that the designed antisense oligonucleotides effectively induced skipping of exon 27. Also for Werner syndrome patient fibroblasts having the skipping mutation c.3139-1G>C in the 26th exon (Werner syndrome patient fibroblasts with exon 26 deleted), the present inventors confirmed that the designed antisense oligonucleotides effectively induced skipping of exon 27. Hereinafter, the present invention will be described in more detail on the basis of different tests.

«Evaluation of induction of exon 27 skipping for WRN gene»

For each single-stranded antisense oligonucleotide produced, evaluation of induction of exon 27 skipping for a WRN gene was carried out in different ways: evaluation using human embryonic kidney cells, HEK293T cells, and evaluation using Werner syndrome patient fibroblasts having the skip mutation c.3139-1G>C in the 26th exon. The

specific procedures will be described in the following.

<Evaluation of induction of exon 27 skipping using human embryonic kidney cells, HEK293T cells>

Human embryonic kidney cells, HEK293T cells (ATCC (R) CRL-3216 (TM)), were cultured in a culture medium under conditions of 37°C and 5% $CO_2$. The culture medium used for HEK293T cells was one having the following composition.

Composition of culture medium for HEK293T cells

Dulbecco’s modified Eagle’s medium (DMEM): manufactured by Thermo Fisher Scientific, Cat# 11995
10% fetal bovine serum (FBS): manufactured by Thermo Fisher Scientific, Cat# 10437028

First, the day before transfection, HEK293T cells were seeded in a 96-well plate (12000 cells/well), and cultured overnight under conditions of 37°C and 5% $CO_2$. Thereafter, cells were transfected with a single-stranded antisense oligonucleotide diluted with phosphate-buffered saline (PBS) (50 nM) by means of lipofection. Cells transfected with PBS dissolving no single-stranded antisense oligonucleotide therein were used as a negative control group. The cells after transfection were cultured in a culture medium for 48 hours under conditions of 37°C and 5% $CO_2$. Thereafter, the culture medium was removed, and reverse transcription reaction was performed for extracted total RNAs by using a Taqman Fast Cells-to-CT Kit (manufactured by Thermo Fisher Scientific, Cat# 4399003). For the complementary DNAs (cDNAs) resulting from the reverse transcription reaction, PCR was performed over exon 25 to exon 29 of a WRN gene with use of specific primers designed in advance (see below).

The program used for the PCR was as follows.

94°C, for 1 minute: thermal denaturation
[98°C, for 10 seconds; 60°C, for 5 seconds; 68°C, for 40 seconds] × 35 cycles: PCR amplification

The gene-specific primers used were WRN_Exon26_Fw and WRN_Exon29_Rv.

WRN_Exon26_Fw: 5’-CTCAGAGCCTCATCCTTCAAGCTAATG-3’
WRN_Exon29_Rv: 5’-CAATCTGAGTCTCCTGCTCTTGTGC-3’

The reaction products of the PCR reaction were separated by 3% agarose gel electrophoresis, and gel photographs were taken using a photographic system constructed by combining the LED transilluminator GELmieru (Wako Pure Chemical Industries, Ltd.) and an iPhone 8 (Apple Inc.).

Fig. 5 shows the experimental results of measurement of the skipping activities of single-stranded antisense oligonucleotides (Fig. 4) for exon 27 of a human WRN gene as determined for a human WRN mRNA precursor with the method described above.

For a sample without introduction of an antisense oligonucleotide therein (labeled with "WITHOUT ADDITION" in Fig. 5), amplification of a region from exon 26 to exon 29 of WRN cDNA from HEK293T cells having a normal WRN gene gave a band of 291 base pairs. Determination of the nucleotide sequence of this amplification product by DNA sequencing showed that this band consisted of exon 26, exon 27, exon 28, and exon 29.

On the other hand, cDNA from HEK293T cells with the addition of one of the antisense oligonucleotides, not only gave an amplification product of the same size as the amplification product given by the cells without the introduction of an antisense oligonucleotide, but also a small amplification product (215 base pairs) after 48 hours of culture. Determination of the nucleotide sequence of this amplification product by sequencing showed that the amplification product consisted of exon 26, exon 28, and exon 29. Under these conditions, an antisense oligonucleotide was also given with which only found amplification products of small size, that is, with high efficiency skipping activity for exon 27.

The PCR products with exon 27 skipped, "215 bp, A", and the PCR products without exon 27 skipped, "291 bp, B" were quantified by using ImageJ (US NIH). The measurements of "A" and "B" were applied to a calculation formula below to determine the skipping efficiencies (%). The results are shown in Table 3-1 to Table 3-3.

$$\text{Skipping efficiency (\%)} = 100 \times A / (A + B)$$

[Table 3-1]

| Sequence name | SEQ ID NO | WRN Exon27 skipping efficiency @ 50 nM |
|---|---|---|
| (-)10-20-A | 7 | 0.0 |
| (-)5-20-A | 8 | 12.4 |
| 1-20-A | 9 | 100.0 |
| 2-20-A | 10 | 37.6 |
| 3-20-A | 11 | 61.7 |
| 4-20-A | 12 | 59.6 |
| 5-20-A | 13 | 0.0 |
| 6-20-A | 14 | 96.9 |
| 7-20-A | 15 | 93.5 |
| 8-20-A | 16 | 98.8 |
| 8-20-B | 17 | 100.0 |
| 8-20-C | 18 | 0.0 |
| 9-20-A | 19 | 100.0 |
| 10-20-A | 20 | 100.0 |
| 11-20-A | 21 | 0.0 |
| 12-20-A | 22 | 100.0 |
| 13-20-A | 23 | 0.0 |
| 14-20-A | 24 | 100.0 |
| 15-20-A | 25 | 100.0 |
| 16-20-A | 26 | 91.2 |
| 17-20-A | 27 | 92.9 |
| 18-20-A | 28 | 100.0 |
| 19-20-A | 29 | 100.0 |
| 20-20-A | 30 | 36.7 |
| 21-20-A | 31 | 85.1 |
| 22-20-A | 32 | 0.0 |

[Table 3-2]

| Sequence name | SEQ ID NO | WRN Exon27 skipping efficiency @ 50 nM |
|---|---|---|
| 23-20-A | 33 | 0.0 |
| 24-20-A | 34 | 43.6 |
| 25-20-A | 35 | 39.6 |
| 26-20-A | 36 | 0.0 |
| 27-20-A | 37 | 0.0 |
| 28-20-A | 38 | 0.0 |
| 29-20-A | 39 | 100.0 |
| 30-20-A | 40 | 100.0 |

(continued)

| Sequence name | SEQ ID NO | WRN Exon27 skipping efficiency @ 50 nM |
|---|---|---|
| 31-20-A | 41 | 0.0 |
| 32-20-A | 42 | 0.0 |
| 33-20-A | 43 | 0.0 |
| 34-20-A | 44 | 100.0 |
| 35-20-A | 45 | 0.0 |
| 36-20-A | 46 | 0.0 |
| 37-20-A | 47 | 0.0 |
| 38-20-A | 48 | 7.3 |
| 39-20-A | 49 | 0.0 |
| 40-20-A | 50 | 0.0 |
| 41-20-A | 51 | 5.9 |
| 46-20-A | 52 | 0.0 |
| 51-20-A | 53 | 0.0 |
| 56-20-A | 54 | 3.4 |
| 62-20-A | 55 | 39.5 |
| 63-20-A | 56 | 0.0 |
| 66-20-A | 57 | 0.0 |
| 70-20-A | 58 | 0.0 |

[Table 3-3]

| Sequence name | SEQ ID NO | WRN Exon27 skipping efficiency @ 50 nM |
|---|---|---|
| 1-15-A | 59 | 0.0 |
| 2-15-A | 60 | 0.0 |
| 3-15-A | 61 | 0.0 |
| 4-15-A | 62 | 0.0 |
| 5-15-A | 63 | 0.0 |
| 6-15-A | 64 | 0.0 |
| 7-15-A | 65 | 0.0 |
| 8-15-A | 66 | 0.0 |
| 9-15-A | 67 | 0.0 |
| 10-15-A | 68 | 21.6 |
| 11-15-A | 69 | 40.2 |
| 12-15-A | 70 | 17.6 |
| 13-15-A | 71 | 18.1 |
| 14-15-A | 72 | 24.8 |
| 15-15-A | 73 | 91.4 |
| 16-15-A | 74 | 44.5 |

(continued)

| Sequence name | SEQ ID NO | WRN Exon27 skipping efficiency @ 50 nM |
|---|---|---|
| 17-15-A | 75 | 4.0 |
| 18-15-A | 76 | 24.9 |
| 19-15-A | 77 | 0.0 |
| 20-15-A | 78 | 9.4 |
| 21-15-A | 79 | 13.9 |
| 1-25-A | 80 | 100.0 |
| 3-25-A | 81 | 100.0 |
| 5-25-A | 82 | 60.0 |
| 7-25-A | 83 | 100.0 |
| 9-25-A | 84 | 89.8 |
| 11-25-A | 85 | 100.0 |

<Confirmation of the mutated form of the WRN gene in Werner syndrome patient fibroblasts>

Werner syndrome patient fibroblasts (Coriell Cell Repositories, Cat# AG12795) were cultured in a culture medium under conditions of 37°C and 5% $CO_2$. The culture medium used for Werner syndrome patient fibroblasts had the following composition.

Composition of culture medium for Werner syndrome patient fibroblasts Eagle's minimum essential medium (MEM): manufactured by Sigma-Aldrich Co. LLC, Cat# M5650
15% FBS: manufactured by Thermo Fisher Scientific, Cat# 10437028

Gene mutation analysis was performed for the mutated form of a WRN gene in the Werner syndrome patient fibroblasts. Werner syndrome patient fibroblasts were seeded in a 96-well plate (10000 cells/well), and cultured overnight under conditions of 37°C and 5% $CO_2$. Thereafter, genomic DNA of the cells was extracted, and PCR was performed over exon 25 to exon 29 of a WRN gene using specific primers designed in advance (see below).

The program used for the PCR was as follows.

94°C, for 1 minute: thermal denaturation
[98°C, for 10 seconds; 60°C, for 5 seconds; 68°C, for 40 seconds] × 35 cycles: PCR amplification

The gene-specific primers used were WRN_Exon25_Fw and WRN_Exon29_Rv.

WRN_Exon25_Fw: 5'-CTGGCAAGGATCAAACAGAGAGTTG-3'
WRN_Exon29_Rv: 5'-CAATCTGAGTCTCCTGCTCTTGTGC-3'

The reaction products of the PCR reaction were separated by 3% agarose gel electrophoresis, amplified PCR fragments were detected by using the LED transilluminator GELmieru (Wako Pure Chemical Industries, Ltd.), and a portion of the gel containing the PCR fragments was excised. The amplified PCR fragments were purified and subjected to analysis by sequencing, which confirmed that the Werner syndrome patient fibroblasts were a homozygous for c. 3 13 9-1 G>C for a WRN gene.

<Evaluation of induction of exon 27 skipping with Werner syndrome patient fibroblasts>

Next, the induction of exon 27 skipping for a WRN gene was evaluated using the Werner syndrome patient fibroblasts that were a homozygote of c.3139-1G>C for a WRN gene. The day before transfection, Werner syndrome patient fibroblasts were seeded in a 96-well plate (10000 cells/well), and cultured overnight under conditions of 37°C and 5% $CO_2$. The cells were then treated with an antisense oligonucleotide for 48 hours to 10 days by of lipofection. The cells were transfected with a single-stranded antisense oligonucleotide diluted with PBS (final concentration: 1 to 50 nM) by lipofection. Cells transfected with PBS, which does not dissolve single-stranded antisense oligonucleotide, therein were used as a negative control group. The cells treated with the antisense oligonucleotide were cultured in a

culture medium for 48 hours or 240 hours under conditions of 37°C and 5% $CO_2$. The culture medium was then removed, and reverse transcription reaction was performed on extracted total RNAs by using a Taqman Fast Cells-to-CT Kit (manufactured by Thermo Fisher Scientific, Cat# 4399003). For the complementary DNAs (cDNAs) resulting from the reverse transcription reaction, PCR was performed over exon 25 to exon 29 of a WRN gene using specific primers designed in advance (see below). The program used for the PCR was as follows.

94°C, for 1 minute: thermal denaturation
[98°C, for 10 seconds; 60°C, for 5 seconds; 68°C, for 40 seconds] × 35 cycles: PCR amplification

The gene-specific primers used were WRN_Exon25_Fw and WRN_Exon29_Rv.

WRN_Exon25_Fw: 5'-CTGGCAAGGATCAAACAGAGAGTTG-3'
WRN_Exon29_Rv: 5'-CAATCTGAGTCTCCTGCTCTTGTGC-3'

The reaction products of the PCR reaction were separated by 3% agarose gel electrophoresis, and gel photographs were taken using a photographic system constructed by combining the LED transilluminator GELmieru (Wako Pure Chemical Industries, Ltd.) and an iPhone 8 (Apple Inc.).

Fig. 6 shows experimental results of measuring of the skipping activities of single-stranded antisense oligonucleotides for exon 27 of a human WRN gene as determined for a human WRN mRNA precursor by the method described above. For a sample without introduction of an antisense oligonucleotide into it (labelled with "WITHOUT ADDITION" in Fig. 6), amplification of a region from exon 25 to exon 29 of WRN cDNA from Werner syndrome patient fibroblasts gave a band of 352 base pairs. Determination of the nucleotide sequence of this amplification product by sequencing showed that this band consisted of exon 25, exon 27, exon 28, and exon 29. This result was in good agreement with the patient's genetic analysis.

For cDNA from the Werner syndrome patient fibroblasts with addition of an antisense oligonucleotide thereto, a product of smaller size (276 base pairs) than the product given by the cells without the addition of an antisense oligonucleotide therein was given. Determination of the nucleotide sequence of this smaller amplification product by sequencing showed that the sequence of exon 25 was directly linking to the sequence of exon 28, revealing a deletion of exon 26 and exon 27. This suggests that the antisense oligonucleotide treatment allowed exon 27 to be skipped.

The PCR products with exon 27 skipped, "276 bp, A", and the PCR products without exon 27 skipped, "352 bp, B" were quantified by using ImageJ (US NIH). The measurements of "A" and "B" were applied to a calculation formula below to determine the skipping efficiencies (%). The results are shown in Tables 4-1A to 4-1F, Tables 4-2A to 4-2D, Tables 4-3A to 4-3D, Tables 4-4A to 4-4D, and Table 4-5.

$$\text{Skipping efficiency (\%)} = 100 \times A / (A + B)$$

[Table 4-1A]

| Sequence name | SEQ ID NO | WRN Exon27 skipping efficiency @ 50 nM |
|---|---|---|
| (-) 10-20-A | 7 | 8.6 |
| (-)5-20-A | 8 | 74.4 |
| 1-20-A | 9 | 100.0 |
| 2-20-A | 10 | 96.3 |
| 3-20-A | 11 | 100.0 |
| 4-20-A | 12 | 100.0 |
| 5-20-A | 13 | 100.0 |
| 6-20-A | 14 | 96.9 |
| 7-20-A | 15 | 62.2 |
| 8-20-A | 16 | 88.9 |
| 8-20-B | 17 | 100.0 |
| 8-20-C | 18 | 100.0 |

(continued)

| Sequence name | SEQ ID NO | WRN Exon27 skipping efficiency @ 50 nM |
|---|---|---|
| 9-20-A | 19 | 100.0 |
| 10-20-A | 20 | 100.0 |
| 11-20-A | 21 | 100.0 |
| 12-20-A | 22 | 100.0 |
| 13-20-A | 23 | 100.0 |
| 14-20-A | 24 | 100.0 |
| 15-20-A | 25 | 100.0 |
| 16-20-A | 26 | 93.8 |
| 17-20-A | 27 | 66.7 |
| 18-20-A | 28 | 95.6 |
| 19-20-A | 29 | 100.0 |
| 20-20-A | 30 | 100.0 |
| 21-20-A | 31 | 99.6 |

[Table 4-1B]

| Sequence name | SEQ ID NO | WRN Exon27 skipping efficiency @ 50 nM |
|---|---|---|
| 22-20-A | 32 | 93.8 |
| 23-20-A | 33 | 83.4 |
| 24-20-A | 34 | 85.2 |
| 25-20-A | 35 | 100.0 |
| 26-20-A | 36 | 82.7 |
| 27-20-A | 37 | 84.7 |
| 28-20-A | 38 | 82.1 |
| 29-20-A | 39 | 83.5 |
| 30-20-A | 40 | 74.0 |
| 31-20-A | 41 | 98.6 |
| 32-20-A | 42 | 100.0 |
| 33-20-A | 43 | 70.3 |
| 34-20-A | 44 | 100.0 |
| 35-20-A | 45 | 100.0 |
| 36-20-A | 46 | 82.4 |
| 37-20-A | 47 | 100.0 |
| 38-20-A | 48 | 88.7 |
| 39-20-A | 49 | 98.8 |
| 40-20-A | 50 | 96.5 |
| 41-20-A | 51 | 13.7 |
| 46-20-A | 52 | 93.1 |

(continued)

| Sequence name | SEQ ID NO | WRN Exon27 skipping efficiency @ 50 nM |
|---|---|---|
| 51-20-A | 53 | 73.0 |
| 56-20-A | 54 | 54.0 |
| 62-20-A | 55 | 81.0 |
| 63-20-A | 56 | 88.2 |

[Table 4-1C]

| Sequence name | SEQ ID NO | WRN Exon27 skipping efficiency @ 50 nM |
|---|---|---|
| 66-20-A | 57 | 19.3 |
| 70-20-A | 58 | 11.7 |
| 1-15-A | 59 | 25.7 |
| 2-15-A | 60 | 20.0 |
| 3-15-A | 61 | 15.3 |
| 4-15-A | 62 | 10.4 |
| 5-15-A | 63 | 12.1 |
| 6-15-A | 64 | 10.6 |
| 7-15-A | 65 | 21.5 |
| 8-15-A | 66 | 33.3 |
| 9-15-A | 67 | 27.8 |
| 10-15-A | 68 | 55.7 |
| 11-15-A | 69 | 81.2 |
| 12-15-A | 70 | 79.8 |
| 13-15-A | 71 | 73.3 |
| 14-15-A | 72 | 70.9 |
| 15-15-A | 73 | 85.4 |
| 16-15-A | 74 | 61.9 |
| 17-15-A | 75 | 72.8 |
| 18-15-A | 76 | 66.6 |
| 19-15-A | 77 | 50.5 |
| 20-15-A | 78 | 41.1 |
| 21-15-A | 79 | 58.3 |
| 1-25-A | 80 | 100.0 |
| 3-25-A | 81 | 100.0 |

[Table 4-1D]

| Sequence name | SEQ ID NO | WRN Exon27 skipping efficiency @ 50 nM |
|---|---|---|
| 5-25-A | 82 | 100.0 |
| 7-25-A | 83 | 100.0 |

(continued)

| Sequence name | SEQ ID NO | WRN Exon27 skipping efficiency @ 50 nM |
|---|---|---|
| 9-25-A | 84 | 100.0 |
| 11-25-A | 85 | 100.0 |
| 1-20-B | 107 | 100.0 |
| 6-20-B | 129 | 100.0 |
| 7-20-B | 131 | 100.0 |
| 8-20-D | 133 | 100.0 |
| 9-20-B | 135 | 100.0 |
| 10-20-B | 105 | 100.0 |
| 12-20-B | 108 | 100.0 |
| 14-20-B | 110 | 100.0 |
| 15-25-A | 86 | 100.0 |
| 15-24-A | 87 | 100.0 |
| 15-23-A | 88 | 100.0 |
| 15-22-A | 89 | 100.0 |
| 15-21-A | 90 | 100.0 |
| 15-20-B | 112 | 100.0 |
| 15-19-A | 91 | 100.0 |
| 15-18-A | 92 | 100.0 |
| 15-17-A | 93 | 100.0 |
| 15-16-A | 94 | 100.0 |
| 16-20-B | 114 | 100.0 |
| 16-19-A | 95 | 100.0 |
| 16-18-A | 96 | 100.0 |

[Table 4-1E]

| Sequence name | SEQ ID NO | WRN Exon27 skipping efficiency @ 50 nM |
|---|---|---|
| 16-17-A | 97 | 100.0 |
| 16-16-A | 98 | 100.0 |
| 17-20-B | 116 | 100.0 |
| 18-20-B | 118 | 100.0 |
| 19-20-B | 119 | 100.0 |
| 21-20-B | 120 | 100.0 |
| 25-20-B | 121 | 100.0 |
| 29-20-B | 122 | 100.0 |
| 31-20-B | 123 | 100.0 |
| 31-20-B | 124 | 100.0 |
| 34-20-B | 125 | 100.0 |

(continued)

| Sequence name | SEQ ID NO | WRN Exon27 skipping efficiency @ 50 nM |
|---|---|---|
| 36-20-B | 126 | 100.0 |
| 46-20-B | 127 | 100.0 |
| 51-20-B | 128 | 100.0 |
| 8-20-E | 133 | 100.0 |
| 10-20-C | 105 | 100.0 |
| 12-20-C | 108 | 100.0 |
| 14-20-C | 110 | 100.0 |
| 16-20-C | 115 | 62.2 |
| 9-20-C | 136 | 70.2 |
| 9-20-D | 137 | 0.0 |
| 6-20-C | 130 | 60.8 |
| 7-20-C | 132 | 51.7 |
| 8-20-F | 134 | 56.4 |
| 10-20-D | 106 | 34.8 |

[Table 4-1F]

| Sequence name | SEQ ID NO | WRN Exon27 skipping efficiency @ 50 nM |
|---|---|---|
| 12-20-D | 109 | 34.6 |
| 14-20-D | 111 | 28.7 |
| 15-20-C | 113 | 32.5 |
| 17-20-C | 117 | 24.2 |
| 10-19-A | 99 | 100.0 |
| 10-18-A | 100 | 100.0 |
| 10-17-A | 101 | 100.0 |
| 10-16-A | 102 | 100.0 |
| 12-19-A | 103 | 100.0 |
| 12-18-A | 104 | 100.0 |

[Table 4-2A]

| Sequence name | SEQ ID NO | WRN Exon27 skipping efficiency @ 10 nM |
|---|---|---|
| 1-20-A | 9 | 39.7 |
| 6-20-A | 14 | 100.0 |
| 7-20-A | 15 | 57.7 |
| 8-20-A | 16 | 100.0 |
| 8-20-B | 17 | 100.0 |
| 8-20-C | 18 | 100.0 |
| 9-20-A | 19 | 100.0 |

(continued)

| Sequence name | SEQ ID NO | WRN Exon27 skipping efficiency @ 10 nM |
|---|---|---|
| 10-20-A | 20 | 100.0 |
| 12-20-A | 22 | 100.0 |
| 14-20-A | 24 | 100.0 |
| 15-20-A | 25 | 100.0 |
| 16-20-A | 26 | 55.5 |
| 17-20-A | 27 | 100.0 |
| 18-20-A | 28 | 100.0 |
| 19-20-A | 29 | 50.6 |
| 21-20-A | 31 | 70.9 |
| 25-20-A | 35 | 20.3 |
| 29-20-A | 39 | 7.9 |
| 30-20-A | 40 | 10.0 |
| 31-20-A | 41 | 23.9 |
| 34-20-A | 44 | 24.5 |
| 36-20-A | 46 | 29.1 |
| 46-20-A | 52 | 46.8 |
| 51-20-A | 53 | 52.1 |
| 1-25-A | 80 | 89.3 |
| 3-25-A | 81 | 95.5 |

[Table 4-2B]

| Sequence name | SEQ ID NO | WRN Exon27 skipping efficiency @ 10 nM |
|---|---|---|
| 5-25-A | 82 | 85.4 |
| 7-25-A | 83 | 90.5 |
| 9-25-A | 84 | 90.4 |
| 11-25-A | 85 | 75.7 |
| 1-20-B | 107 | 51.9 |
| 6-20-B | 129 | 100.0 |
| 7-20-B | 131 | 100.0 |
| 8-20-D | 133 | 100.0 |
| 9-20-B | 135 | 100.0 |
| 10-20-B | 105 | 100.0 |
| 12-20-B | 108 | 100.0 |
| 14-20-B | 110 | 100.0 |
| 15-25-A | 86 | 100.0 |
| 15-24-A | 87 | 24.1 |
| 15-23-A | 88 | 44.9 |

(continued)

| Sequence name | SEQ ID NO | WRN Exon27 skipping efficiency @ 10 nM |
|---|---|---|
| 15-22-A | 89 | 33.4 |
| 15-21-A | 90 | 71.7 |
| 15-20-B | 112 | 100.0 |
| 15-19-A | 91 | 100.0 |
| 15-18-A | 92 | 74.6 |
| 15-17-A | 93 | 63.5 |
| 15-16-A | 94 | 81.4 |
| 16-20-B | 114 | 77.7 |
| 16-19-A | 95 | 100.0 |
| 16-18-A | 96 | 100.0 |

[Table 4-2C]

| Sequence name | SEQ ID NO | WRN Exon27 skipping efficiency @ 10 nM |
|---|---|---|
| 16-17-A | 97 | 19.4 |
| 16-16-A | 98 | 100.0 |
| 17-20-B | 116 | 62.1 |
| 18-20-B | 118 | 51.3 |
| 19-20-B | 119 | 100.0 |
| 21-20-B | 120 | 86.1 |
| 25-20-B | 121 | 58.5 |
| 29-20-B | 122 | 64.4 |
| 30-20-B | 123 | 62.3 |
| 31-20-B | 124 | 100.0 |
| 34-20-B | 125 | 100.0 |
| 36-20-B | 126 | 33.6 |
| 46-20-B | 127 | 100.0 |
| 51-20-B | 128 | 100.0 |
| 8-20-E | 133 | 66.7 |
| 10-20-C | 105 | 69.8 |
| 12-20-C | 108 | 67.4 |
| 14-20-C | 110 | 89.4 |
| 16-20-C | 115 | 34.3 |
| 9-20-C | 136 | 36.6 |
| 9-20-D | 137 | 0.0 |
| 6-20-C | 130 | 30.3 |
| 7-20-C | 132 | 24.2 |
| 8-20-F | 134 | 22.6 |

(continued)

| Sequence name | SEQ ID NO | WRN Exon27 skipping efficiency @ 10 nM |
|---|---|---|
| 10-20-D | 106 | 18.2 |

[Table 4-2D]

| Sequence name | SEQ ID NO | WRN Exon27 skipping efficiency @ 10 nM |
|---|---|---|
| 12-20-D | 109 | 14.7 |
| 14-20-D | 111 | 12.9 |
| 15-20-C | 113 | 29.3 |
| 17-20-C | 117 | 20.1 |
| 10-19-A | 99 | 62.2 |
| 10-18-A | 100 | 69.4 |
| 10-17-A | 101 | 75.4 |
| 10-16-A | 102 | 58.0 |
| 12-19-A | 103 | 57.2 |
| 12-18-A | 104 | 65.5 |

[Table 4-3A]

| Sequence name | SEQ ID NO | WRN Exon27 skipping efficiency @ 3 nM |
|---|---|---|
| 1-20-A | 9 | 9.3 |
| 6-20-A | 14 | 67.7 |
| 7-20-A | 15 | 50.2 |
| 8-20-A | 16 | 49.2 |
| 8-20-B | 17 | 100.0 |
| 8-20-C | 18 | 63.8 |
| 9-20-A | 19 | 50.7 |
| 10-20-A | 20 | 38.8 |
| 12-20-A | 22 | 71.8 |
| 14-20-A | 24 | 48.6 |
| 15-20-A | 25 | 50.5 |
| 16-20-A | 26 | 22.8 |
| 17-20-A | 27 | 50.1 |
| 18-20-A | 28 | 46.8 |
| 19-20-A | 29 | 46.7 |
| 21-20-A | 31 | 13.2 |
| 25-20-A | 35 | 4.3 |
| 29-20-A | 39 | 13.5 |
| 30-20-A | 40 | 7.7 |
| 31-20-A | 41 | 4.8 |

(continued)

| Sequence name | SEQ ID NO | WRN Exon27 skipping efficiency @ 3 nM |
|---|---|---|
| 34-20-A | 44 | 8.2 |
| 36-20-A | 46 | 14.6 |
| 46-20-A | 52 | 10.5 |
| 51-20-A | 53 | 20.6 |
| 1-25-A | 80 | 33.9 |
| 3-25-A | 81 | 31.6 |
| 5-25-A | 82 | 31.7 |

[Table 4-3B]

| Sequence name | SEQ ID NO | WRN Exon27 skipping efficiency @ 3 nM |
|---|---|---|
| 7-25-A | 83 | 34.2 |
| 9-25-A | 84 | 34.6 |
| 11-25-A | 85 | 36.2 |
| 1-20-B | 107 | 0.0 |
| 6-20-B | 129 | 50.0 |
| 7-20-B | 131 | 42.2 |
| 8-20-D | 133 | 46.5 |
| 9-20-B | 135 | 52.5 |
| 10-20-B | 105 | 79.8 |
| 12-20-B | 108 | 51.3 |
| 14-20-B | 110 | 51.5 |
| 15-25-A | 86 | 79.3 |
| 15-24-A | 87 | 15.1 |
| 15-23-A | 88 | 0.0 |
| 15-22-A | 89 | 9.6 |
| 15-21-A | 90 | 0.0 |
| 15-20-B | 112 | 87.7 |
| 15-19-A | 91 | 60.0 |
| 15-18-A | 92 | 15.3 |
| 15-17-A | 93 | 30.8 |
| 15-16-A | 94 | 16.4 |
| 16-20-B | 114 | 14.3 |
| 16-19-A | 95 | 0.0 |
| 16-18-A | 96 | 31.2 |
| 16-17-A | 97 | 22.5 |

[Table 4-3C]

| Sequence name | SEQ ID NO | WRN Exon27 skipping efficiency @ 3 nM |
|---|---|---|
| 16-16-A | 98 | 15.7 |
| 17-20-B | 116 | 36.4 |
| 18-20-B | 118 | 45.5 |
| 19-20-B | 119 | 42.0 |
| 21-20-B | 120 | 8.8 |
| 25-20-B | 121 | 37.7 |
| 29-20-B | 122 | 11.9 |
| 30-20-B | 123 | 0.0 |
| 31-20-B | 124 | 82.1 |
| 34-20-B | 125 | 100.0 |
| 36-20-B | 126 | 21.2 |
| 46-20-B | 127 | 50.1 |
| 51-20-B | 128 | 0.0 |
| 8-20-E | 133 | 37.9 |
| 10-20-C | 105 | 27.9 |
| 12-20-C | 108 | 61.7 |
| 14-20-C | 110 | 66.9 |
| 16-20-C | 115 | 26.1 |
| 9-20-C | 136 | 26.0 |
| 9-20-D | 137 | 0.0 |
| 6-20-C | 130 | 17.6 |
| 7-20-C | 132 | 7.2 |
| 8-20-F | 134 | 1.3 |
| 10-20-D | 106 | 9.8 |
| 12-20-D | 109 | 13.8 |

[Table 4-3D]

| Sequence name | SEQ ID NO | WRN Exon27 skipping efficiency @ 3 nM |
|---|---|---|
| 14-20-D | 111 | 7.7 |
| 15-20-C | 113 | 0.0 |
| 17-20-C | 117 | 0.0 |
| 10-19-A | 99 | 17.9 |
| 10-18-A | 100 | 26.7 |
| 10-17-A | 101 | 45.5 |
| 10-16-A | 102 | 22.2 |
| 12-19-A | 103 | 28.0 |
| 12-18-A | 104 | 17.2 |

[Table 4-4A]

| Sequence name | SEQ ID NO | WRN Exon27 skipping efficiency @ 1 nM |
|---|---|---|
| 1-20-A | 9 | 5.8 |
| 6-20-A | 14 | 52.2 |
| 7-20-A | 15 | 20.0 |
| 8-20-A | 16 | 45.4 |
| 8-20-B | 17 | 30.8 |
| 8-20-C | 18 | 79.7 |
| 9-20-A | 19 | 25.1 |
| 10-20-A | 20 | 38.1 |
| 14-20-A | 24 | 47.2 |
| 15-20-A | 25 | 25.9 |
| 16-20-A | 26 | 36.7 |
| 17-20-A | 27 | 47.7 |
| 18-20-A | 28 | 47.7 |
| 19-20-A | 29 | 51.4 |
| 21-20-A | 31 | 5.9 |
| 25-20-A | 35 | 2.7 |
| 29-20-A | 39 | 14.7 |
| 30-20-A | 40 | 5.9 |
| 31-20-A | 41 | 1.3 |
| 34-20-A | 44 | 8.1 |
| 36-20-A | 46 | 10.4 |
| 46-20-A | 52 | 5.2 |
| 51-20-A | 53 | 10.1 |
| 1-25-A | 80 | 13.1 |
| 1-20-B | 107 | 0.0 |
| 6-20-B | 129 | 43.6 |

[Table 4-4B]

| Sequence name | SEQ ID NO | WRN Exon27 skipping efficiency @ 1 nM |
|---|---|---|
| 7-20-B | 131 | 36.0 |
| 8-20-D | 133 | 10.5 |
| 9-20-B | 135 | 51.6 |
| 10-20-B | 105 | 50.7 |
| 12-20-B | 108 | 49.8 |
| 14-20-B | 110 | 47.0 |
| 15-25-A | 86 | 66.1 |
| 15-24-A | 87 | 9.0 |

(continued)

| Sequence name | SEQ ID NO | WRN Exon27 skipping efficiency @ 1 nM |
|---|---|---|
| 15-23-A | 88 | 0.0 |
| 15-22-A | 89 | 0.0 |
| 15-21-A | 90 | 0.0 |
| 15-20-B | 112 | 9.2 |
| 15-19-A | 91 | 15.2 |
| 15-18-A | 92 | 0.0 |
| 15-17-A | 93 | 0.0 |
| 15-16-A | 94 | 0.0 |
| 16-20-B | 114 | 18.7 |
| 16-19-A | 95 | 0.0 |
| 16-18-A | 96 | 0.0 |
| 16-17-A | 97 | 0.0 |
| 16-16-A | 98 | 0.0 |
| 17-20-B | 116 | 32.2 |
| 18-20-B | 118 | 46.3 |
| 19-20-B | 119 | 44.0 |
| 21-20-B | 120 | 17.4 |

[Table 4-4C]

| Sequence name | SEQ ID NO | WRN Exon27 skipping efficiency @ 1 nM |
|---|---|---|
| 25-20-B | 121 | 7.9 |
| 29-20-B | 122 | 11.9 |
| 30-20-B | 123 | 0.0 |
| 31-20-B | 124 | 23.2 |
| 34-20-B | 125 | 50.5 |
| 36-20-B | 126 | 0.0 |
| 46-20-B | 127 | 19.0 |
| 51-20-B | 128 | 0.0 |
| 8-20-E | 133 | 22.8 |
| 10-20-C | 105 | 25.8 |
| 12-20-C | 108 | 13.3 |
| 14-20-C | 110 | 11.9 |
| 16-20-C | 115 | 25.7 |
| 9-20-C | 136 | 25.5 |
| 9-20-D | 137 | 0.0 |
| 6-20-C | 130 | 15.7 |
| 7-20-C | 132 | 4.5 |

(continued)

| Sequence name | SEQ ID NO | WRN Exon27 skipping efficiency @ 1 nM |
|---|---|---|
| 8-20-F | 134 | 3.1 |
| 10-20-D | 106 | 5.9 |
| 12-20-D | 109 | 10.0 |
| 14-20-D | 111 | 9.4 |
| 15-20-C | 113 | 0.0 |
| 17-20-C | 117 | 0.0 |
| 10-19-A | 99 | 7.1 |
| 10-18-A | 100 | 15.7 |

[Table 4-4D]

| Sequence name | SEQ ID NO | WRN Exon27 skipping efficiency @ 1 nM |
|---|---|---|
| 10-17-A | 101 | 10.6 |
| 10-16-A | 102 | 9.4 |
| 12-19-A | 103 | 11.5 |
| 12-18-A | 104 | 13.0 |

[Table 4-5]

| Sequence name | SEQ ID NO | WRN Exon27 skipping efficiency @ 10 nM, 240 h |
|---|---|---|
| 8-20-D | 133 | 100.0 |
| 15-25-A | 86 | 100.0 |
| 15-16-A | 94 | 100.0 |
| 16-20-B | 114 | 100.0 |
| 17-20-B | 116 | 100.0 |

<Induction of expression of functional WRN protein with antisense oligonucleotide in Werner syndrome patient fibroblasts>

The evaluation of the induction of the expression of a functional WRN protein by exon 27 skipping for a WRN gene was performed with Werner syndrome patient fibroblasts, that were homozygous for c.3139-1G>C for a WRN gene. The day before evaluation, Werner syndrome patient fibroblasts were seeded in a 96-well plate (10000 cells/well) and cultured overnight under conditions of 37°C and 5% $CO_2$. Thereafter, the cells were transfected with a single-stranded antisense oligonucleotide consisting of a nucleotide sequence set forth in SEQ ID NO: 28 and diluted with PBS (final concentration: 50 nM) by means of lipofection. Cells transfected with PBS in which no single-stranded antisense oligonucleotide was dissolved were used as a negative control group. After transfection, the cells were cultured in a culture medium for 48 hours under conditions of 37°C and 5% $CO_2$. Thereafter, the cells were fixed with 4% paraformaldehyde (FUJIFILM Wako Pure Chemical Corporation, Cat# 163-20145) for 15 minutes, and incubated with 0.5% Triton-X (manufactured by NACALAI TESQUE, INC., Cat# 12967-45) for 10 minutes. Subsequently, the incubated cells were blocked with PBS containing 5% goat serum for 1 hour, and incubated in anti-WRN antibody (manufactured by Cell Signaling Technology, Inc., Cat# 4666) solution overnight. The cells were further incubated in anti-mouse IgG antibody (manufactured by Thermo Fisher Scientific, A32766) solution for 1 hour. Finally, the cells were incubated in Hoechst (R) 33342 nucleic acid staining (manufactured by Thermo Fisher Scientific, Cat# H3570) solution for 15 minutes, and observed under a fluorescence microscope.

Fig. 7 shows a result of induction of expression of a functional WRN protein by the skipping activity of the single-stranded antisense oligonucleotide for exon 27 of a WRN gene as determined for a human WRN mRNA precursor by the method described above. It is apparent from Fig. 7 that the cells transfected with the single-stranded antisense oligonucleotide consisting of the nucleotide sequence set forth in SEQ ID NO: 28 ("ASO: No. 28" in Fig. 7) have undergone nuclear localization of functional WRN protein.

<Promotion of cell growth with an antisense oligonucleotide in Werner syndrome patient fibroblasts>

Evaluation of cell growth ability by exon 27 skipping for a WRN gene was performed using Werner syndrome patient fibroblasts that were homozygous for c.3139-1G>C for a human WRN gene. The day before evaluation, Werner syndrome patient fibroblasts were seeded in a 96-well plate (12000 cells/well), and cultured overnight under conditions of 37°C and 5% $CO_2$. Thereafter, the cells were transfected with a single-stranded antisense oligonucleotide diluted with PBS (final concentration: 10 nM) by means of lipofection. Cells transfected with PBS, which does not dissolve single-stranded antisense oligonucleotide therein were used as a negative control group. The cells after transfection were cultured in culture medium for 288 hours under conditions of 37°C and 5% $CO_2$. Thereafter, Celltiter-Glo 2.0 Assay (manufactured by Promega Corporation, Cat# G9242) was added to the culture medium, and the cell number was determined. The measurements of cell count of the cells transfected with the single-stranded antisense oligonucleotide, "A", and the cell count of the negative control group, "B", were applied to a calculation formula below to determine the cell growth rate (%). The results are shown in Table 5.

$$\text{Cell growth rate (\%)} = 100 \times (A) / B$$

[Table 5]

| Sequence Name | SEQ ID NO | Cell Growth Rate (%) n=3 @10nM |
|---|---|---|
| 8-20-A | 16 | 162.6 |
| 10-20-A | 20 | 168.2 |
| 14-20-A | 24 | 122.5 |
| 16-20-A | 26 | 177.5 |
| 70-20-A | 58 | 103.3 |
| 8-20-D | 133 | 164.6 |
| 10-20-B | 105 | 179.7 |
| 14-20-B | 110 | 146.4 |
| 15-25-A | 86 | 155.8 |
| 15-16-A | 94 | 173.8 |
| 16-20-B | 114 | 169.5 |

«Evaluation of the cytotoxicity of single-stranded antisense oligonucleotides»

HepG2 cells, a cell strain derived from human liver cancer, were cultured in a growth medium under conditions of 37°C and 5% $CO_2$. The growth medium used had the following composition.

Composition of growth medium used for the evaluation of cytotoxicity

10% FBS: manufactured by Gibco, CAT# 10270-106
Minimum Essential Medium (containing L-glutamine and phenol red) (manufactured by Gibco, CAT# 11095-080)

The day before the experiment, the cells were seeded in a 96-well plate (1.5 × 104 cells/well). The seeded cells were cultured overnight under conditions of 37°C and 5% CO2, and then transfected by lipofection with a single-stranded antisense oligonucleotide diluted in PBS (final concentration: 3 to 30 nM). After the addition, the cells were

cultured for 24 hours under conditions of 37°C and 5% CO2. Cells transfected with PBS without single-stranded antisense oligonucleotide therein were used as a negative control group. Thereafter, a Caspase-Glo 3/7 Assay System (manufactured by Promega Corporation, Cat# G8093) was added to the growth medium, and the caspase activity was evaluated. Table 6 shows the caspase activities of different single-stranded antisense oligonucleotides as determined by the method described above.

[Table 6]

| Sequence Name | SEQ ID NO | Caspase Activity (%) n=3 @3nM | Caspase Activity (%) n=3 @10nM | Caspase Activity (%) n=3 @30nM |
|---|---|---|---|---|
| 6-20-A | 14 | 101.0 | 102.5 | 113.1 |
| 7-20-A | 15 | 102.5 | 106.0 | 122.0 |
| 8-20-A | 16 | 99.3 | 105.7 | 107.3 |
| 9-20-A | 19 | 104.9 | 100.0 | 103.6 |
| 10-20-A | 20 | 99.1 | 100.8 | 112.1 |
| 12-20-A | 22 | 107.2 | 107.1 | 120.0 |
| 14-20-A | 24 | 103.8 | 110.0 | 119.2 |
| 29-20-A | 39 | 97.6 | 103.4 | 99.8 |
| 6-20-B | 129 | 99.7 | 97.9 | 110.8 |
| 7-20-B | 131 | 97.1 | 100.2 | 106.8 |
| 8-20-D | 133 | 99.0 | 106.9 | 111.8 |
| 9-20-B | 135 | 110.4 | 110.2 | 117.9 |
| 10-20-B | 105 | 98.8 | 107.7 | 109.5 |
| 12-20-B | 108 | 101.5 | 100.2 | 112.9 |
| 14-20-B | 110 | 101.3 | 107.0 | 111.0 |
| 15-20-B | 112 | 99.5 | 110.1 | 111.0 |
| 15-19-A | 91 | 99.7 | 104.3 | 106.6 |
| 15-16-A | 94 | 98.8 | 97.5 | 103.8 |
| 19-20-B | 119 | 106.4 | 104.5 | 106.3 |
| 21-20-B | 120 | 105.4 | 109.6 | 109.6 |
| 29-20-B | 122 | 102.3 | 100.6 | 101.8 |
| 30-20-B | 123 | 102.0 | 105.3 | 103.2 |
| 31-20-B | 124 | 105.5 | 104.5 | 114.0 |
| 34-20-B | 125 | 102.0 | 104.9 | 116.3 |
| 46-20-B | 127 | 96.6 | 96.9 | 98.8 |

While embodiments and Examples of the present invention have been thus described as above, combining any of the configurations of the embodiments and Examples as appropriate has been originally contemplated, too.

The embodiments and Examples disclosed herein are exemplary in all aspects, and should be regarded as being non-limiting. The scope of the present invention is shown not by the embodiments and Examples described above but by claims, and intended to include all modifications within a meaning and scope equivalent to those of claims.

## Claims

**1.** An oligonucleotide that expresses a functional human WRN protein against a skip mutation in a 26th or 28th exon

of a human WRN gene, or a pharmaceutically acceptable salt thereof, wherein

one nucleotide is bound to another via a phosphate group and/or modified phosphate group in the oligonucleotide,
the oligonucleotide contains a modified nucleic acid having at least one modified sugar,
the oligonucleotide is 10 to 30mer in nucleotide length, and
the nucleotide sequence of the oligonucleotide is:

a nucleotide sequence having a sequence identity of 90% or more and 100% or less with a nucleotide sequence complementary to at least one target region having the same nucleotide length as the oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6;
a nucleotide sequence complementary to a nucleotide sequence formed by deleting, substituting, inserting, or adding one or several nucleotides in the target region; or
a nucleotide sequence hybridizable with an oligonucleotide having the target region under stringent conditions.

2. The oligonucleotide or a pharmaceutically acceptable salt thereof according to claim 1, wherein the nucleotide sequence of the oligonucleotide is a nucleotide sequence having a sequence identity of 95% or more and 100% or less with a nucleotide sequence complementary to at least one target region having the same nucleotide length as the oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6.

3. The oligonucleotide or a pharmaceutically acceptable salt thereof according to claim 1, wherein the nucleotide sequence of the oligonucleotide is a nucleotide sequence complementary to at least one target region having the same nucleotide length as the oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6.

4. The oligonucleotide or a pharmaceutically acceptable salt thereof according to any one of claims 1 to 3, wherein the oligonucleotide is 15 to 25mer in nucleotide length.

5. The oligonucleotide or a pharmaceutically acceptable salt thereof according to any one of claims 1 to 4, wherein a sugar constituting the oligonucleotide is D-ribofuranose, and modification of a sugar is sugar modification on a hydroxy group at position 2' of the D-ribofuranose.

6. The oligonucleotide or a pharmaceutically acceptable salt thereof according to any one of claims 1 to 5, wherein modification of a sugar constituting the oligonucleotide is made to form a group represented by the following formula (A1):

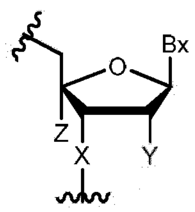

(A1)

wherein Bx is a nucleobase, being independently in each occurrence a group formed from adenine, guanine, cytosine, 5-methylcytosine, thymine, or uracil; X is a phosphate bond, being independently in each occurrence a phosphodiester bond, a phosphorothioate bond, a phosphorodithioate bond, a phosphoamidate bond, a boranophosphate bond, or an alkylphosphonate bond; Y is independently in each occurrence a hydrogen atom, a hydroxy group, a fluorine atom, an optionally substituted alkoxy group having one to six carbon atoms, or an optionally substituted amino group; and Z is independently in each occurrence a hydrogen atom, or an alkyl group having one to five carbon atoms and optionally having a carbon-oxygen double bond or a cyclic structure, or a certain carbon atom in the alkyl group and Y are taken together to form a ring.

7. The oligonucleotide or a pharmaceutically acceptable salt thereof according to any one of claims 1 to 6, wherein modification of a sugar constituting the oligonucleotide is made to form a group represented by the following formula (A2) or formula (A3):

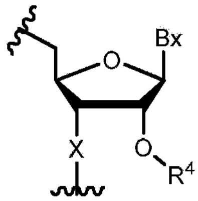

(A2)

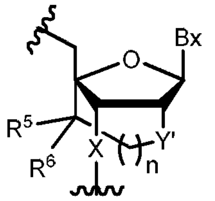

(A3)

wherein Bx is a nucleobase, being independently in each occurrence a group formed from adenine, guanine, cytosine, 5-methylcytosine, thymine, or uracil; X is a phosphate bond, being independently in each occurrence a phosphodiester bond, a phosphorothioate bond, a phosphorodithioate bond, a phosphoamidate bond, a boranophosphate bond, or an alkylphosphonate bond; $R^4$ is independently in each occurrence a hydrogen atom or an optionally substituted alkyl group having one to six carbon atoms; Y' is an oxygen atom or an optionally substituted nitrogen atom; $R^5$ and $R^6$ are each independently in each occurrence a hydrogen atom or an optionally substituted alkyl group having one to six carbon atoms, or $R^5$ and $R^6$ are taken together to form a carbonyl group or a ring; and n is 0 or 1.

**8.** The oligonucleotide or a pharmaceutically acceptable salt thereof according to claim 7, wherein modification of a sugar constituting the oligonucleotide is made to form a group represented by the formula (A2), and the $R^4$ is independently in each occurrence a methyl group, a methoxyethyl group, or an N-methylpropanamide group.

**9.** The oligonucleotide or a pharmaceutically acceptable salt thereof according to claim 7, wherein modification of a sugar constituting the oligonucleotide is made to form a group represented by the formula (A3), the Y' is an oxygen atom, or a nitrogen atom optionally substituted with a hydrogen atom, a methyl group, a methoxyethyl group, or an N-methylpropanamide group, the $R^5$ and $R^6$ are each independently in each occurrence a hydrogen atom or an alkyl group having one or two carbon atoms, or the $R^5$ and $R^6$ are taken together to form a carbonyl group or a ring having three to six carbon atoms, and n is 0 or 1.

**10.** The oligonucleotide or a pharmaceutically acceptable salt thereof according to any one of claims 1 to 9, wherein at least one internucleotide bond in the oligonucleotide is a phosphorothioate bond.

**11.** The oligonucleotide or a pharmaceutically acceptable salt thereof according to any one of claims 1 to 10, wherein at least one internucleotide bond in the oligonucleotide is a phosphodiester bond.

**12.** The oligonucleotide or a pharmaceutically acceptable salt thereof according to any one of claims 1 to 9, wherein an internucleotide bond in the oligonucleotide is a phosphodiester bond or a phosphorothioate bond.

**13.** The oligonucleotide or a pharmaceutically acceptable salt thereof according to any one of claims 1 to 4, wherein the nucleotide sequence of the oligonucleotide is such that the oligonucleotide is composed of a morpholino nucleic acid represented by a formula (A4) below, and is a morpholino oligonucleic acid having a nucleotide sequence complementary to at least one target region having the same nucleotide length as the oligonucleotide in a nucleotide sequence set forth in SEQ ID NO: 1, SEQ ID NO: 2, SEQ ID NO: 3, SEQ ID NO: 4, SEQ ID NO: 5, or SEQ ID NO: 6:

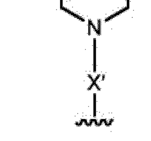

(A4)

wherein Bx is a nucleobase, being independently in each occurrence a group formed from adenine, guanine, cytosine, 5-methylcytosine, thymine, or uracil; and X' is a phosphate bond, being independently in each occurrence a phosphorodiamidate bond, a phosphorodiamidothioate bond, or a phosphorodiamidodithioate bond.

**14.** The oligonucleotide or a pharmaceutically acceptable salt thereof according to any one of claims 1 to 13, wherein the nucleotide sequence of the oligonucleotide is:

a nucleotide sequence having a sequence identity of 90% or more and 100% or less with a nucleotide sequence complementary to a target region composed of continuous 15 to 25mer starting from a nucleotide at any of positions 1 to 40, 46, 51, 56, and 62 to 63 counted from a 5' end in a nucleotide sequence set forth in SEQ ID NO: 1 or a target region composed of continuous 15 to 25mer starting from a nucleotide at any of positions 108873, 108878 to 108917, 108923, 108928, 108933, and 108939 to 108940 counted from a 5' end in a nucleotide sequence set forth in SEQ ID NO: 2;
a nucleotide sequence complementary to a nucleotide sequence formed by deleting, substituting, inserting, or adding one or several nucleotides in the target region; or
a nucleotide sequence hybridizable with an oligonucleotide having the target region under stringent conditions.

**15.** The oligonucleotide or a pharmaceutically acceptable salt thereof according to any one of claims 1 to 14, wherein the nucleotide sequence of the oligonucleotide is a nucleotide sequence having a sequence identity of 90% or more and 100% or less with a nucleotide sequence complementary to a target region composed of continuous 15 to 25mer starting from a nucleotide at any of positions 1 to 32, 34 to 40, 46, 51, and 62 to 63 counted from a 5' end in a nucleotide sequence set forth in SEQ ID NO: 1 or a target region composed of continuous 15 to 25mer starting from a nucleotide at any of positions 108878 to 108909, 108911 to 108917, 108923, 108928, and 108939 to 108940 counted from 5' end in a nucleotide sequence set forth in SEQ ID NO: 2.

**16.** The oligonucleotide or a pharmaceutically acceptable salt thereof according to any one of claims 1 to 15, wherein the nucleotide sequence of the oligonucleotide is a nucleotide sequence having a sequence identity of 90% or more and 100% or less with a nucleotide sequence complementary to a target region composed of continuous 15 to 25mer starting from a nucleotide at any of positions 1, 3, 5 to 12, 14 to 19, 21, 25, 29, 30, 31, 34, 46, and 51 counted from a 5' end in a nucleotide sequence set forth in SEQ ID NO: 1 or a target region composed of continuous 15 to 25mer starting from a nucleotide at any of positions 108878, 108880, 108882 to 108889, 108891 to 108896, 108898, 108902, 108906 to 108908, 108911, 108923, and 108928 counted from a 5' end in a nucleotide sequence set forth in SEQ ID NO: 2.

**17.** The oligonucleotide or a pharmaceutically acceptable salt thereof according to any one of claims 1 to 16, wherein the nucleotide sequence of the oligonucleotide is one nucleotide sequence selected from the group consisting of nucleotide sequences set forth in SEQ ID NOs: 9 to 14, 16 to 26, 28 to 39, 41 to 42, 44 to 50, 52, 55 to 56, 69, 73, 80 to 105, 107 to 108, 110, 112, 114, 116, 118 to 131, 133, and 135.

**18.** The oligonucleotide or a pharmaceutically acceptable salt thereof according to any one of claims 1 to 17, wherein the oligonucleotide is one oligonucleotide selected from the group consisting of 6-20-A, 8-20-A, 8-20-B, 8-20-C, 9-20-A, 10-20-A, 12-20-A, 14-20-A, 16-20-A, 17-20-A, 18-20-A, 19-20-A, 15-25-A, 10-17-A, 10-20-B, 12-20-B, 14-20-B, 15-20-B, 16-20-B, 19-20-B, 31-20-B, 34-20-B, 6-20-B, 7-20-B, 8-20-D, and 9-20-B as sequence names shown in Table 2-1 to Table 2-7.

**19.** The oligonucleotide or a pharmaceutically acceptable salt thereof according to any one of claims 1 to 18, wherein the oligonucleotide is a single-stranded antisense oligonucleotide.

**20.** A double-stranded antisense oligonucleotide comprising the oligonucleotide according to claim 19 and a second-strand oligonucleotide hybridizing with the single-stranded antisense oligonucleotide, or a pharmaceutically acceptable salt thereof, wherein
the nucleotide sequence of the second-strand oligonucleotide is a nucleotide sequence having a sequence identity of 90% or more and 100% or less with a nucleotide sequence complementary to the nucleotide sequence of the single-stranded antisense oligonucleotide.

**21.** An oligonucleotide complex or a pharmaceutically acceptable salt thereof, the oligonucleotide complex comprising:

the oligonucleotide or a pharmaceutically acceptable salt thereof according to any one of claims 1 to 19, or the

double-stranded antisense oligonucleotide or a pharmaceutically acceptable salt thereof according to claim 20; and

an additive substance bound to the oligonucleotide or the second-strand oligonucleotide directly or via a linker bond, wherein

the additive substance is selected from the group consisting of polyethylene glycol, a peptide, an alkyl chain, a ligand compound, an antibody, a protein, and a sugar chain, and

the linker bond is independently in each occurrence a phosphodiester bond, a phosphorothioate bond, a phosphorodithioate bond, a phosphoamidate bond, a boranophosphate bond, an alkylphosphonate bond, a phosphorodiamidate bond, a phosphorodiamidothioate bond, or a phosphorodiamidodithioate bond.

**22.** A pharmaceutical product comprising the oligonucleotide or a pharmaceutically acceptable salt thereof according to any one of claims 1 to 19, the double-stranded antisense oligonucleotide or a pharmaceutically acceptable salt thereof according to claim 20, or the oligonucleotide complex or a pharmaceutically acceptable salt thereof according to claim 21 as an active ingredient.

**23.** A skipping agent for a 27th exon of a human WRN gene, the skipping agent comprising the oligonucleotide or a pharmaceutically acceptable salt thereof according to any one of claims 1 to 19, the double-stranded antisense oligonucleotide or a pharmaceutically acceptable salt thereof according to claim 20, or the oligonucleotide complex or a pharmaceutically acceptable salt thereof according to claim 21 as an active ingredient.

**24.** A functional WRN recovery agent that possesses a nuclear localization signal by virtue of skipping of a 27th exon of a human WRN gene, the functional WRN recovery agent comprising the oligonucleotide or a pharmaceutically acceptable salt thereof according to any one of claims 1 to 19, the double-stranded antisense oligonucleotide or a pharmaceutically acceptable salt thereof according to claim 20, or the oligonucleotide complex or a pharmaceutically acceptable salt thereof according to claim 21 as an active ingredient.

**25.** A therapeutic agent for Werner syndrome, the therapeutic agent comprising the oligonucleotide or a pharmaceutically acceptable salt thereof according to any one of claims 1 to 19, the double-stranded antisense oligonucleotide or a pharmaceutically acceptable salt thereof according to claim 20, or the oligonucleotide complex or a pharmaceutically acceptable salt thereof according to claim 21 as an active ingredient.

**26.** A prophylactic agent for Werner syndrome, the prophylactic agent comprising the oligonucleotide or a pharmaceutically acceptable salt thereof according to any one of claims 1 to 19, the double-stranded antisense oligonucleotide or a pharmaceutically acceptable salt thereof according to claim 20, or the oligonucleotide complex or a pharmaceutically acceptable salt thereof according to claim 21 as an active ingredient.

**27.** A method for treating or preventing Werner syndrome, the method comprising administering the oligonucleotide or a pharmaceutically acceptable salt thereof according to any one of claims 1 to 19, the double-stranded antisense oligonucleotide or a pharmaceutically acceptable salt thereof according to claim 20, or the oligonucleotide complex or a pharmaceutically acceptable salt thereof according to claim 21 to an individual.

**28.** The oligonucleotide or a pharmaceutically acceptable salt thereof according to any one of claims 1 to 19, the double-stranded antisense oligonucleotide or a pharmaceutically acceptable salt thereof according to claim 20, or the oligonucleotide complex or a pharmaceutically acceptable salt thereof according to claim 21 for use in treating or preventing Werner syndrome.

**29.** The oligonucleotide or a pharmaceutically acceptable salt thereof according to any one of claims 1 to 19, the double-stranded antisense oligonucleotide or a pharmaceutically acceptable salt thereof according to claim 20, or the oligonucleotide complex or a pharmaceutically acceptable salt thereof according to claim 21 for use in producing a therapeutic agent or prophylactic agent for Werner syndrome.

FIG.1

RQC : RecQ C-terminal
HRDC : helicase-and-ribonuclease D-C-terminal
NLC : nuclear localization signal

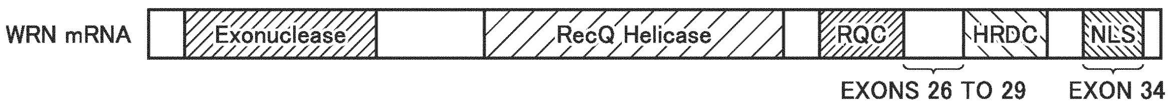

FIG.2

<WERNER SYNDROME PATIENT HAVING c.3139-1G>C MUTATION>

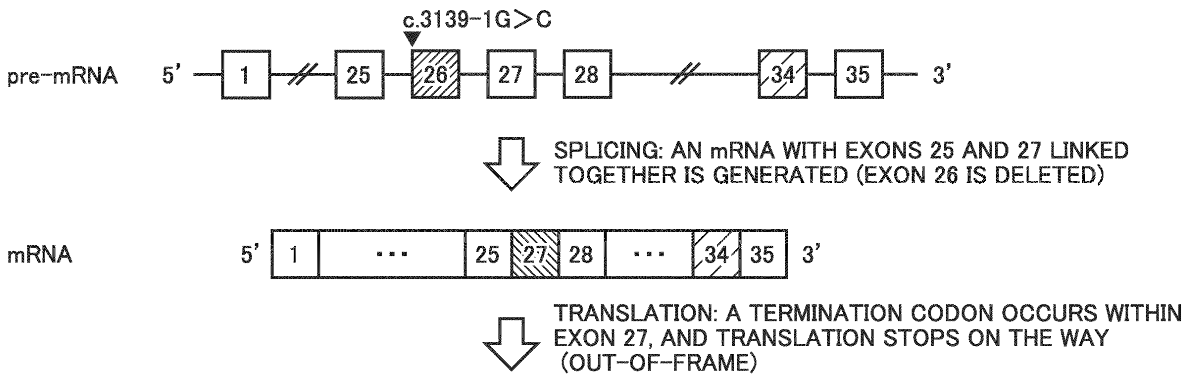


PROTEIN THE PART IN THE C-TERMINAL SIDE IS LOST AND A WRN PROTEIN LACKING THE NUCLEAR LOCALIZATION SIGNAL IS GENERATED
→ THE ORIGINAL PHYSIOLOGICAL FUNCTIONS CANNOT BE EXHIBITED

FIG.3

<EXON 27 SKIPPING FOR c.3139-1G>C MUTATION>

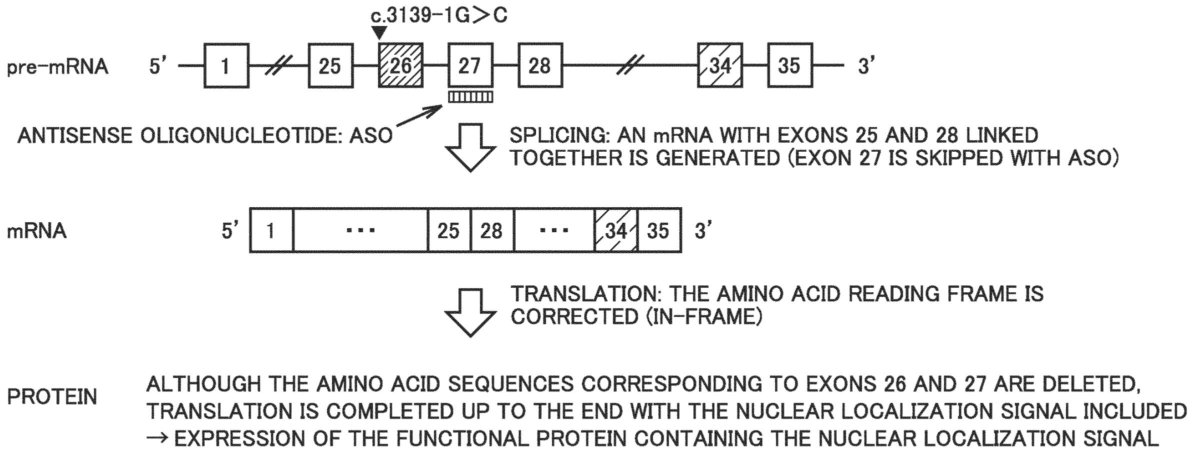

FIG.4

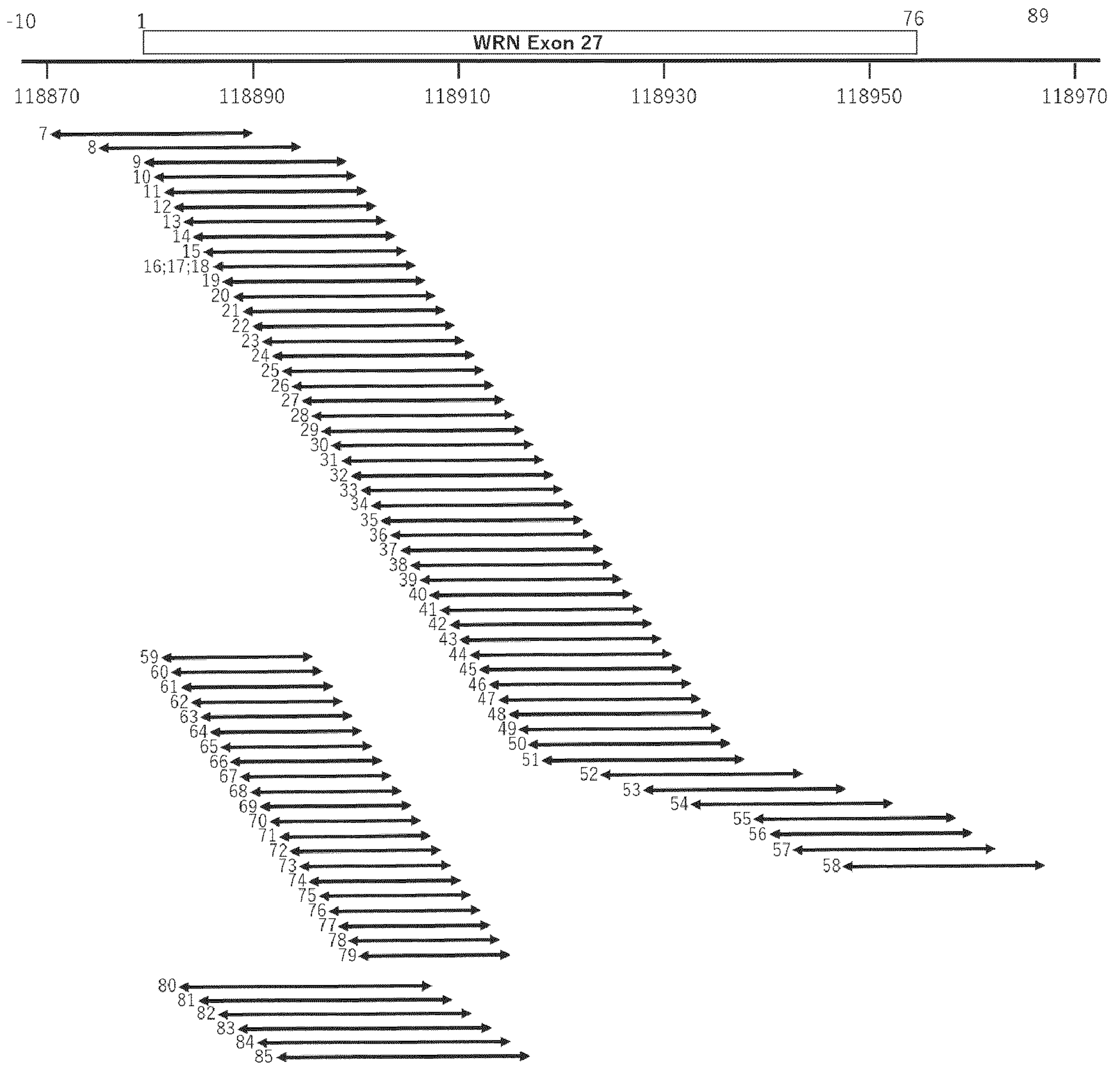
-10
1
76
89
WRN Exon 27
118870
118890
118910
118930
118950
118970
7
8
9
10
11
12
13
14
15
16;17;18
19
20
21
22
23
24
25
26
27
28
29
30
31
32
33
34
35
36
37
38
39
40
41
42
43
44
45
46
47
48
49
50
51
52
53
54
55
56
57
58
59
60
61
62
63
64
65
66
67
68
69
70
71
72
73
74
75
76
77
78
79
80
81
82
83
84
85

FIG.5

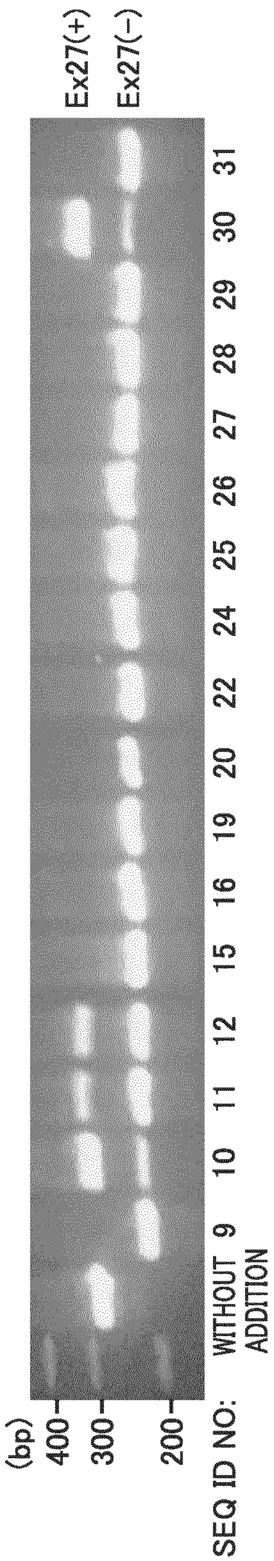
Ex27(+)
Ex27(−)
(bp)
400
300
200
SEQ ID NO:
WITHOUT ADDITION
9
10
11
12
15
16
19
20
22
24
25
26
27
28
29
30
31

FIG.6

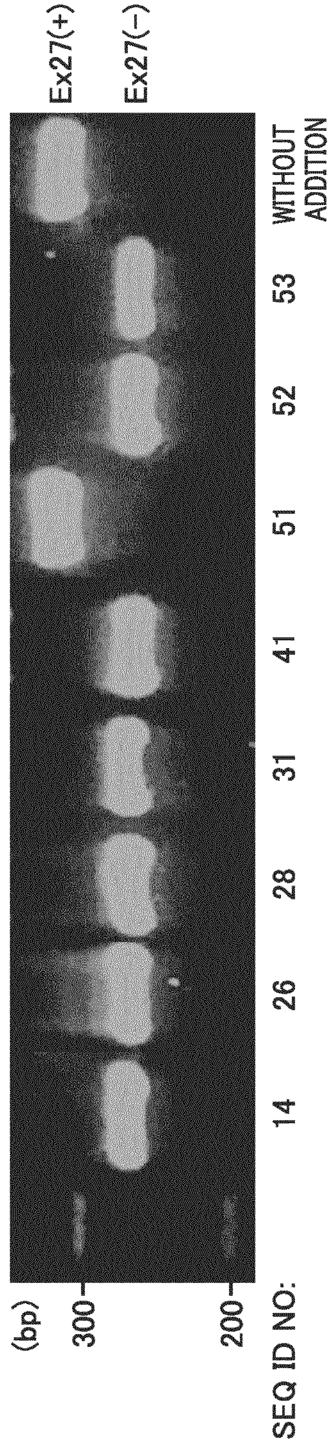
Ex27(+)
Ex27(−)
(bp)
300
200
SEQ ID NO:
14
26
28
31
41
51
52
53
WITHOUT ADDITION

FIG.7

EXON 26-DELETED PATIENT
(WITHOUT ASO)

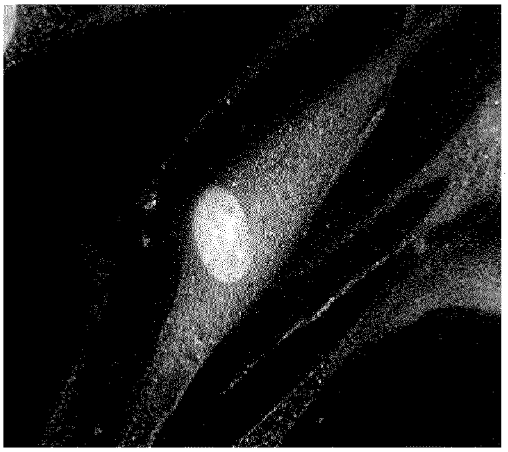

EXON 26-DELETED PATIENT
(ASO: No. 28)

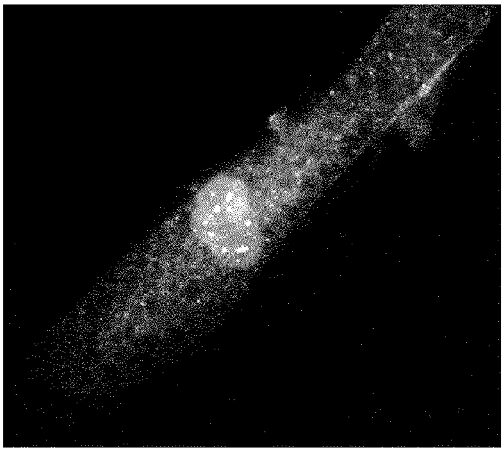

**INTERNATIONAL SEARCH REPORT**

International application No. **PCT/JP2022/021818**

**A. CLASSIFICATION OF SUBJECT MATTER**

***C12N 15/113***(2010.01)i; ***A61K 31/7088***(2006.01)i; ***A61K 31/7115***(2006.01)i; ***A61K 31/712***(2006.01)i; ***A61K 31/7125***(2006.01)i; ***A61K 47/54***(2017.01)i; ***A61K 47/55***(2017.01)i; ***A61K 47/60***(2017.01)i; ***A61K 47/61***(2017.01)i; ***A61K 47/62***(2017.01)i; ***A61K 47/68***(2017.01)i; ***A61K 48/00***(2006.01)i; ***A61P 43/00***(2006.01)i; ***C12N 15/12***(2006.01)i; ***C12N 15/55***(2006.01)i

FI: C12N15/113 130Z; A61P43/00; A61K48/00; A61K47/60; A61K47/62; A61K47/54; A61K47/55; A61K47/68; A61K47/61; A61K31/7088; A61K31/7115; A61K31/712; A61K31/7125; C12N15/55; C12N15/12 ZNA

According to International Patent Classification (IPC) or to both national classification and IPC

**B. FIELDS SEARCHED**

Minimum documentation searched (classification system followed by classification symbols)

C12N15/113; A61K31/7088; A61K31/7115; A61K31/712; A61K31/7125; A61K47/54; A61K47/55; A61K47/60; A61K47/61; A61K47/62; A61K47/68; A61K48/00; A61P43/00; C12N15/12; C12N15/55

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | 嶋本 顕, 遺伝子診断検査法, ウェルナー症候群の診断・診療ガイドライン 2012年版, 2012, pages 29-36, non-official translation (SHIMAMOTO, Akira. Genetic Diagnostic Test. Diagnosis and treatment guidelines for Werner syndrome 2012.) entire text, particularly, pages 34-35 | 1-29 |
| Y | BENNETT, C. F. et al. RNA Targeting Therapeutics: Molecular Mechanisms of Antisense Oligonucleotides as a Therapeutic Platform. Annual Review of Pharmacology and Toxicology. 2010, vol. 50, pages 259-293 page 264 | 1-29 |
| Y | JP 2012-506703 A (AVI BIOPHARMA, INC.) 22 March 2012 (2012-03-22) entire text, particularly, claims, examples | 1-29 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 August 2022** | **16 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/021818**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/021818**

| Patent document cited in search report | Publication date (day/month/year) | Patent family member(s) | Publication date (day/month/year) |
|---|---|---|---|
| JP 2012-506703 A | 22 March 2012 | WO 2010/048586 A1<br>claims, examples | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011052436 A
- WO 2014046212 A
- WO 2015125783 A
- WO 1991009033 A
- WO 2009064471 A


### Non-patent literature cited in the description


- *Geriatr Gerontol Int,* 2013, vol. 13, 475-481
- *Nature,* 1992, vol. 355, 735-738
- *Am. J. Hum. Genet.,* 1997, vol. 60, 330-341
- *Human Genetics,* 1981, vol. 58, 310-316
- *Rinsho derma,* 2000, vol. 40, 1512-1513
- *Annu. Rev. Pharmacol. Toxicol.,* 2010, vol. 50, 259-293
- *J. Med. Chem.,* 2016, vol. 59, 9645-9667
- *J. Am. Chem. Soc,* 2016, vol. 138, 15663-15672